# EUROPEAN PATENT SPECIFICATION

(11) **EP 3 004 120 B1**
(45) Date of publication and mention of the grant of the patent: **29.08.2018**
(21) Application number: 14807914.8
(22) Date of filing: 05.06.2014
(51) Int. Cl.: C07B 37/04, C07C 17/266, C07C 2/86, C07D 207/20, C07D 209/08, C07D 277/64, C07D 307/28, C07D 307/80, C07D 309/18, C07D 311/58, C07D 321/06, C07D 333/54, C07D 409/04, C07D 417/04, C07F 15/00, C07F 9/50, C07F 9/6558, B01J 31/24, C07F 9/53

(54) **SPIRO-1,1'-BIINDANE-7,7-BISPHOSPHINE OXIDES AS HIGHLY ACTIVE SUPPORTING LIGANDS FOR PALLADIUM-CATALYZED ASYMMETRIC HECK REACTION**
SPIRO-1,1'-BIINDAN-7,7-BISPHOSPHINOXIDE ALS HOCHAKTIVE UNTERSTÜTZENDE LIGANDEN FÜR PALLADIUMKATALYSIERTE ASYMMETRISCHE HECK-REAKTION
OXYDES DE SPIRO-1,1'-BIINDANE-7,7-BISPHOSPHINE COMME LIGANDS DE SUPPORT HAUTEMENT ACTIFS POUR UNE RÉACTION DE HECK ASYMÉTRIQUE CATALYSÉE PAR DU PALLADIUM

(30) Priority: 05.06.2013 US 201361831313 P
(43) Date of publication of application: 13.04.2016
(73) Proprietor: Nanyang Technological University, Singapore 639798 (SG)
(72) Inventor: ZHOU, Jianrong, Steve, Singapore 639798 (SG); HU, Jian, Singapore 639798 (SG); WU, Chunlin, Singapore 639798 (SG)
(74) Representative: Viering, Jentschura & Partner mbB Patent- und Rechtsanwälte
(86) International application number: PCT/SG2014/000260
(87) International publication number: WO 2014/196930

(56) References cited:
- CN-A- 1 439 643
- XIE J.H. ET AL: 'APPLICATION OF SDP LIGANDS FOR PD-CATALYZED ALLYLIC ALKYLATION' ADVANCED SYNTHESIS AND CATALYSIS vol. 346, no. 6, 2004, pages 625 - 632, XP055299146
- XIE J.H. ET AL: 'SYNTHESIS OF SPIRO DIPHOSPHINES AND THEIR APPLICATION IN ASYMMETRIC HYDROGENATION OF KETONES' JOURNAL OF THE AMERICAN CHEMICAL SOCIETY vol. 125, no. 15, 2003, pages 4404 - 4405, XP009125884

## Description

### FIELD OF THE INVENTION

The present invention relates to the field of Pd-catalyzed covalent carbon-carbon single bond forming. The present invention is further directed to the catalyst complex as disclosed herein and its respective use.

### BACKGROUND

Pd-catalyzed coupling reactions for forming covalent carbon-carbon single bonds are one of the most powerful strategies and commonly used in organic synthesis, particularly in the field of pharmaceuticals and fine chemicals. The coupling reactions invented by R. Heck, E. Negishi and A. Suzuki involving a Pd catalyst complex have been awarded with the Nobel Prize in Chemistry in 2010. Ligands, most commonly phosphorous ligands, are used in these coupling reactions in order to obtain products with high isomer- and enantioselectivity and good yield. Hayashi reported the first example of asymmetric intermolecular Heck reaction using a reactive oelfin, 2,3-dihydrofuran. In his case, the major isomer was formed as the carbon-carbon double migration products of the initial Heck isomer.¹ Since then, the reaction has become a test ground for many chiral phosphorus ligands.² For example, the use of the PHOX ligand can minimize double-bond isomerization, but its catalytic activity is not satisfying due to long reaction time and limited substrate scope.³ Many different types of phosphorus ligands, such as bisphosphites, *P,N-*ligands and bisphosphines, were also tested but high enantioselectivity was limited to reactive olefins, such as 2,3-dihydrofuran and cyclopentene and no general catalysts were available for most cyclic olefins.⁴ Intermolecular reactions were therefore rarely used in synthesis due to limitation in scope of olefins and aryl electrophiles as well as limited catalytic activity.⁵ Oestreich et al. recently reported the use of (*R*)-BINAP oxide as a chiral ligand in asymmetric Heck coupling reaction of cyclopentene and 2,3-dihydrofuran, but the scope of olefin and aryl triflates were limited to a few compounds and the enantioselectivity was also not satisfying.⁶ Chiral bisphosphine oxides, on the other hand, have been used as supporting ligands in other asymmetric metal-catalyzed reactions.⁷ The spiro-1,1'-biindane-7,7-bisphosphines were developed by Q.-L. Zhou et al., but monoxides of this class of bisphosphines were not used previously as chiral ligands in Pd-catalyzed carbon-carbon single bond coupling reactions, particularly Heck reaction of cyclic olefins.⁸ Xie et al. discloses the synthesis of a new chiral spiro diphosphine ligand (SDP), 7,7'-bis[di-(3,5-dimethyl-4-methoxy-phenyl)phosphino]-1,1'-spirobiindane (DMM-SDP), useful for the Pd-catalyzed asymmetric allylic alkylation of 1,3-diphenyl-2-propenyl acetate with dimethyl malonate and related nucleophiles (see Abstract). A compound falling within the scope of the presently-defined Formula (I) is created in a step of the process for the formation of DMM-SDP (see Introduction; Compound 6, Scheme 2). However, this compound is not isolated, nor is there any clear direction or suggestion of the formation of a palladium complex comprising said compound²⁵.

Hence, there is a need in the art for phosphine ligands in the field of Pd-catalyzed carbon-carbon single bond formation providing catalyst complexes with high catalytic activity for most substrates, and products with high isomer- and enantioselectivity and high product yield.

### SUMMARY OF THE INVENTION

Thus, in a first aspect the present invention is directed to a catalyst complex comprising Pd and at least one ligand of Formula (I) 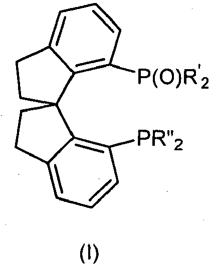 wherein,
R' and R" are independently selected from the group consisting of substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

In another aspect the present invention relates to a method for forming a covalent carbon-carbon single bond in the Pd-catalyzed Heck carbon-carbon coupling reaction, the method comprising:
(i) providing a catalyst complex as disclosed herein; and
(ii) reacting at least one electrophilic compound of the Formula (IV) with at least one olefin of the Formula (V)

   R¹-LG¹ (IV)

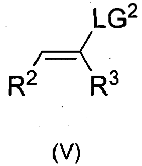

   wherein
   R¹ is any organic compound;
   R² and R³ combine to form together with the carbon atoms to which they are attached a substituted or unsubstituted 5- to 40-membered cycloalkenyl or heterocycloalkenyl; and LG¹ and LG² are leaving groups;
in the presence of the catalyst complex under conditions suitable for forming the covalent carbon-carbon single bond.

In a still further aspect the present invention relates to the use of a catalyst complex in the Pd-catalyzed Heck coupling reaction for forming a covalent carbon-carbon single bond, the catalyst complex comprising Pd and at least one ligand of Formula (I) 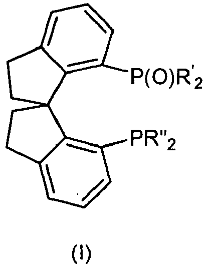 wherein,
R' and R" are independently selected from the group consisting of substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

### DETAILED DESCRIPTION OF THE PRESENT INVENTION

The present invention is based on the inventors' surprising finding that catalyst complexes comprising spiro-1,1'-biindane-7,7'-bisphosphine oxide ligands of Formula (I) and Pd show high catalytic activity in reaction couplings for forming carbon-carbon single bonds between electrophilic compounds of Formula (IV) and olefins of Formula (V). The resulting products were obtained with high product yields as well as high selectivity, in particular with respect to isomeric and enantiomeric ratios. Without wishing to be bond to any particular theory, it is believed that due to the spiro and the monoxide group of the ligand the catalyst complex provides for excellent sterical and electronic properties and a bite angel suitable for the Pd-catalyzed coupling reactions for forming carbon-carbon single bonds.

The catalysts previously developed for Pd-catalyzed asymmetric Heck reaction were not general with respect to structural changes in aryl, heteroaryl and alkenyl electrophiles and in cyclic olefins. Typically, structural modification of catalysts was needed when substrates were changed. In addition, some catalysts such as those supported PHOX ligands showed slow turnover frequency and demand long reaction times.

In contrast to the existing approaches, the present invention provides for a catalyst complex, particularly ligands, with high catalytic activity resulting in high product yields and selectivity.

The inventive method can be conducted at ambient conditions and as a simple *one-pot* synthesis.

Based on this finding, in a first aspect the present invention thus relates to a catalyst complex comprising Pd and at least one ligand of Formula (I)

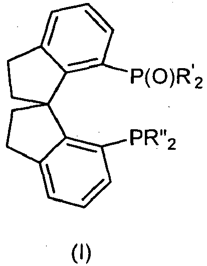

The moieties R' and R" of Formula (I) are independently selected from the group consisting of substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

The term "catalyst complex" as used herein relates to a metal organic complex with catalytic activity comprising Pd and an organic ligand as disclosed herein.

The term "at least one" as used herein relates to one or more, for example 1, 2, 3, 4, 5, 6, 7, 8, 9 or more of the referenced species.

The term "substituted" as used herein in relation to the above moieties refers to a substituent other than hydrogen. Such a substituent is preferably selected from the group consisting of halogen, -CF₃, -C₂F₅, -C₃F₇, -C₄F₉, -C₅F₁₁, and other fluoroalkyl of 2-5 carbons,-OH, -NH₂, -NO₂, -CHO, -CN, -COOH, -SH, -SO₂OH, -CONH₂, -NH-NH₂, -OR, -NRR', -C(O)R,-C(O)OR, -(CO)NRR', -NR'C(O)R, -OC(O)R, aryl with 5 to 20 carbon atoms, cycloalk(en)yl with 3 to 20 carbon atoms, 3- to 8-membered heterocycloalk(en)yl, and 5- to 20-membered heteroaryl, wherein R and R' are independently selected from hydrogen, alkyl with 1 to 10 carbon atoms, alkenyl with 2 to 10 carbon atoms, alkynyl with 2 to 10 carbon atoms, aryl with 5 to 14 carbon atoms, cycloalk(en)yl with 3 to 20 carbon atoms, 5- to 14- membered heteroaryl, comprising 1 to 4 heteroatoms selected from nitrogen, oxygen, and sulfur, and 5- to 14-membered heterocycloalk(en)yl, comprising 1 to 4 heteroatoms selected from nitrogen, oxygen, and sulfur. Any of these substituents may again be substituted, it is however preferred that these substituents are unsubstituted.

Alkyl refers to a saturated hydrocarbon moiety, such as methyl, ethyl, and the like.

Akenyl and Alkynyl comprise at least one carbon-carbon double bonds or triple bonds, respectively, and are otherwise defined as alkyl above.

Cycloalkyl refers to a non-aromatic carbocyclic moiety, such as cyclopentanyl, cyclohexanyl, and the like.

Cycloalkenyl refers to non-aromatic carbocyclic compounds that comprise at least one carbon-carbon double bond.

Similarly, heterocycloalk(en)yl relates to cycloalk(en)yl groups wherein 1 or more ring carbon atoms are replaced by heteroatoms, preferably selected from nitrogen, oxygen, and sulfur.

Aryl relates to an aromatic ring that is preferably monocyclic or consists of condensed aromatic rings. Preferred aryl substituents are moieties with 6 to 14 carbon atoms, such as phenyl, naphthyl, anthracenyl, and phenanthrenyl.

Heteroaryl refers to aromatic moieties that correspond to the respective aryl moiety wherein one or more ring carbon atoms have been replaced by heteroatoms, such as nitrogen, oxygen, and sulfur.

All of the afore-mentioned groups can be substituted or unsubstituted. When substituted the substituent can be selected from the above list of substituents.

Halogen refers to F, Cl, Br, and I.

The term "one-pot synthesis" as used herein means that the reactions (i) and (ii) according to the present invention are carried out in the same reaction vessel without any purification step of an intermediate.

In various embodiments of the present invention, the ligand of Formula (I) is a ligand of Formula (II) or Formula (III) 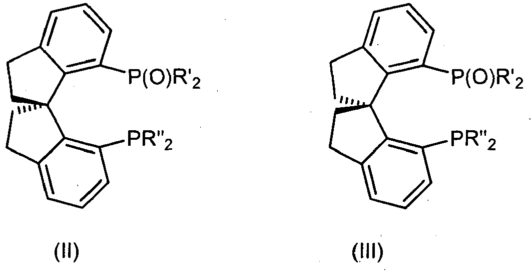 wherein the moieties R' and R" are independently selected from the group consisting of substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

In a preferred embodiment, the moieties R' and R" are independently selected from the group consisting of cyclohexanyl (Cy), phenyl (Ph), 1,3-dimethylbenzene (*m*eta-Xylyl), and *para-*FC₆H₄. In some embodiments, R' and R" of one ligand are the same or different from each other. For example, in a ligand of Formula (II) R' is Cy and R" is *para*-FC₆H₄ or *vice versa.* A further ligand of Formula (II) according to the present invention bears R' and R" being the same moiety, for example Ph. The same applies to the ligands of Formulas (I) and (III). According to the present invention mixtures of these ligands can be used in the same catalyst complex as well.

In a further preferred embodiment, the catalyst complex as described herein comprises ligands which are independently selected from the group consisting of 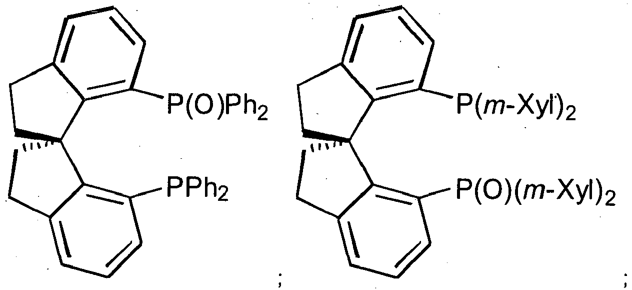 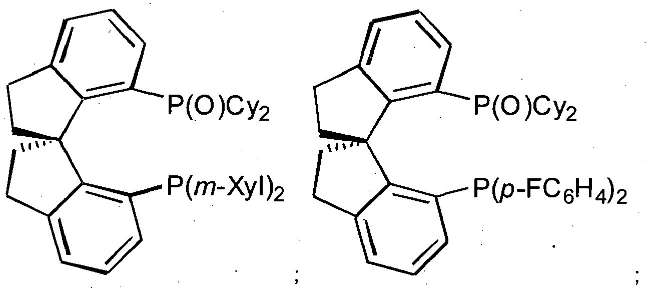 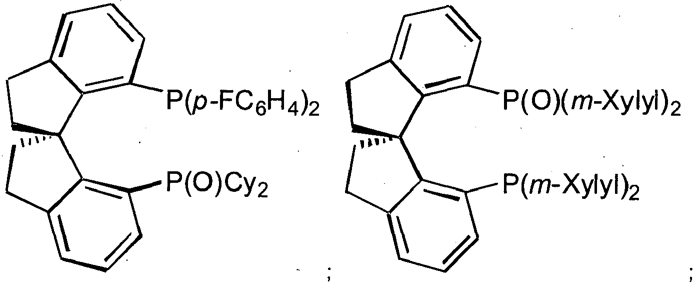 and mixtures thereof.

In accordance with the present invention, the catalyst complex comprises Pd, wherein the Pd is Pd(0) and/or Pd(+II), wherein the number in the brackets relates to the oxidation state of the metal. Due to the well-known catalytic reaction cycle of the covalent carbon-carbon single bond formation, the Pd switches between the oxidation number +II and 0. In general, the Pd(0) specie is known to be the catalytically active specie in the Pd-catalyzed Heck reaction. Therefore, in a preferred embodiment the Pd of the catalyst complex is Pd(0). In another preferred embodiment of the present invention the Pd(0) specie is generated *in situ.* Suitable precursors for providing said Pd specie are organo Pd compounds and Pd salts, wherein the Pd has the oxidations number +II or 0. In a preferred embodiment the Pd of the catalyst complex is generated from a Pd precursor selected from the group consisting of Pd(dba)₂, Pd₂(dba)₃, Pd(PPh₃)₄, Pd(OAc)₂, PdCl₂, PdBr₂, Pdl₂, PdCl₂(PPh₃)₂, Pd(OAc)₂, Pd(PPh₃)₄, or a mixture thereof. In a more preferred embodiment the Pd is generated from Pd(OAc)₂, Pd(dba)₂, or Pd₂(dba)₃. In the case the Pd precursor is a Pd(II) specie, said Pd(II) specie is reduced to Pd(0) *in situ* by generally known techniques and compounds suitable for the described reaction and purpose. For example, Pd(II) can be reduced by triphenylphosphine in the reaction conditions.

The term *"in situ"* as used herein means in the reaction mixture. Specifically, this means that the respective compound is synthesized in the reaction mixture.

In another aspect, the present invention is directed to a method for forming a covalent carbon-carbon single bond in a Pd-catalyzed carbon-carbon Heck coupling reaction, the method comprising:
(i) providing a catalyst complex as described herein; and
(ii) reacting at least one electrophilic compound of the Formula (IV) with at least one olefin of the Formula (V)

   R¹-LG¹ (IV)

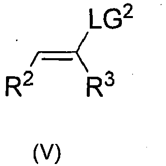

   wherein R¹ is any organic compound, R² and R³ combine to form together with the carbon atoms to which they are attached a substituted or unsubstituted 5- to 40-membered cycloalkenyl or heterocycloalkenyl, and LG¹ and LG² are leaving groups in the presence of the catalyst complex under conditions suitable for forming the carbon-carbon bond.

. The catalyst complex of the present invention can be generated from suitable Pd precursors and ligands as disclosed herein by solving the precursors in a solvent as described herein and by optional stirring and/or heating.

In various embodiments of the invention, the reaction time of step (i) is from 0.1 hours to 12 hours. The reaction time can also be from 0.1 hour to 8 hours, from 0.5 hour to 6 hours or from 0.5 hour to 4 hours. In a preferred embodiment, the reaction time is from 3 hours.

In various embodiments of the invention, the reaction temperature of step (i) of the method disclosed herein ranges 0°C to 120°C. The reaction temperatures of these steps can also be from 0°C to 75°C. Preferably, the reaction temperature used in step (i) is from 10°C to 40°C, more preferably 25°C.

As already mentioned above, in the case that a precursor with Pd(II) is used, said Pd(II) is reduced in order to generate the catalytic active Pd(0) specie by generally known techniques and compounds. Optionally, a solvent as described herein can be used to provide the catalyst complex. In the case the ligand itself is liquid, the Pd precursor can be solved in the ligand without additional solvent. If necessary, the catalyst complex is purified before step (ii) of the present invention is conducted.

In various embodiments of the invention, the catalyst complex is typically in an amount of 2.5 to 5 mol.% Pd, although catalyst loading can be lowered, such as 0.1 mol.%.

As already mentioned above, the method can be conducted as a one-pot synthesis. Therefore, the reaction mixture comprising the Pd precursor, the at least one ligand, the at least olefin, and the at least one electrophilic compound are provided in a reaction vessel. If necessary, a base and a solvent are added to the mixture. If necessary, the steps (i) and (ii) are carried out under inert gas. In the case a component of this mixture is liquid and the other components can be dissolved in the liquid component, no additonal solvent is needed. The providing of the catalyst complex of step (i) can be conducted by simple stirring at ambient temperatures, for example 25°C, in the presence of the at least one olefin and the at least one electrophilic compound. When step (i) is completed and the catalyst complex is formed, step (ii) of the present invention can be conducted without purification of the catalyst complex.

If required, the mixture comprising the Pd precursor, the ligand, and the solvent are heated to generate the catalyst complex.

The term "any organic moiety" as used herein refers to carbon-containing moieties. These moieties can be linear or branched, substituted or unsubstituted, and are preferably derived from hydrocarbons, typically by substitution of one or more hydrogen or carbon atoms by other atoms, such as oxygen, nitrogen, sulfur, phosphorous, or functional groups that contain oxygen, nitrogen, sulfur, phosphorous. The organic moiety can comprise any number of carbon atoms, for example up to up to 5000 or more (typically in case of polymeric moieties), but preferably it is a low molecular weight organic moiety with up to 100, or more preferably up to 40 carbon atoms and, optionally, a molecular weight Mw of 1000 or less. It is preferred that the organic moiety is compatible with the activation reaction described herein and does not adversely affect the described reaction mechanism. Suitable groups and moieties are well known to those skilled in the art or can be readily identified by routine experimentation.

In a preferred embodiment the organic moiety is independently selected from the group consisting of linear or branched, substituted or unsubstituted C₁-Cₓ alkyl; linear or branched, substituted or unsubstituted alkenyl with 2 to x carbon atoms; linear or branched, substituted or unsubstituted alkynyl with 2 to x carbon atoms; linear or branched, substituted or unsubstituted alkoxy with 1 to x carbon atoms; substituted or unsubstituted cycloalkyl with 3 to x carbon atoms; substituted or unsubstituted cycloalkenyl with 3 to x carbon atoms; substituted or unsubstituted aryl with 6 to x carbon atoms; and substituted or unsubstituted heteroaryl with 3 to x carbon atoms; with x being any integer of 2 or more, preferably up to 50, more preferably up to 30.

The organic moiety can also be a combination of any of the above-defined groups, including but not limited to alkylaryl, arylalkyl, alkylheteroaryl and the like, to name only a few, all of which may be substituted or unsubstituted.

In a further embodiment of the present invention R¹ is selected from the group consisting of linear or branched, substituted or unsubstituted alkenyl with 2 to x carbon atoms; substituted or unsubstituted cycloalkenyl with 3 to x carbon atoms; substituted or unsubstituted cycloalkenyl with 3 to x carbon atoms; substituted or unsubstituted aryl with 6 to x carbon atoms; and substituted or unsubstituted heteroaryl with 3 to x carbon atoms; with x being any integer of 2 or more, preferably up to 50, more preferably up to 30. In a preferred embodiment, R¹ is selected from the group consisting of substituted or unsubstituted, linear or branched alkenyl with 3 to 15 carbon atoms; substituted or unsubstituted benzene; and substituted or unsubstituted naphthalene.

The term "leaving group" as used herein refers to a moiety that is released from a molecule it was covalently bound to by keeping the pair of electrons previously forming the bond. In the event that hydrogen is the leaving group of the compound of Formulas (IV) to (VII), respectively, the hydrogen does not keep the pair of electrons. A leaving group can be a single atom, a molecule, or a functional group. These groups can be an anion or a neutral molecule. The leaving group may have a -I effect. The leaving group of the present invention depends on the conducted coupling reaction type. In general, a different leaving group is used for each coupling reaction described herein.

The catalyst complexes, methods, and uses disclosed herein relate to the Heck coupling reaction which is well established in the art. The skilled person is capable to adopt and modify the catalyst complexes, methods, and uses disclosed herein for a coupling reaction type so to optimize other coupling reaction types. For example, the skilled person is capable to choose suitable bases, solvents, suitable moieties R¹, R², and R³ as well as suitable leaving groups LG¹ and LG² for each coupling reaction type. Further, the skilled person is capable to amend the methods by adding and/or omitting concrete compounds which are necessary for the respective coupling reactions. In particular, the skilled person is capable to adopt and modify the methods and uses when different leaving groups LG¹ are used since it is known in the art that different solvents, bases, acidic additives and the like are used in the case that leaving group is halogen instead of triflate.

In various embodiments, LG¹ is selected from the group consisting of halogen,-OSO₂C₄F₉, -OSO₂CF₃, -OSO₂F, -OTs, and -OMs, preferably from the group consisting of Cl, Br, I, and -OSO₂CF₃. In a more preferred embodiment, LG¹ is -OSO₂CF₃ and Br.

The electrophilic compound of Formula (IV) can be any compound of aromatic triflates, heteroaromatic triflates, and alkenyl triflates.

In a preferred embodiment, the electrophilic compound of Formula (IV) is selected from the group consisting of 1-bromonaphthalene, 2-bromonaphthalene, bromobenzene, 4-bromoanisole, 4-bromotoluene, 1-bromo-4-fluorobenzene, 2-bromoanisole, *N*-methyl-2-bromopyrrole, 3-bromoindole, 5-bromo-2-methyl-1,3-benzothiazole, 3-bromobenzofuran, 3-bromobenzothiophene, 2-bromothiophene, 2-bromothiophene, 4-bromo-3-chromene, 1-bromostyrene and (*E*)-2-bromostyrene, 1-bromocyclohexene, 1-bromocyclopentene, bromoethene, (*E*)-1-bromopropene, 2-bromopropene, iodobenzene, 1-iodonaphthalene, 2-iodonaphthalene, 4-iodoanisole, 4-iodotoluene, 4-chlorotoluene, 2-chlorotoluene, 1-chloronaphthalene, 2-chloronaphthalene, chlorobenzene, 4-chloroanisole, 2-chloroanisole, 3-chloroindole, *N*-methyl-2-chloropyrrole, 5-chloro-1,3-benzothiazole, 3-chlorobenzofuran, 3-chlorobenzothiophene, 2-chlorothiophene, 2-chlorothiophene, 1-naphthyl triflate, 2-naphthyl triflate, phenyl triflate, *para*-(ethoxycarbonyl)phenyl triflate, *para*-anisyl triflate, *para-tert-*butylphenyl triflate, *ortho*-methylphenyl triflate, *ortho*-anisyl triflate, *para*-chlorophenyl triflate, *para*-benzophenonyl triflate, *para*-formylphenyl triflate, 2-methylcyclohexenyl triflate, 2-methylbenzo[*d*]thiazol-5-yl triflate, 1-tosyl-1*H*-indol-5-yl triflate, *meta*-anisyl triflate, *para-tert-*butylphenyl triflate, *para*-(trifluoromethyl)phenyl triflate, *para*-chlorophenyl triflate, and *para-*fluorophenyl triflate, triflate, 2-thienyl and 3-thienyl trilfates and their benzo-derivatives, 2-furanyl and 3-furanyl triflates and their benzo-derivatives, *N*-Boc-2-pyrrolidinyl and *N*-Boc-3-pyrrolidinyl trilfates, cyclohexenyl triflate, 1-styryl and (*E*)-2-styryl trilfates.

The olefin of Formula (V), the moieties R² and R² combine to form together with the carbon atoms to which they are attached to a substituted or unsubstituted ring. In a further preferred embodiment, R² and R³ combine to form together with the carbon atoms to which they are attached a substituted or unsubstituted 5- to 40-membered cycloalkenyl or heterocycloalkenyl, more preferably a substituted or unsubstituted 5- to 20-membered cycloalkenyl or heterocycloalkenyl, and even more preferably a substituted or unsubstituted 5- or 6-membered cycloalkenyl or heterocycloalkenyl.

In another preferred embodiment of the present invention, the at least one olefin of Formula (V) is selected from the group consisting of cyclopentene; cyclohexene; cyloheptene; cyclooctene; 2,3-dihydrofuran; 2,5-dihydrofuran; 2,3-dihydropyran; *N*-acyl-2-pyrroline; 1,3-dioxep-5-ene and its 2-substituted derivatives; 3,4-dihydro-2*H*-pyran; *cis*-4,7-dihydro-1,3-dioxepine and its 2-substituted derivatives; *N*-acyl-, *N*-formyl and *N*-alkoxycarbonyl-2,3-dihydro-1*H*-pyrroles and *N*-Boc-2,3-dihydro-1*H*-pyrrole.

In a more preferred embodiment, the at least one olefin of Formula (V) is selected from the group consisting of cyclopentene; cyclohexene; cycloheptene; cyclooctene; 2,3-dihydrofuran; 2,5-dihydrofuran; 2,3-dihydropyran; *N*-acyl-2-pyrroline; 1,3-dioxep-5-ene and its 2-substituted derivatives; *cis*-4,7-dihydro-1,3-dioxepine and its 2-substituted derivatives; *N*-acyl-, *N*-formyl. and *N*-alkoxycarbonyl-2,3-dihydro-1*H*-pyrroles.

In an embodiment of the present invention, a base is provided. When the leaving group LG² of Formula (V) is hydrogen, the base is essential in order to deprotonate and regenerate the Pd(0) catalyst complex. Therefore, any base which is suitable for this purpose can be used. In a preferred embodiment the base is selected from the group consisting of inorganic carbonate; inorganic phosphate; inorganic acetate; nitrogen containing organic compound; or a mixture thereof. Preferably, the nitrogen containing organic compound is selected from the group consisting of trialkyl amine; dialkyl amine; *N,N*-dialkylpyridine; *N,N*-dilalkylaniline; or a mixture thereof. Preferred trialkyl amines are of the formula NR^{a}₃, wherein each R^{a} is independently selected from group consisting of ethyl; *n*-propyl; and *n*-butyl. Dialkyl amines are of the formula HNR^{b}₂, wherein preferably each R^{b} is independently selected from group consisting of ethyl; *n*-propyl; and *n*-butyl. In another preferred embodiment the base is selected from the group consisting of diethylamine, di-*n*-butylamine, pyrrolidine, piperidine and other dialkylamines, triethylamine, tri-n-butylamine, diisopropylethylamine, dicyclohexylmethylamine and other trialkylamines; *N*-methyl-2,2,6,6,tetramethylpiperidine; 2,2,6,6,tetramethylpiperidine; pyridine; 2,6-dimethylpyridine; 2,6-di-*tert*-butylpyridine; DABCO (1,4-Diazabicyclo[2.2.2]octane); Li₂CO₃; Na₂CO₃; K₂CO₃; K₃PO₄; LiOAc; NaOAc; 4-aminopyridine; and KOAc.

The reactions can be carried out in a solvent. The solvent used in the present invention can be any solvent which is suitable. In a preferred embodiment the method is carried out in a solvent which is selected from the group consisting of an organic solvent; water; and a mixture thereof.

The organic solvent is preferably selected from the group consisting of aromatic solvents; chlorinated solvents; ester solvents; amide solvents; urea solvents; and mixture thereof. In a more preferred embodiment the organic solvent is selected from the group consisting of diethyl ether; THF (tetrahydrofuran); 1,4-dioxane; tetrahydropyran; *tert*-butyl methyl ether; cyclopentyl methyl ether; di-*i*so-propyl ether; 1,2-dimethoxyethane; diglyme; triglyme; benzene; *ortho*-xylene, *meta*-xylene, *para*-xylene, and mixtures of xylenes; toluene; mesitylene; anisole; 1,2-dimethoxybenzene; *α*,*α*,*α*-trifluoromethylbenzene; fluorobenzene; chlorobenzene; dichloromethane; 1,2-dichloroethane; 1,1-dichloroethane; chloroform; acetone; ethyl acetate; propyl acetate, acetonitrile; propionitrile; butyronitrile; benzonitrile; dimethyl sulfoxide (DMSO); Dimethylformamide (DMF), Dimethylacetamide (DMA); *N*-Methyl-2-pyrrolidone (NMP), ethylene carbonate; propylene carbonate; 1,3-Dimethyl-3,4,5,6-tetrahydro-2(1H)-pyrimidinone (DMPU), and a mixture thereof.

In another embodiment of the present invention the used solvent is a mixture of an organic solvent and water in a ratio of 1 : 1 to 100 : 1. The ratio can also be from 5 : 1 to 50 : 1 or 10 : 1 to 30 : 1. Preferably, the mixture of an organic solvent and water is in a ratio of 15 : 1 to 25 : 1; and more preferably 19 : 1.

In a preferred embodiment the organic solvent is dioxane.

In another preferred embodiment the used solvent is a mixture of dioxane and water in a ratio of 19 : 1.

In various embodiments, the reaction temperature of step (ii) of the method disclosed herein ranges 0°C to 120°C. The reaction temperatures of these steps can also be from 15°C to 90°C. Preferably, the reaction temperature used in step (ii) is from 30°C to 80°C, more preferably 50°C to 70°C.

The reaction temperatures of steps (i) and (ii) may be selected independently from each other, for instance the reaction temperature of step (i) may be 25°C whereas the reaction temperature of step (ii) may be 70°C or *vice versa.*

In various embodiments of the invention, the reaction time of step (ii) is from 0.1 hours to 144 hours. The reaction time can also be from 0.1 hour to 72 hours, from 0.1 hour to 48 hours or from 0.1 hour to 24 hours. In a preferred embodiment, the reaction time is from 0.25 hour to 18 hours, more preferably from 0.5 hour to 12 hours, still more preferably from 0.5 hour to 6 hours, and even more preferably from 1 hour to 4 hours.

As already mentioned above, the herein disclosed methods and uses provides for products with high yield and selectivity of the general Formula (VI)

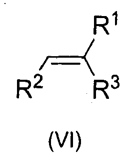

Depending on the used type of olefin, double bond migration may occur. Thus, the double bond may be located between other carbon atoms than in the olefin used as starting material. The obtained product may be of the Formula (VII) 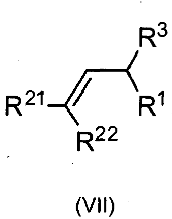 wherein the moieties R²¹ and R²² are of the used olefin and can be derived from the olefin as described above.

In a further aspect, the present invention relates to the use of a catalyst complex as described herein in the Pd-catalyzed Heck coupling reaction for forming a covalent carbon-carbon single bond, the catalyst complex comprising Pd and at least one ligand of Formula (I).

In various embodiments of the present invention, the used ligand of Formula (I) is the used ligand of Formula (II) or Formula (III) as described herein.

In a preferred embodiment, the used ligand according to the present invention is selected from the group consisting of 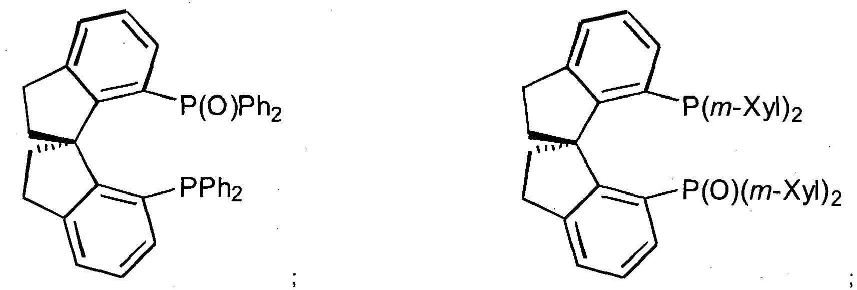 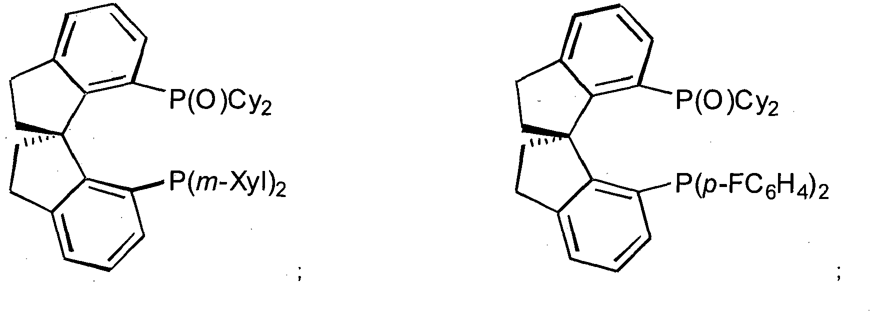 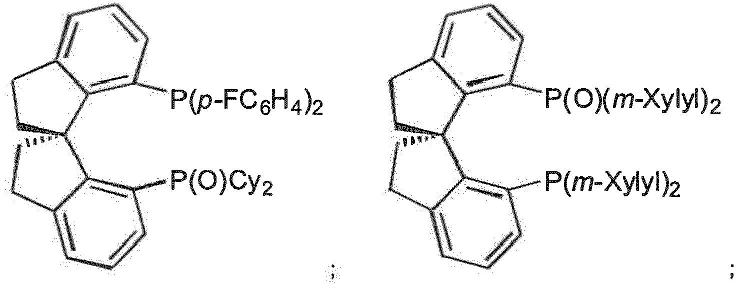 and a mixture thereof.

In a further embodiment, the Pd used in the catalyst complex is a Pd(0) and/or a Pd(+II) specie, preferably a Pd(0) specie.

All features and embodiments described for the catalyst complex are also applicable to the method and use disclosed herein and *vice versa.*

The following examples are provided to better illustrate the claimed invention.

### EXAMPLES

### I. GENERAL

All NMR spectra were acquired on Bruker BBFO1 400 MHz and Bruker AV 500 MHz NMR spectrometers. ¹H NMR (400 MHz) chemical shifts were recorded relative to SiMe₄ (δ 0.00) or residual protiated solvents (CDCl₃: δ 7.26; CD₂Cl₂: δ 5.32). Multiplicities were given as: s (singlet), d (doublet), t (triplet), q (quartet) and m (multiplet). The number of protons (n) for a given resonance was indicated by nH. Coupling constants were reported as a *J* value in Hz. ¹³C NMR (100 MHz) chemical shifts were recorded relative to solvent resonance (CDCl₃: δ 77.16; CD₂Cl₂: δ 53.84). ¹⁹F NMR (376 MHz) chemical shifts were recorded relative to an external standard (BF₃□OEt₂: *δ* 153.0). ³¹P NMR (126 MHz) chemical shifts were relative to an external standard (85% H₃PO₄: δ 0.00). Proof of purity of new compounds was demonstrated with copies of ¹H, ¹³C, ³¹P and ¹⁹F NMR spectra.

Glassware was dried at 120 °C for at least 3 h before use. Methanol was distilled over CaH₂ under argon. Dry toluene, hexane, diethyl ether and dichloromethane were collected from a solvent purification system containing a column of activated alumina (1 m x 2) under argon. Dry THF was freshly distilled from sodium/benzophenone under argon. *N*-Diisopropylethylamine was distilled from CaH₂ under argon before use. Methanol was distilled from sodium under argon before use. All anhydrous solvents were stored in Schlenk tubes in the glove box. Unless noted otherwise, commercially available chemicals were used as received without purification. The GC internal standard, *n*-C₁₂H₂₆ and *n*-C₁₄H₃₀ was degassed with argon and dried over activated 4 Å molecular sieve beads before use. Flash chromatography was performed using Merck 40-63D 60 Å silica gel. Gas chromatography (GC) analysis was performed on a Shimadzu GC-2010 instrument with Agilent J & W GC column DB-5MS-UI. GC/MS analysis was conducted on a Thermo Scientific DSQ II single quadrupole GC/MS instrument with Agilent J & W GC column DB-5MS-UI. ESI/MS analysis was conducted on a ThermoFinnigan LCQ Fleet MS spectrometer.

Chiral HPLC analysis was performed on a Shimadzu LC-20AD instrument using Daicel Chiracel columns at 25°C and a mixture of HPLC-grade hexanes and isopropanol as eluent. Optical rotation was measured using a JASCO P-1030 Polarimeter equipped with a sodium vapor lamp at 589 nm and the concentration of samples was denoted as *c*.

### A. Examples of synthesis of bisphosphine oxides

### (R)-2-diphenylphosphino-2'-diphenylphosphinyl-1,1'-binaphthyl or (R)-BINAP(O) [152646-80-5].¹

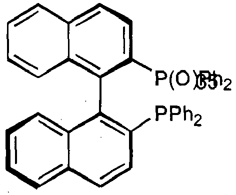

It was prepared from (*R*)-BINAP according to a modification of Grushin's method using Pdl₂ catalyst and 1,2-dibromoethane as terminal oxidant.¹ Under argon, a dry 25-mL Schlenk tube was charged with Pdl₂ (3.7 mg, 0.010 mmol), (*R*)-BINAP (250 mg, 0.40 mmol) and CH₂Cl₂ (2.5 mL). The mixture was stirred at 25°C for 3 h until all Pdl₂ was dissolved. A solution of NaOH (0.23 g, 5.75 mmol) in H₂O (1.5 mL) was then added, followed by 1,2-dibromoethane (0.50 g, 2.66 mmol). The resulting mixture was stirred in a 50°C bath for 14 h until (R)-BINAP was fully consumed (monitored by ³¹P NMR spectroscopy). The pH of the reaction mixture was then adjusted to ∼4 with 20% H₃PO₄ solution, followed by stirring with dppe (10 mg) for 5 min to remove Pd. The organic layer was separated, dried over MgSO₄, concentrated on a rotary evaporator, and purified by flash chromatography (10:1 CH₂Cl₂/EtOAc) in air, which afforded 0.216 g (84%) of the product as a white solid.
¹H NMR (400 MHz, CDCl₃): 7.92 (dd, *J* = 8.6, 2.2 Hz, 1H), 7.82 (d, *J* = 8.2 Hz, 1H), 7.74 (d, *J* = 8.4 Hz, 1H), 7.70 (d, *J* = 8.2 Hz, 1H), 7.62 - 7.57 (m, 3H), 7.41 (dd, *J* = 8.5, 2.9 Hz, 1H), 7.38-7.31 (m, 6H), 7.29-7.19 (m, 7H), 7.18-7.12 (m 1H), 7.10-7.01 (m, 4H), 6.98-6.93 (m, 2H), 6.90-6.86 (m, 1H), 6.81 (d, *J* = 8.2 Hz, 1H), 6.74-6.70 (m, 1H), 6.64 (d, *J* = 8.4 Hz, 1H).
³¹P{¹H} NMR (162 MHz, CDCl₃): 27.0 (s), -15.3 (s).

### (R)-2,2'-bis(dicyclohexylphosphino)-1,1'-binaphthyl or (R)-Cy-BINAP [139139-92-7].²

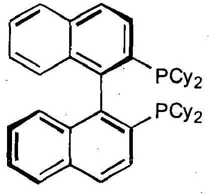

It was prepared according to a reported procedure (reported yield 34%).² In an argon-filled glove-box, HPCy₂ (0.23 g, 1.16 mmol), (dppe)NiCl₂ (0.106 g, 0.20 mmol) and dry DMF (4.07 mL) were charged into a 25-mL Schlenk tube. The mixture was heated with stirring in a 100°C bath for 30 min. A solution of DABCO (0.90 g, 8.0 mmol) and (*R*)-1,1'-binaphthyl 2,2'-bistriflate (1.10 g, 2.0 mmol) in dry DMF (6.0 mL) was added against flow of argon, followed by the addition of zinc dust (0.80 g, 12 mmol; activated by aqueous HCl and dried under vacuum). The mixture was stirred in a 115°C bath. Additional portions of HPCy₂ (0.23 g, 1.16 mmol each portion) were added after stirring at 115°C for 1 h, 3 h and 12 h, respectively. The mixture was further stirred at 115 oC for 3 days and 125°C for 1 day. Upon cooling to rt, the product precipitated out, and it was collected by filtration together with zinc dust and washed with MeOH (3 mL x 3) under argon. It was redissolved in CH₂Cl₂ (5.0 mL) and passed through a pad of silica gel with CH₂Cl₂ washings to remove zinc dust. Removal of solvent under vacuum afforded 0.31 g (24%) of the product as a white powder, which was pure by ¹H and ³¹P NMR spectroscopy.
¹H NMR (400 MHz, CDCl₃): 7.91 (d, *J* = 8.4 Hz, 2 H), 7.86 (d, *J* = 8.1 Hz, 2 H), 7.72 (d, *J* = 8.4 Hz, 2 H), 7.38 (ψt, *J* = 7.4 Hz, 2H), 7.14 (ψt, *J* = 7.5 Hz, 2H), 6.99 (d, *J* = 8.5 Hz, 2 H), 2.15 (br s, 2H), 1.87 (d, *J* = 11.1 Hz, 2H), 1.80-1.68 (m, 8 H), 1.51-1.16 (m, 22H), 1.00- 0.71 (m, 10H). ³¹P{¹H) NMR (162 MHz, CDCl₃): -9.6 (s).

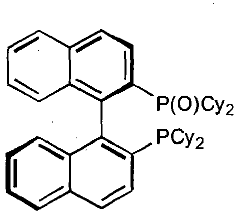

**(*R*)-2-(Dicyclohexylphosphino)-2'-(dicyclohexylphosphinyl)-1,1'-binaphthyl or (*R*)-Cy-BINAP(O).** Grushin's procedure cannot be applied to other biphosphines due to low yield and poor P-monoxide selectivity. The titled compound was prepared according to our modification using a stoichiometric amount of Pdl₂. Under argon, (*R*)-Cy-BINAP (41 mg, 0.063 mmol) and Pdl₂ (33 mg, 0.091 mmol) were stirred in CH₂Cl₂ (5.0 mL) at RT for 6 h. Unreacted Pdl₂ was then filtered off via a syringe filter. To the filtrate was added bis(pmethoxybenzylidene)- acetone (0.28 g, 0.95 mmol) and an aqueous NaOH solution (3.75 M, 2.5 mL). The resulting mixture was stirred at RT for 15 h. The organic layer was then separated and was stirred with dppe (56 mg, 0.14 mmol) at RT for 3 h to removed Pd. The resulting mixture was directly subjected to flash chromatography (CH2Cl2 and then 5:1 CH₂Cl₂/EtOAc) in the argon-filled glove box to give the product in 73% yield (31 mg) as white powder.⁸
¹H NMR (400 MHz, CDCl₃): 7.96 (dd, *J* = 8.6, 1.9 Hz, 1H), 7.89 (ψd, *J* = 8.6 Hz, 2H), 7.85 (d, *J* = 8.1 Hz, 1H), 7.73-7.65 (m, 2H), 7.49-7.45 (m, 1H), 7.38-7.34 (m, 1H), 7.23-7.19 (m, 1H), 7.13-7.06 (m, 2H), 6.97 (d, *J* = 8.4 Hz, 1H), 2.29-2.22 (m, 1H), 2.15-2.07 (m, 1H), 1.97-0.68 (m, 42H). ¹³C NMR (100 MHz, CDCl₃): 145.0 (dd, *J* = 7.1, 5.3 Hz), 144.1 (d, *J* = 3.5 Hz), 143.8 (d, *J* = 3.7 Hz), 135.8 (d, *J* = 17.8 Hz), 134.5 (dd, *J* =10.6, 2.1 Hz), 133.9 (3 peaks), 133.8, 133.3, 130.3 (d, *J* = 2.5 Hz), 129.5 (d, *J* = 2.4 Hz), 128.8, 128.7 (2 peaks), 128.1, 127.9, 127.8 (2 peaks), 127.3, 127.2, 127.1 (3 peaks), 126.9, 126.1, 125.7, 125.1, 38.7 (d, *J* = 64.8 Hz), 37.0 (d, *J* = 65.1 Hz), 35.7 (d, *J* = 16.3 Hz), 33.0 (d, *J* = 15.3 Hz), 32.2 (d, *J* = 18.3 Hz), 31.7 (d, *J* = 16.3 Hz), 30.2 (d, *J* = 14.7 Hz), 29.0 (d, *J* = 7.7 Hz), 27.9, 27.8 , 27.7, 27.6, 27.4 (d, *J* = 9.4 Hz), 26.9 (3 peaks), 26.8 (2 peaks), 26.7, 26.6 (2 peaks), 26.5 (2 peaks), 26.4, 26.2, 26.1. (Not all *J*(13C-31P) couplings were determined).
³¹P{¹H} NMR (162 MHz, CDCl₃): 44.0 (s), -9.4 (s).
ESI-MS: Calcd for C₄₄H₅₆OP₂ [M+H]+: 663.4, Found: 663.5.

### (R)-2-Di(p-tolyl)phosphino-2'-di(p-tolyl)phosphinyl-1,1'-binaphthyl or (R)-Tol-BINAP(O) [(S)-isomer 337529-05-2].³

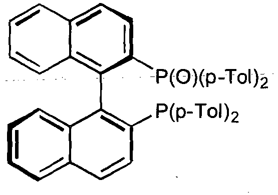

It was prepared from (*R*)-Tol-BINAP similarly according to the procedure described for (*R*)-Cy-BINAP(O). The reaction was set up on 30 mg (0.044 mmol) scale and the product was isolated as a white powder in 70% yield (21 mg). ¹H NMR (400 MHz, CDCl₃): 7.91 (dd, *J* = 8.6, 1.9 Hz, 1H), 7.81 (d, *J* =8.1 Hz, 1H), 7.70-7.65 (m, 3H), 7.44-7.37 (m, 3H), 7.35-7.23 (m, 4H), 7.18-7.14 (m, 2H), 7.00 (d, *J* = 7.1 Hz, 2H), 6.96-6.92 (m, 3H), 6.88-6.86 (m, 3H), 6.82-6.79 (m, 4H), 6.75-6.71 (m, 1H), 6.61 (d, *J* = 8.6 Hz, 1H), 2.28 (s, 3H), 2.26 (s, 3H), 2.23 (s, 6H).
³¹P{¹H} NMR (162 MHz, CDCl₃): 27.3 (s), -17.0 (s).

### (R)-7-diphenylphosphino-7'-diphenylphosphinyl-1,1'-spirobiindane or (R)-SDP(O)[(S)-isomer 528521-82-6].⁴

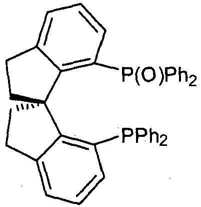

It was prepared from (*R*)-7,7'-diphenylphosphino-1,1'-spirobiindane or (*R*)-SDP using a stoichiometric amount of Pdl₂ as oxidant. All the operations were conducted in an argon-filled glove box. (*R*)-SDP (50 mg, 0.085 mmol) was stirred with Pdl₂ (43 mg, 0.12 mmol) in CH₂Cl₂ (8.0 mL) for 3 h until full conversion of the biphosphine (monitored by ³¹P NMR spectroscopy). Unreacted Pdl₂ was filtered off via a syringe filter. To the filtrate was added dibenzylideneacetone (200 mg, 0.85 mmol) and an aqueous NaOH solution (3.75 M, 4.0 mL). The mixture was stirred at RT for 12 h until full conversion of the Pd(II) complex (monitored by ³¹P{¹H} NMR spectroscopy). The organic phase was separated and was stirred with dppe (75 mg, 0.19 mmol) at RT for 3 h to remove Pd. The solution was then passed through a pad of silica gel, eluted with 10:1 CH₂Cl₂/EtOAc and concentrated under vacuum. Purification of the residue by flash chromatography (20:1 to 10:1 CH₂Cl₂/EtOAc) gave a white solid.
Yield: 33 mg, 64%.
¹H NMR (400 MHz, CDCl₃): 7.47-7.43 (m, 3H), 7.40-7.33 (m, 6H), 7.27-7.19 (m, 7H),
7.16-7.05 (m, 7H), 6.95 (dd, *J* = 7.0, 5.2 Hz, 1H), 6.86-6.82 (m, 2H), 3.02-2.85 (m, 3H), 2.79-2.72 (m, 1H), 2.61 (dd, *J* = 22.0, 9.9 Hz, 1H), 2.09-2.03 (m, 1H), 2.01-1.95 (m, 1H), 1.93-1.85 (m, 1H).
³¹P{¹H} NMR (162 MHz, CDCl₃): 29.4 (s), -18.3 (s).

### (R)-7-Bis(m-xylyl)phosphino-7'-bis(3,5-dimethylphenyl)phosphinyl-1,1'-spirobiindane or (R)-Xyl-SDP(O)[(S)-isomer 528521-85-9].⁴

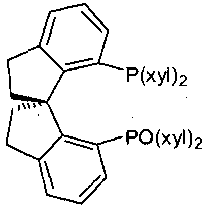

All the operations were conducted in an argon filled glove box. (*R*)-7,7'-bis(*m*-xylyl)phosphino-1,1'-spirobiindane ((*R*)-Xyl-SDP) (100 mg, 0.14 mmol) and Pdl₂ (72 mg, 0.20 mmol) were stirred in CH₂Cl₂ (14 mL) at RT for 8 h, until the bisphosphine was fully converted to the Pdl₂ complex (monitored by ³¹P NMR spectroscopy: 27.4 for Pd complex). Unreacted Pdl₂ was filtered off via a syringe filter. To the filtrate was added dba (334 mg, 1.43 mmol) and an aqueous NaOH solution (3.75 M, 7.0 mL). The mixture was stirred at RT for 15 h, until ³¹P NMR spectroscopy showed full conversion of the Pdl₂ complex. Then, the organic phase was separated and stirred with dppe (113 mg, 0.28 mmol) at RT for 3 h to removed Pd. The solution was then passed through a silica gel pad, eluted with 20:1 to 4:1 CH₂Cl₂/EtOAc. The fractions containing the desired product were concentrated under vacuum and purified as white solid by flash chromatography (20:1 to 4:1 CH₂Cl₂/EtOAc). Yield: 95 mg, 92%. This ligand was also prepared from (*R*)-7,7'-bis(trifluoromethanesulfonyloxy)-1,1'-spirobiindane according to a literature procedure⁴ en route to (*S*)-Xyl-SDP (*vide infra*).
¹H NMR (400 MHz, CDCl₃): 7.38-7.36 (m, 1H), 7.27-7.05 (m, 10H), 6.95 (s, 1H), 6.90 (s, 1H), 6.86 (s, 1H), 6.84 (s, 1H), 6.73 (s, 1H), 6.57 (s, 1H), 6.55 (s, 1H), 2.94-2.91 (m, 2H), 2.87-2.79 (m, 1H), 2.64-2.51 (m, 2H), 2.27 (s, 6H), 2.23 (s, 6H), 2.02 (s, 12H), 1.99-1.93 (m, 2H), 1.74-1.66 (m, 1H).
³¹P{¹H} NMR (162 MHz, CDCl₃): 29.8 (s), -17.3 (s).

### (R)-5-Diphenylphosphino-5'-diphenylphosphinyl-4,4'-bi(1,3-benzodioxole) or (R)-Segphos(O)[(S)-isomer 950189-61-4].⁵

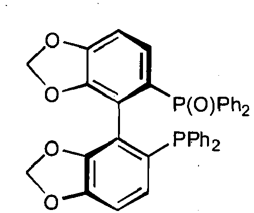

The titled compound was prepared according to our modification of Grushin procedure using a stoichiometric amount of Pdl₂. Under argon, (*R*)-Segphos (50 mg, 0.082 mmol) and Pdl₂ (42 mg, 0.12 mmol) were stirred in CH₂Cl₂ (8.0 mL) for 3 h at RT until full conversion of the bisphosphine, monitored by ³¹P NMR spectroscopy (Pd complex: 16.1). Unreacted Pdl₂ was filtered off via a syringe filter and the filtrate was stirred with bis(p-methoxybenzylidene)acetone (266 mg, 0.90 mmol equiv) and an aqueous NaOH solution (3.75 M, 4.0 mL) at RT for 16 h, until all conversion of the Pd(II) complex, monitored by ³¹P NMR spectroscopy. The organic layer was separated and stirred with dppe (65 mg, 0.16 mmol) at RT for 3 h to remove Pd. The resulting solution was then passed through a pad of silica gel, washed with CH₂Cl₂, eluted by 2:1 CH₂Cl₂/EtOAc and concentrated under vacuum. The product was further purified as pale yellow solid by flash chromatography (CH₂Cl₂ to 1:2 CH₂Cl₂/EtOAc) in an argon-filled glove box.
Yield: 50 mg, 97%.
¹H NMR (400 MHz, CDCl₃): 7.71-7.66 (m, 2H), 7.64-7.59 (m, 2H), 7.46-7.42 (m, 2H), 7.38-7.31 (m, 4H), 7.28-7.22 (m, 10H), 6.98 (dd, *J* = 14.1, 8.1 Hz, 1H), 6.75 (dd, *J* = 8.1, 1.9 Hz, 1H), 6.62 (d, *J* = 8.0 Hz, 1H), 6.55 (d, *J* = 8.0, 3.4 Hz, 1H), 5.70 (d, *J* = 1.6 Hz, 1H), 5.65 (d, *J* = 1.4 Hz, 1H), 5.22 (d, *J* = 1.6 Hz, 1H), 4.82 (d, *J* = 1.4 Hz, 1H).
³¹P{¹H} NMR (162 MHz, CDCl₃): 26.7 (s), -15.0 (s).

### (R)-5-Diphenylphosphino-5'-diphenylphosphinyl-2,2,2',2'-tetrafluoro-4,4'-bi(1,3-benzodioxole) or (R)-difluorphos(O).

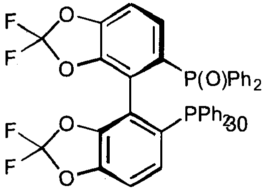

The titled compound was prepared according to our modification of Grushin procedure using a stoichiometric amount of Pdl₂. Under argon, (*R*)-Difluorphos (30 mg, 0.044 mmol) and Pdl₂ (23 mg, 0.064 mmol) were stirred in CH₂Cl₂ (4.0 mL) at RT for 3 h until full conversion to the Pdl₂ complex, monitored by ³¹P NMR spectroscopy (Pdl₂ complex: 15.4). Unreacted Pdl₂ was filtered off via a syringe filter and the filtrate was stirred with dba (155 mg, 0.66 mmol) and an aqueous NaOH solution (3.75 M, 1.5 mL) at RT for 15 h. The organic layer was separated and stirred with dppe (53 mg, 0.13 mmol) at RT for 3 h to removed Pd. The resulting solution was then passed through a pad of silica gel, washed with CH2Cl2, eluted by 2:1 CH₂Cl₂/EtOAc. The fractions containing the product were concentrated under vacuum and further purified by flash chromatography (CH₂Cl₂ and then 1:1 CH₂Cl₂/EtOAc) in an argon-filled glove box, to afford white solid (29 mg, 71%).
¹H NMR (400 MHz, CDCl₃): 7.59-7.54 (m, 2H), 7.52-7.46 (m, 3H), 7.44-7.36 (m, 3H), 7.34-7.22 (m, 10H), 7.20-7.16 (m, 2H), 7.12 (dd, *J* = 13.1, 8.3 Hz, 1H), 7.06 (dd, *J* = 8.3, 2.2 Hz, 1H), 6.95 (d, *J* = 8.3 Hz, 1H), 6.87 (dd, *J* = 8.2, 3.1 Hz, 1H).
¹³C NMR (100 MHz, CDCl₃): 145.6 (d, *J* = 2.3 Hz), 143.9, 143.6 (d, *J* = 16.1), 142.4 (d, *J* = 12.2 Hz), 137.1 (d, *J* = 12.3 Hz), 136.5 (d, *J* = 12.2), 134.6 (d, *J* = 15.0 Hz), 134.0 (2 peaks), 133.9 (d, *J* = 4.6 Hz), 133.8, 133.6, 133.4, 133.3, 133.0, 132.3, 132.2, 132.0, 131.9 (2 peaks), 131.8, 131.3 (d, *J* = 6.3 Hz), 130.5 (d, *J* = 13.0 Hz), 130.3 (d, *J* = 2.2 Hz), 129.0, 128.8 (d, *J* =8.7 Hz), 128.6, 128.5 (2 peaks), 128.4 (2 peaks), 128.3, 128.1, 127.0, 121.2-121.0 (m), 120.7 (d, *J* = 3.0 Hz), 120.3 (d, *J* = 3.2 Hz), 110.3, 109.2 (d, *J* = 14.8 Hz).
¹⁹F{¹H) NMR (376 MHz, CDCl₃): -48.2 (d, *J* = 94.2 Hz), -49.2 (d, *J* = 90.0 Hz), -49.6 (d, *J* = 90.4 Hz), -50.1 (d, *J* = 94.1 Hz).
³¹P{¹H} NMR (162 MHz, CDCl₃): 27.7 (s), -15.0 (s).
ESI-MS: Calcd for C₃₈H₂₄F₄O₅P₂ [M+H]+: 699.1, Found: 699.1.

### (R)-7,7'-bis(trifluoromethanesulfonyloxy)-1,1'-spirobiindane [528521-72-4].⁴

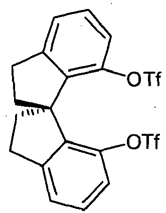

(*R*)-1,1'-spirobiindane-7,7'-diol (1.00 g, 3.96 mmol), dry CH2Cl2 (16.0 mL) and analytical-grade pyridine (1.28 mL, 16.0 mmol) were charged into a 100-mL Schlenk flash under argon. The solution was cooled to 0 oC in an ice/water bath, followed by dropwise addition of triflic anhydride (1.47 mL, 8.72 mmol) via a syringe. The mixture was allowed to warm up to RT and stirred overnight. The resulting mixture was directly subjected to flash chromatography (10:1 hexane/EtOAc), which afforded 1.99 g of the product (97%) as a white solid.
¹H NMR (400 MHz, CDCl₃): 7.32-7.27 (m, 4H), 7.17-7.13 (m, 2H), 3.17-3.05 (m, 4H), 2.41-2.28 (m, 4H).
¹⁹F{¹H} NMR (376 MHz, CDCl₃): -75.0 (s).

### Bis(m-xylyl)phosphine oxide [187344-92-9].

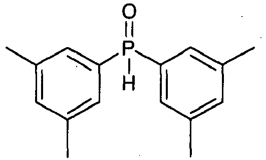

It was prepared according to a modified procedure.⁶ Under argon, finely ground LiCl (1.59 g, 37.5 mmol) was dissolved in dry THF (75 mL) in a 250-mL Schlenk flask. Magnesium turnings (0.73 g, 30.0 mmol) were added to this solution, followed by addition of diisobutylaluminum hydride (0.30 mL, 1-M in cyclohexane). The mixture was stirred for 5 min at RT, followed by addition of 1-bromo-3,5- dimethylbenzene (5.55 g, 30.0 mmol). The stirring was continued for another 6 h at RT until all magnesium turnings were consumed. The resulting solution was cooled to -10°C in a salt/ice bath, followed by the addition of HP(O)(OEt)₂ (1.38 g, 10.0 mmol) in THF (2.0 mL). The mixture was allowed to warm up to RT and the stirring was continued for 18 h. The reaction was quenched by a saturated aqueous NH4Cl solution (100 mL), followed by extraction with EtOAc (100 mL x 2). The organic extracts were combined and dried over MgSO₄, and concentrated on a rotary evaporator. The residue was purified by flash chromatography (hexane to 1:2 EtOAc/hexane), which afforded 2.35 g (90%) of the product as white powder.
¹H NMR (400 MHz, CDCl₃): 7.95 (d, *J* = 478.9 Hz, 1H), 7.31 (d, *J* = 14.2 Hz, 4H), 7.18(s, 2H), 2.35 (s, 12H).
³¹P{¹H} NMR (162 MHz, CDCl₃): 22.6 (s).

### (R)-7-Bis(3,5-dimethylphenyl)phosphinyl-7'-trifluoromethanesulfonyloxy-1,1'-spirobiindane [(S)-isomer: 528521-77-9].⁴

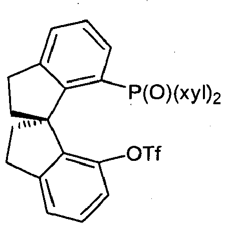

It was prepared according to the procedure for the (*S*)-isomer.⁴ Under argon, (*R*)-1,1'-spirobiindane 7,7'-bistriflate (0.516 g, 1.0 mmol), bis(3,5- dimethylphenyl)phosphine oxide (0.517 g, 2.0 mmol), Pd(OAc)₂ (11.2 mg, 0.05 mmol), 1,4-bis(diphenylphosphino)butane (21.3 mg, 0.05 mmol), *N-*ethyldiisopropylamine (0.68 mL, 4.0 mol) and dry DMSO (3.3 mL) were charged into a dry 25-mL Schlenk tube and the mixture was stirred in a 100°C bath for 21 h. After cooled to RT, the reaction was quenched by addition of 1 M HCl (30 mL), followed by extraction with CH₂Cl₂ (20 mL x 3). The combined organic extracts were washed with a saturated NaHCO3 solution (10 mL), dried over MgSO₄, and concentrated on a rotary evaporator. The residue was purified by flash chromatography (3:1 hexane/EtOAc), which afforded 0.44 g (70%) of the product as white powder.
¹H NMR (400 MHz, CDCl₃): δ: 7.39 (d, *J* = 7.4 Hz, 1H), 7.19 (d, *J* = 7.7 Hz, 1H), 7.16 (dd, *J* = 7.5, 2.2 Hz, 1H), 7.09 (s, 1H), 7.06-6.98 (m, 5H), 6.86 (s, 1H), 6.83 (s, 1H), 6.26 (d, *J* = 8.2 Hz, 1H), 3.42-3.27 (m, 2H), 3.14-2.98 (m, 3 H), 2.35-2.30 (m, 2H), 2.25 (s, 6H, CH₃), 2.23 (s, 6H, CH₃), 2.20-2.17 (m, 1H).
¹⁹F{¹H} NMR (376 MHz, CDCl₃): *δ*: -75.2 (s).
³¹P{¹H} NMR (162 MHz, CDCl₃): *δ*: 30.3 (s).

### (R)-7-Bis(3,5-dimethylphenyl)phosphino-7'-trifluoromethanesulfonyloxy-1,1'-pirobiindane[(S)-isomer: 528521-81-5].⁴

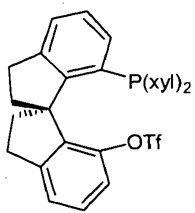

It was prepared based on a modification of a reported procedure for the (*S*)-isomer.⁴ Under argon, (*R*)-7-bis(3,5-dimethylphenyl)phosphinyl-7-trifluoromethanesulfonyloxy-1,1'-spirobiindane (465 mg, 0.74 mmol), *N*ethyldiisopropylamine (5.1 mL, 30 mmol) and dry toluene (7.4 mL) were mixed in a dry 50-14 mL Schlenk tube. The solution was cooled to 0°C and HSiCl₃ (1.2 mL, 12 mmol) was then added. The mixture was stirred rigorously in a 120 °C oil bath for 36 h and ³¹P NMR spectroscopy of the reaction mixture showed full conversion of phosphine oxide. The reaction was quenched by dilution with hexane (20 mL) and a saturated Na₂CO₃ solution (2.5 mL) under argon. The organic layer was passed through a pad of silica gel with Et₂O washings. Removal of solvent under vacuum furnished 409 mg (90%) of the product as a white solid.
¹H NMR (400 MHz, CDCl₃): 7.25-7.20 (m, 2H), 7.17-7.12 (m, 2H), 7.00 (dd, *J* = 7.3, 4.6 Hz, 1H), 6.90 (s, 1H), 6.84 (s, 1H), 6.69 (d, *J* = 8.1 Hz, 1H), 6.65-6.61 (m, 4H), 3.10-3.03 (m, 4H), 2.59-2.51 (m, 1H), 2.37-2.22 (m, 3H), 2.19 (s, 6H), 2.17 (s, 6H).
¹⁹F NMR (376 MHz, CDCl₃): -75.1 (s).
³¹P{1H} NMR (162 MHz, CDCl₃): -21.7 (s).

### (R)-7-Bis(3,5-dimethylphenyl)phosphino-7'-bis(m-xylyl)phosphinyl-1,1'-spirobiindane. CAS for (S)-isomer: 528521-85-9.⁴

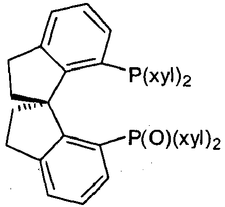

It was prepared according to a reported procedure of the (*S*)- isomer.⁴ Under argon, (*R*)-7-bis(3,5-dimethylphenyl)phosphino-7'-trifluoromethanesulfonyloxy-1,1'-spirobiindane (367 mg, 0.60 mmol), bis(3,5-dimethylphenyl)phosphineoxide (312 mg, 1.21 mmol), Pd(OAc)₂ (6.8 mg, 0.030 mmol), 1,4-bis(diphenylphosphino)-butane (13 mg, 0.030 mmol), *N*-ethyldiisopropylamine (0.41 mL, 2.4 mmol) and dry DMSO(4.2 mL) were charged into a dry 25-mL Schlenk tube. The mixture was stirred in a 100°C bath for 18 h, until ³¹P NMR spectroscopy of the reaction mixture showed full conversion of the triflate. The reaction was quenched by dilution with EtOAc (20 mL) and a saturated Na₂CO₃ solution (5 mL). The organic layer was separated and concentrated under vacuum. Purification by flash chromatography (CH₂Cl₂ to 20:1 CH₂Cl₂/EtOAc) under argon afforded 354 mg (82%) of the product as a white powder.
¹H NMR (400 MHz, CDCl₃): 7.38-7.36 (m, 1H), 7.27-7.05 (m, 10H), 6.95 (s, 1H), 6.90 (s, 1H), 6.86 (s, 1H), 6.84 (s, 1H), 6.73 (s, 1H), 6.57 (s, 1H), 6.55 (s, 1H), 2.94-2.91 (m, 2H), 2.87-2.79 (m, 1H), 2.64-2.51 (m, 2H), 2.27 (s, 6H), 2.23 (s, 6H), 2.02 (s, 12H), 1.99-1.93 (m, 2H), 1.74-1.66 (m, 1H).
³¹P{¹H} NMR (162 MHz, CDCl₃): 29.8 (s), -17.3 (s).

### (R)-7-dicylcohexylphosphinyl-7'-bis(3,5-dimethylphenyl)phosphino-1,1'-spirobiindane.

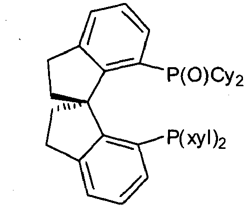

It was prepared following a similar procedure of those symmetrically substituted analogs with HP(O)Cy₂. Under argon, (*R*)-7-bis(3,5-dimethylphenyl)phosphino-7'-trifluoromethanesulfonyloxy-1,1'-spirobiindane (140 mg, 0.23 mmol), dicyclohexylphosphine oxide (100 mg, 0.47 mmol), Pd(OAc)₂ (10.3 mg, 0.046 mmol), 1,4-bis(diphenylphosphino)butane (19.6 mg, 0.046 mmol), *N-*ethyldiisopropylamine (0.16 mL, 0.92 mmol) and dry DMSO (1.6 mL) were charged into a dry 25-mL Schlenk tube. The mixture was stirred in a 100°C bath for 40 h. The reaction was quenched by dilution with EtOAc (10 mL) and a saturated Na₂CO₃ solution (4 mL). The organic layer was separated and concentrated under vacuum. Purification by flash chromatography (CH₂Cl₂ to 20:1 CH₂Cl₂/EtOAc) under argon afforded 113 mg (73 %) of the product as a white powder.
¹H NMR (400 MHz, CDCl₃): 7.33-7.29 (m, 2H), 7.26-7.18 (m, 2H), 7.14-7.11 (m, 1H), 7.08-7.05 (m, 1H), 6.84 (br s, 2H), 6.70 (d, *J* = 7.6 Hz, 2H), 6.57 (d, *J* = 7.3 Hz, 2H), 3.29- 3.19 (m, 2H), 3.06-2.95 (m, 1H), 2.92-2.83 (m, 1H), 2.66-2.59 (m, 1H), 2.18 (s, 6H), 2.17 (s, 6H), 2.15-1.94 (m, 3H), 1.91-0.75 (m, 22H).
¹³C NMR (100 MHz, CDCl₃): 158.1 (d, *J* = 251.4 Hz), 156.1 (m), 148.0 (d, *J* = 3.9 Hz), 147.9 (d, *J* = 4.0 Hz), 145.2 (d, *J* = 7.7 Hz), 139.9 (d, *J* = 12.8 Hz), 138.0 (d, *J* = 15.5 Hz), 137.2, 137.1, 132.8, 132.6, 131.9, 131.7, 131.5, 130.8 (d, *J* = 21.6 Hz), 130.3, 129.9 (d, *J* = 3.3 Hz), 129.8, 129.4 (d, *J* =12.3 Hz), 129.1 (d, *J* = 3.2 Hz), 126.8 (d, *J* = 2.8 Hz), 126.4, 126.0 (d, *J* = 11.8 Hz), 125.2, 64.3, 42.4, 41.1 (d, *J* = 4.8 Hz), 38.6 (d, *J* = 66.1 Hz), 38.3 (d, *J* = 64.6 Hz), 31.9, 31.4, 27.0, 26.9, 26.8, 26.7, 26.6, 26.5, 26.4, 26.3, 26.2 (d, *J* = 2.6 Hz), 26.1, 26.0, 25.8 (d, *J* = 3.0 Hz), 21.5, 21.4.
³¹P{¹H} NMR (162 MHz, CDCl₃): 44.0 (s), -9.4 (s).
ESI-MS: Calcd for C₄₅H₅₅OP₂ [M+H]+: 673.4, Found: 673.4.

### Di(p-fluorophenyl)phosphine oxide [94940-35-9].⁷

It was prepared according to a modified procedure.6 Under argon, finely ground LiCl (1.59 g, 37.5 mmol) was dissolved in dry THF (75 mL) in a 250-mL Schlenk flask. Magnesium turnings (0.73 g, 30.0 mmol) and a solution of diisobutylaluminium hydride solution in cyclohexane (0.30 mL, 1 M) were then added. The mixture was stirred for 10 min at RT, followed by addition of 3,5-dimethylbromobenzene (5.55 g, 30.0 mmol). The stirring was continued for another 2 h at RT until all magnesium turnings were consumed. The resulting solution was cooled to -10 oC in a salt/ice bath, followed by the addition of a solution of HP(O)(OEt)₂ (1.38 g, 10.0 mmol) in THF (2.0 mL). The mixture was allowed to warm up to RT and was stirred for 18 h. At the end of the reaction, it was quenched by 3 M HCl (50 mL) and extracted with EtOAc (100 mL x2). The organic extracts were combined and dried over MgSO₄, concentrated on a rotary evaporator. Purification of the residue by flash chromatography (10:1 hexane/EtOAc to EtOAc) afforded 1.97 g (83 %) of the product as a pale yellow liquid.
1H NMR (400 MHz, CDCl₃): 8.09 (d, *J* = 484.8 Hz, 1H), 7.74-7.67 (m, 4H), 7.25-7.19 (m, 4H). ¹⁹F{1H} NMR (100 MHz, CDCl₃): -105.0
³¹P{1H} NMR (162 MHz, CDCl₃): 18.7 (s).

### (R)-7-Di(p-fluorophenyl)phosphinyl-7'-trifluoromethanesulfonyloxy-1,1'-spirobiindane.

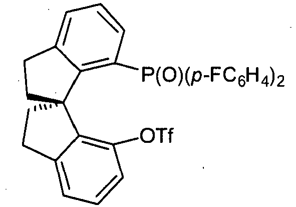

Under argon, (*R*)-7,7'-bis(trifluoromethanesulfonyloxy)-1,1'-spirobiindane (516 mg, 1.00 mmol), di(*p-*fluorophenyl)phosphine oxide (520 mg, 2.18 mmol), Pd(OAc)₂ (45 mg, 0.20 mmol), 1,4-bis(diphenylphosphino)butane (85 mg, 0.20 mmol), *N*-ethyldiisopropylamine (0.68 mL, 4.0 mmol) and dry DMSO (3.3 mL) were charged sequentially into a dry 25-mL Schlenk tube. The mixture was capped tightly and stirred in a 100 °C bath for 36 h. After cooled to RT, the solution was diluted with EtOAc (10 mL), washed sequentially with 1M HCl (5 mL) and a saturated NaHCO₃ solution (5 mL), dried over MgSO₄ and concentrated on a rotary evaporator. Purification of the residue by flash chromatography (hexane to 1:2 hexane/EtOAc) afforded 454 mg (75%) of the product as pale yellow powder.
¹H NMR (400 MHz, CDCl₃): 7.45 (d, *J* =7.5 Hz, 1H), 7.37-7.25 (m, 4H), 7.21-7.17 (m, 2H), 7.06-7.01(m, 5H), 6.93 (dd, *J* = 14.3, 7.6Hz, 1H), 6.33 (d, *J* = 8.2 H, 1H), 3.34 (dd, *J* = 15.4, 9.3 Hz, 1H), 3.22 (dd, *J* = 20.7, 9.5 Hz, 1H), 3.16-3.03 (m, 3H), 2.39-2.32 (m, 2H), 2.28-2.17 (m, 1H).
¹³C NMR (100 MHz, CDCl₃): 166.2 (dd, *J* = 34.1, 3.3 Hz), 163.7 (dd, *J* = 34.4, 3.2 Hz), 153.4 (d, *J* =7.1), 150.0, 146.6 (d, *J* = 10.1 Hz), 145.1, 141.0, 134.3 (d, *J* = 8.6 Hz), 134.2 (d, *J* = 8.6 Hz), 134.0 (d, *J* = 8.7 Hz), 133.8 (d, *J* = 8.9 Hz), 133.3 (d, *J* = 13.6 Hz), 132.0 (dd, *J* = 107.9, 3.4 Hz), 128.6 (m, 2C), 127.4 (dd, *J* = 105.0, 3.3 Hz), 126.9 (d, *J* = 102.2 Hz), 126.4 (d, *J* = 13.0 Hz), 124.0, 117.9 (q, *J* = 319.6), 117.5, 115.9-115.5 (m, 4C), 62.0 (d, *J* = 2.0 Hz), 40.2, 40.1, 32.0, 30.9.
¹⁹F{¹H} NMR (376 MHz, CDCl₃): -75.1 (s), -107.5 (s), -107.6 (s).
³¹P{¹H} NMR (162 MHz, CDCl₃): 29.2 (s).
ESI-MS: Calcd for C₃₀H₂₂F₅O₄PS [M+H]+: 605.1, Found: 605.1.

### (R)-7-Di(p-fluorophenyl)phosphino-7'-trifluoromethanesulfonyloxy-1,1'-spirobiindane.

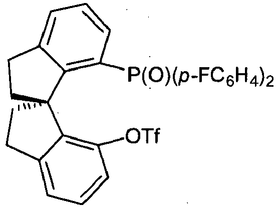

The entire operation including filtration was performed in an argon-filled glove box. (*R*)-7-Di(pfluorophenyl) phosphinyl-7'-trifluoromethanesulfonyloxy-1,1'-spirobiindane (320 mg, 0.53 mmol), *N*-ethyldiisopropylamine (3.62 mL, 21 mmol) and dry toluene (5.3 mL) were charged into a dry 25-mL Schlenk tube. The solution was cooled to 0°C and then HSiCl₃ (0.86 mL, 8.5 mmol) was added. The resulting mixture was stirred rigorously in a 120 °C bath for 42 h, until ³¹P NMR spectroscopy showed >95% conversion of the phosphine oxide. The reaction mixture was then diluted with hexane and saturated NaHCO₃ solution (-1.5 mL). The organic layer was directly loaded onto a silica gel pad and eluted with Et2O. Removal of the solvent under vacuum afforded 280 mg (90%) of the pure product as a white powder.
¹H NMR (400 MHz, CDCl₃): 7.28 (d, *J* = 7.4 Hz, 1H), 7.22 (dd, *J* = 7.5, 0.7 Hz, 1H), 7.16 (dd, *J* = 16.5, 7.6 Hz, 2H), 7.03-6.97 (m, 2H), 6.96-6.86 (m, 7H), 6.68 (d, *J* =8.1 Hz, 1H), 3.16-.301 (m, 4H), 2.54-2.46 (m, 1H), 2.38-2.23 (m, 3H).
¹³C NMR (100 MHz, CDCl3): 164.6 (d, *J* = 37.1 Hz), 162.1 (d, *J* =36.9), 152.8 (d, *J* =25.6 Hz), 148.1 (d, *J* = 2.8 Hz), 145.7 (d, *J* = 2.4 Hz), 144.2 (d, *J* = 7.9 Hz), 142.0 (d, *J* = 3.7 Hz), 135.7 (d, *J* = 8.0 Hz), 135.5 (d, *J* = 8.1 Hz), 135.2 (d, *J* = 7.9 Hz), 135.0 (d, *J* = 7.8 Hz), 134.1-134.0 (m, 2C), 132.1 (d, *J* = 19.2 Hz), 131.5 (dd, *J* = 11.9, 3.4 Hz), 129.0, 127.8, 126.0, 124.3, 118.4, 118.0 (q, *J* = 319.7 Hz), 115.8 (d, *J* = 7.9 Hz), 115.6 (d, *J* = 7.6Hz), 115.5 (d, *J* = 6.8 Hz), 115.4 (d, *J* = 7.7 Hz), 61.7 (d, *J* = 3.3 Hz), 40.2 (d, *J* = 5.9 Hz), 39.2, 31.6, 30.9.
¹⁹F{¹H} NMR (376 MHz, CDCl₃): -75.1 (s), -112.8 (d, *J* = 4.3 Hz), -113.2 (d, *J* = 5.4 Hz).
³¹P{¹H} NMR (162 MHz, CDCl₃): -23.8 (s).
ESI-MS: Calcd for C₃₀H₂₂F₅O₃PS [M+H]+: 589.1, Found: 589.1.

### (R)-7-Dicylcohexylphosphinyl-7'-di(p-fluorophenyl)phosphino-1,1'-spirobiindane, R)-pFPh-Cy-SDP(O).

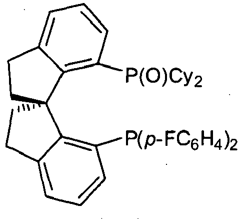

The entire operation including flash chromatography was performed in an argon-filled glove box. (*R*)-7-Di(4-fluorophenyl)phosphino-7'-trifluoromethanesulfonyloxy- 1,1'-spirobiindane (430 mg, 0.73 mmol), dicyclohexylphosphine oxide (313 mg, 1.46 mmol), Pd(OAc)2 (32 mg, 0.14 mmol), 1,4-bis(diphenylphosphino)butane (61 mg, 0.14 mmol), *N*ethyldiisopropylamine (0.50 mL, 2.9 mmol) and dry DMSO (5.10 mL) were charged into a dry 25-mL Schlenk tube. The mixture was tightly capped and stirred vigorously in a 100 oC bath for 20 h. Upon cooled to RT, the solution was diluted with EtOAc (20 mL), washed sequentially with 1 M HCl (5 mL) and a saturated NaHCO3 solution (5 mL), and concentrated under vacuum. Purification of the residue by flash chromatography (CH2CI2 to 10:1 CH2CI2/EtOAc) furnished 414 mg (87%) of the pure product as white foam.
¹H NMR (400 MHz, CDCl₃): 7.35-7.28 (m, 3H), 7.25-7.20 (m, 1H), 7.11-7.07 (m, 1H), 7.04-6.99 (m, 2H), 6.96-6.88 (m, 7H), 3.30-3.19 (m, 2H), 3.03-2.86 (m, 2H), 2.69-2.63 (m, 1H), 2.16-2.04 (m, 2H), 1.90-0.76 (m, 23H).
¹³C NMR (100 MHz, CDCl₃): 164.4 (d, *J* = 8.0 Hz), 161.9 (d, *J* = 7.7 Hz), 158.1 (d, *J* = 25.5 Hz), 155.9 (3 peaks), 147.7 (dd, *J* = 9.0, 4.1 Hz), 145.9 (d, *J* = 7.7 Hz), 136.7 (dd, *J* = 22.8, 7.9 Hz), 135.44 (dd, *J* =21.5, 7.8 Hz), 135.39 (d, *J* = 13.7 Hz), 133.5 (dd, *J* = 15.9, 3.3 Hz), 131.4 (d, *J* = 1.4 Hz), 129.8, 129.7, 129.6 (2 peaks), 129.5, 128.9, (d, *J* = 3.2 Hz), 126.8 (d, *J* = 2.6 Hz), 126.7, 126.2 (d, *J* = 11.7 Hz), 125.7, 115.5 (d, *J* = 7.3 Hz), 115.4 (d, *J* = 5.6 Hz), 115.3 (d, *J* = 5.9 Hz), 115.2 (d, *J* = 7.2 Hz), 64.2 (3 peaks), 42.3, 41.1 (d, *J* = 4.4 Hz), 38.9 (d, *J* = 21.5 Hz), 38.2 (d, *J* = 20.0 Hz), 31.9, 31.3 (d, *J* = 1.3 Hz), 27.0, 26.8 (2 peaks), 26.7, 26.6, 26.5 (2 peaks), 26.4, 26.2 (2 peaks), 26.0 (3 peaks), 25.9, 25.8 (2 peaks). Not all coupling was assigned.
¹⁹F{¹H} NMR (376 MHz, CDCl₃): -113.1
³¹P{¹H) NMR (162 MHz, CDCl₃): 45.7(s), -20.4(s).
ESI-MS: Calcd for C₄₁H₄₄F₂OP₂ [M+H]+: 653.3, Found: 653.3.

### II. EXAMPLES OF ASYMMETRIC HECK REACTIONS USING VARIOUS PHOSPHOROUS LIGANDS AND OLEFIN BEARING TRIFLATE

In an argon-filled glove-box, Pd(dba)₂ (1.4 mg, 2.5 mol%) and chiral ligand (3.0 mol%) were stirred in dioxane (0.10 mL) in a 4-mL reaction tube for 15 min, followed by addition of *p-*ethoxycarbonylphenyl triflate (29.8 mg, 0.1 mmol), cyclopentene (27 mg, 0.4 mmol), *i*Pr₂NEt (26 mg, 0.2 mmol) and *n*-C₁₄H₃₀ (5 *µ*L, GC internal standard). The tube was capped and the reaction was stirred in an oil bath at 70 oC. Aliquotes were taken after stirring for 4 h and 16 h for GC and chiral HPLC analysis. The conversion of the triflate and yield of the Heck isomers were determined by GC. The ee of the major Heck product was determined by chiral HPLC (Daicel chiralcel OJ-H, 98:2 hexanes/isopropanol).

**Table 1: Model reaction after 4 h and 16 h**

| Entry | Ligands | Time | Conv. (%) | Prod. (%) | ArH (%) | Olefinic selectivity | Ee (%) |
|---|---|---|---|---|---|---|---|
| 1 | (*R*)-BINAP(O) | 4 h | 82 | 45 | 27 | 14:1 | 88 |
| | | 16 h | 100 | 57 | 35 | 15:1 | 88 |
| 2 | (*R*)-Tol-BINAP(O) | 4 h | 53 | 26 | 20 | 19:1 | 87 |
| | | 16 h | 94 | 42 | 34 | 22:1 | 86 |
| 3 | (*R*)-Cy-BINAP(O) | 4 h | 28 | 16 | 8 | 142:1 | 97 |
| | | 16 h | 47 | 26 | 16 | 82:1 | 96 |
| 4 | (*R*)-Segphos(O) | 4 h | 44 | 12 | 21 | 25:1 | 85 |
| | | 16 h | 85 | 23 | 43 | 22:1 | 80 |
| 5 | (*R*)-Difluoruphos(O) | 4 h | 50 | 18 | 24 | 44:1 | 81 |
| | | 16 h | 100 | 33 | 50 | 47:1 | 78 |
| 6 | (*R*)-SDP(O) | 4 h | 99 | 60 | 27 | 14:1 | -88 |
| | | 16 h | 100 | 57 | 23 | 13:1 | -88 |
| 7 | (*R*)-Xyl-SDP(O) | 4 h | 100 | 83 | 15 | 26:1 | -98 |
| 8 | (*R*)-Xyl-Cy-SDP(O) | 4 h | 97 | 77 | 8 | 23:1 | -85 |
| | | 16 h | 100 | 77 | 8 | 17:1 | -84 |
| 9 | (R)-*p*FPh-Cy-SDP(O) | 4 h | 41 | 33 | 2 | 27:1 | -78 |
| | | 16 | 68 | 54 | 5 | 21:1 | -76 |

### A. Examples of asymmetric Heck reaction of cyclic olefins

### General procedure

In an argon-filled glove box, Pd(dba)₂ (7.2 mg, 0.013 mmol) and (*R*)-Xyl-SDP(O) (10.8 mg, 0.015 mmol) were stirred in dry 1,4-dioxane (0.50 mL) for 10 to 20 min in a 4-mL vial, followed by addition of *n*-C₁₄H₃₀ (25 *µ*L, GC internal standard), aryl triflate (0.50 mmol), *N-*ethyldiisopropylamine (170 *µ*L, 1.0 mmol, 2 equiv) and cyclic olefin (2.0 mmol, 4 quiv). The mixture was vigorously stirred in a preheated oil bath at a set temperature until the aryl triflate was fully consumed (monitored by GC). The reaction mixture was cooled to RT and subjected to flash chromatography (basic alumina, Brockmann grade I, pentane/Et₂O) to give the purified product. Silica gel may be also used for purification. The olefinic selectivity of Heck isomers in the crude mixture was determined by GC. The ee of the purified product was determined by chiral HPLC analysis with Daicel Chiralcel columns or by chiral GC analysis. Racemic products were prepared using the racemic ligand to facilitate determination of ee.

### (S)-3-(1-Naphthyl)cyclopentene [racemate: 90173-55-0]⁸

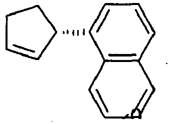

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 1-naphthyl triflate (140 mg, 0.51 mmol), *N*-ethyldiisopropylamine (170 *µ*L, 1.0 mmol) and cyclopentene (180 *µ*L, 2.0 mmol) were used. The reaction completed in 2.5 h at 50°C. The product was isolated by flash chromatography (pentane) as colorless oil. Yield: 94 mg, 95%. Olefinic selectivity in the crude product: 17:1. [α]²⁰_{D} = +116° (c = 1.5, CHCl₃).
Ee: 99%. Daicel Chiralcel OJ-H, 98:2 n-hexane/isopropanol, flow rate = 0.5 mL/min. TR = 13.1 min (major) and 14.6 min (minor). The middle signal in the second trace was the olefin isomer.
¹H NMR (400 MHz, CDCl₃): 8.16 (d, *J* = 8.3 Hz, 1H), 7.87-7.83 (m, 1H), 7.71 (d, *J* = 8.1 Hz, 1H), 7.54-7.46 (m, 2H), 7.42-7.38 (m, 1H), 7.31 (dd, *J* = 7.1, 1.0 Hz, 1H), 6.08-6.05 (m, 1H), 5:96-5.93 (m, 1H), 4.69-4.64 (m, 1H), 2.67-2.58 (m, 1H), 2.55-2.43 (m, 2H), 1.83-1.74 (m, 1H).

### (S)-3-(2-Naphthyl)cyclopentene [racemate 92425-28-0]⁹

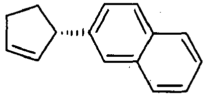

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 2- naphthyl triflate (140 mg, 0.51 mmol), *N*-ethyldiisopropylamine (170 *µ*L, 1.0 mmol) and cyclopentene (180 *µ*L, 2.0 mmol) were used. The reaction completed in 1 h at 50 oC. The product was isolated by flash chromatography with pentane as colorless oil.
Yield: 94 mg, 95%. Olefinic selectivity in the crude product: 11:1.
Ee: 94%. Daicel Chiralcel OJ-H, 98:2 hexanes/isopropanol, flow rate = 0.5 mL/min. TR = 18.5 min (major) and 20.4 min (minor).
¹H NMR (400 MHz, CDCl₃): 7.81-7.77 (m, 3H), 7.62 (s, 1H), 7.46-7.39 (m, 2H), 7.33 (dd, *J* = 8.5, 1.7 Hz, 1H), 6.02-5.99 (m, 1H), 5.87-5.83 (m, 1H), 4.10-4.04 (m, 1H), 2.60-2.41 (m, 3H), 1.86-1.76 (m, 1H).

### (S)-3-Phenylcyclopentene [175274-02-9]¹⁰.

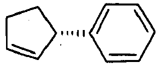

Pd(dba)2 (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), phenyl triflate (140 mg, 0.51 mmol), *N*-ethyldiisopropylamine (170 *µ*L, 1.0 mmol) and cyclopentene (180µL, 2.0 mmol) were used. The reaction completed in 13 h at 50 oC. The product was isolated by flash chromatography (silica gel, pentane) as colorless oil. Yield: 53 mg, 73% (92% GC yield).
Olefinic selectivity in the crude product: 38:1.
[α]²⁰_{D} = -210° (c = 1.3, CHCl3). Lit. value: -212°.^{10a,10b}
Ee: 98%. Daicel Chiralcel OJ-H, 98:2 hexane/isopropanol, flow rate = 0.5 mL/min. TR = 12.6 min (major) and 13.4 min (minor).
¹H NMR (400 MHz, CDCl₃): 7.31-7.27 (m, 2H), 7.20-7.17 (m, 3H), 5.95-5.93 (m, 1H), 5.79-5.77 (m, 1H), 3.90-3.88 (m, 1H), 2.53-2.36 (m, 3H), 1.77-1.69 (m, 1H).

### (S)-3-[4-(Ethoxycarbonyl)phenyl]cyclopentene [racemate 115419-30-2]^{11.}

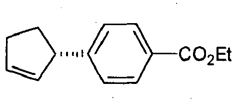

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p-*(ethoxycarbonyl)phenyl triflate (150 mg, 0.50 mmol), *N-*ethyldiisopropylamine (170 *µ*L, 1.0 mmol) and cyclopentene (180 *µ*L, 2.0 mmol) were used. The reaction was complete 23 in 17 h at 50 oC. The product was isolated by flash chromatography with pentane/Et₂O (10:1 to 5:1) as colorless oil. Yield: 99 mg, 91%. Olefinic selectivity in the crude product: 41:1. Ee: 98%. Daicel Chiralcel OJ-H, 98:2 n-hexane/isopropanol, flow rate = 0.5 mL/min. TR = 18.3 min (major) and 31.0 min (minor).
¹H NMR (400 MHz, CDCl₃): 7.96 (d, *J* = 8.3 Hz, 2H), 7.25 (d, *J* = 8.3 Hz, 2H), 5.99-5.97 (m, 1H), 5.78-5.75 (m, 1H), 4.36 (q, *J* = 7.1 Hz, 2 H), 3.97-3.92 (m, 1H), 2.55-2.38 (m, 3H), 1.75-1.68 (m, 1H), 1.38 (t, *J* = 7.1 Hz, 3H).
¹³C NMR (100 MHz, CDCl₃): 166.8, 152.0, 133.6, 132.8, 129.9, 128.5, 127.3, 60.9, 51.5, 33.8, 32.6, 14.5.

### (S)-3-(4-Methoxyphenyl)cyclopentene. CAS of (-)-Isomer 38806-00-7.¹² Pd(dba)₂ (7.2 mg,

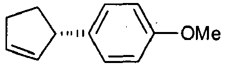

0.013 mmol), (*R*)-Xyl-SDP(O) (10.8 mg, 0.015 mmol), 1,4-dioxane (0.50 mL), *n*-C₁₄H₃₀ (25 *µ*L), *p*-anisyl triflate (128 mg, 0.50 mmol), *N-*ethyldiisopropylamine (170 *µ*L, 1.0 mmol) and cyclopentene (180 *µ*L, 2.0 mmol) were used. The reaction was complete in 21 h at 70°C. The product was isolated by flash chromatography with pentane/Et₂O (10:0 to 10:1) as a colorless liquid. Yield: 73 mg, 84%. Olefinic selectivity in the crude product: 10:1. Ee: 94%. Daicel Chiralcel OJ-H, 98:2 hexanes/isopropanol, flow rate = 0.5 mL/min. TR = 14.0 min (major) and 16.3 min (minor). The middle signal in the second trace was the olefin isomer.
¹H NMR (400 MHz, CDCl₃): 7.13-7.09 (m, 2H), 6.85-6.82 (m, 2H), 5.93-5.90 (m, 1H), 5.77-5.74 (m, 1H), 3.88-3.81 (m, 1H), 3.79 (s, 3H), 2.49-2.35 (m, 3H), 1.71-1.65 (m, 1H).

### (S)-3-(4-tert-Butylphenyl)cyclopentene.

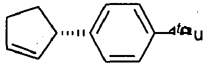

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p*-*tert*-butylphenyl triflate (141 mg, 0.50 mmol), *N*-ethyldiisopropylamine (170 *µ*L, 1.0 mmol) and cyclopentene (180 *µ*L, 2.0 mmol) were used. The reaction was complete in 39 h at 70°C. The product was isolated by flash chromatography with pentane as colorless oil. Yield: 76 mg, 76%. Olefinic selectivity in the crude product: 31:1. A small amount of diarylated by product was also isolated.
GC-MS (EI): Calcd for C15H20 M+: 200.2. Found: 200.1.
[α]²⁰_{D} = -20o (c = 1.2, CHCl3).
Ee: 96%. Daicel Chiralcel OJ-H, *n*-hexane, flow rate = 0.33 mL/min. TR = 18.6 min (major) and 23.0 min (minor). The middle signal in the second trace was the olefin isomer.
¹H NMR (400 MHz, CDCl₃): 7.33-7.30 (m, 2H), 7.14-7.11 (m, 2H), 5.94-5.91 (m, 1H), 5.79-5.76 (m, 1H), 3.90-3.84 (m, 1H), 2.53-2.34 (m, 3H), 1.77-1.69 (m, 1H), 1.31 (s, 9H).
¹³C NMR (CDCl₃, 100 MHz): 149.0, 143.7, 134.8, 131.9, 127.1, 125.5, 51.0, 34.6, 34.0, 32.8, 31.7.

### (S)-3-(o-Tolyl)cyclopentene [racemate 78135-01-0]¹³.

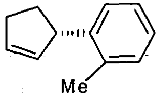

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *o*methylphenyl triflate (141 mg, 0.50 mmol), *N*-ethyldiisopropyl-amine (170 *µ*L, 1.0 mmol) and cyclopentene (180 *µ*L, 2.0 mmol) were used. The reaction was complete in 39 h at 50°C. The product was isolated by flash chromatography with pentane as colorless oil. Yield: 59 mg, 75%. Olefinic selectivity in the crude product: 20:1. A small amount of diarylated product (1,3-di(2-methylphenyl)-cyclopentene) was also isolated from the reaction.
Ee: 99%. Chiral GC with Chiraldex B-PH column (30 m x 0.25 mm): carrier gas: He; flow rate: 0.91 mL/min; column temperature: 90 °C. TR = 36.3 min (minor) and 38.5 min (major). (The second trace showed the results using (*R*)-BINAP(O) ligand.)
¹H NMR (400 MHz, CDCl₃): 7.17-7.07 (4H), 5.98-5.95 (m, 1H), 5.79-5.76 (m, 1H), 4.13-4.09 (m, 1H), 2.53-2.38 (m, 3H), 2.37 (s, 3H), 1.65-1.57 (m, 1H).

### (S)-3-(2-Methoxyphenyl)cyclopentene [racemate 31169-37-6].¹¹

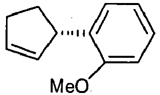

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *o*-anisyl triflate (128 mg, 0.50 mmol), *N*-ethyldiisopropylamine (170 *µ*L, 1.0 mmol) and 25 cyclopentene (180 *µ*L, 2.0 mmol) were used. The reaction completed in 21 h at 0 °C. The product was isolated by flash chromatography with pentane/Et₂O (10:1) as colorless oil.
Yield: 68 mg, 78%. Olefinic selectivity in the crude product: 19:1.
[α]²⁰_{D} = -79° (c = 1.2, CHCl₃).
Ee: 98%. Chiral GC with Chiraldex B-PH column (30 m x 0.25 mm): carrier gas: He; flow rate: 0.91 mL/min; column temperature: 90 °C for 100 min then raised to 150 °C at the rate of 15 °C/min. TR = 102.5 min (minor) and 103.0 min (major).
¹H NMR (400 MHz, CDCl₃): 7.17 (ψtd, *J* = 7.8, 1.8 Hz, 1H), 7.12 (dd, *J* = 7.5, 1.7 Hz, 1H), 6.90 (ψtd, *J* = 7.4, 1.0 Hz, 1H), 6.86 (d, *J* =8.2 Hz, 1H), 5.95-5.92 (m, 1H), 5.78-5.75 (m, 1H), 4.29-4.25 (m, 1H), 3.84 (s, 3H), 2.47-2.37 (m, 3H), 1.67-1.61 (m, 1H).

### (S)-3-(4-Chlorophenyl)cyclopentene [racemate: 2362-71-2]¹³.

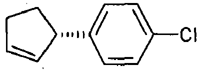

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p*-chlorophenyl triflate (130 mg, 0.50 mmol), *N*-ethyldiisopropylamine (170 *µ*L, 1.0 mmol) and cyclopentene (180 *µ*L, 2.0 mmol) were used. The reaction completed in 35 h at 50 °C. The product was isolated by flash chromatography with pentane as colorless oil. Yield: 62 mg, 70%. Olefinic selectivity in the crude product: 31:1.
Ee: >99%. Chiral GC with Chiraldex B-PH column (30 m x 0.25 mm): carrier gas: He; flow rate: 0.91 mL/min; column temperature: 80 oC for 200 min then raised at the rate of 15 °C/min to 150 °C.
¹H NMR (400 MHz, CDCl₃): 7.26-7.23 (m, 2H), 7.13-7.09 (m, 2H), 5.96-5.93 (m, 1H), 5.74-5.71 (m, 1H), 3.87-3.83 (m, 1H), 2.53-2.35 (m, 3H), 1.73-1.60 (m, 1H).
¹³C NMR (100 MHz, CDCl₃): 145.1, 134.0, 132.6, 131.7, 128.7, 128.6, 50.8, 33.9, 32.6.

### (S)-3-(4-Benzophenonyl)cyclopentene.

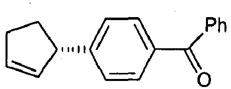

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p*-benzophenonyl triflate (164 mg, 0.50 mmol), *N*-ethyldiisopropylamine (170 *µ*L, 1.0 mmol) and cyclopentene (180 *µ*L, 2.0 mmol) were used. The reaction was complete in 5 h at 50 °C. The product was isolated by flash chromatography with pentane/Et₂O (10:1 to 5:1) as colorless oil.
Yield: 122 mg, 98%. Olefinic selectivity in the crude product: 70:1.
GC-MS (EI): Calcd for C₁₈H₁₆O M+: 248.1, Found: 248.1.
[α]²⁰_{D} = -170° (*c* = 1.3, CHCl₃).
Ee: 99%. Daicel Chiralcel OJ-H, 98:2 *n*-hexane/isopropanol, flow rate = 0.5 mL/min. TR = 25.1 min (major) and 27.4 min (minor).
¹H NMR (400 MHz, CDCl₃): 7.81-7.78 (m, 2H), 7.76-7.74 (m, 2H), 7.60-7.56 (m, 1H), 7.49-7.46 (m, 2H), 7.30 (d, *J* = 8.2 Hz, 2H), 6.01-5.98 (m, 1H), 5.81-5.78 (m, 1H), 4.01-3.95 (m, 1H), 2.59-2.40 (m, 3H), 1.80-1.70 (m, 1H).
¹³C NMR (100 MHz, CDCl₃): 196.6, 151.8, 138.1, 135.6, 133.6, 132.9, 132.3, 130.6, 130.1, 128.4, 127.3, 51.5, 33.8, 32.7.

### (S)-3-(4-Formylphenyl)cyclopentene.

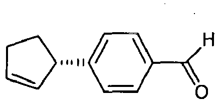

Pd(dba)₂ (14.4 mg, 0.025 mmol), (*R*)-Xyl-SDP(O) (21.6 mg, 0.030 mmol), 1,4-dioxane (0.50 mL), *n*-C₁₄H₃₀ (25 *µ*L), *p*-formylphenyl triflate (125 mg, 0.49 mmol), *N*-ethyldiisopropylamine (170 *µ*L, 1.0 mmol) and cyclopentene (180 *µ*L, 2.0 mmol) were used. The reaction completed in 24 h at 50°C. The product was isolated by flash chromatography with pentane/Et₂O (10:1 to 5:1) as colorless oil.
Yield: 80 mg, 94%. Olefinic selectivity in the crude product: 20:1.
GC-MS (EI): Calcd for C₁₂H₁₂O M+: 172.1, Found: 172.1.
Ee: 99%. Daicel Chiralcel OJ-H, *n*-hexane, flow rate = 0.3 mL/min. TR = 48.1 min (major) and 51.4 min (minor).
¹H NMR (400 MHz, CDCl₃): 9.98 (s, 1H), 7.1 (d, *J* = 8.2 Hz, 2H), 7.35 (d, *J* = 8.1 Hz, 2H), 6.02-5.99 (m, 1H), 5.78-5.75 (m, 1H), 4.00-3.95 (m, 1H), 2.57-2.40 (m, 3H), 1.77-1.69 (m, 1H).
¹³C NMR (100 MHz, CDCl₃): 192.2, 154.1, 134.9, 133.3, 133.2, 130.2, 128.0, 51.7, 33.8, 32.7.

### (S)-3-(2-Methyl-1-cyclohexenyl)cyclopentene.

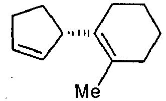

Pd(dba)₂ (7.2mg, 0.025 mmol), (*R*)-Xyl- SDP(O) (10.8 mg, 0.030 mmol), 1,4-dioxane (0.75 mL), 2-methylcyclohexenyl triflate (122 mg, 0.50 mmol), *N-*ethyldiisopropylamine (170 *µ*L, 1.0 mmol) and cyclopentene (180 *µ*L, 2.0 mmol) were used. The reaction completed in 4 h at 50°C. The product was isolated by flash chromatography (silica gel, pentane) as colorless oil. Yield: 67 mg, 83%. Olefinic selectivity in the crude product: 92:1.
GC-MS (EI): Calcd for C₁₂H₁₈ M+: 162.1, Found: 162.1.
Ee: 97%. Chiral GC with Chiraldex B-PH column (30 m x 0.25 mm): carrier gas: He with flow rate of 0.91 mL/min; isothermal at 90 °C. TR = 25.4 min (major) and 26.6 min (minor).
¹H NMR (400 MHz, CDCl₃): 5.79-5.75 (m, 1H), 5.55-5.52 (m, 1H), 3.87-3.81 (m, 1H), 2.40-2.28 (m, 2H), 2.07-1.98 (m, 1H), 1.96-1.93 (m, 2H), 1.86-1.79 (m, 2H), 1.66 (s, 3H), 1.59-1.47 (m, 5H).
¹³C NMR (100 MHz, CDCl₃): 134.3, 132.6, 131.1, 125.9, 47.6, 32.8, 32.5, 28.5, 25.0, 23.7, 23.5, 19.2.

### (S)-5-(Cyclopent-2-en-1-yl)-2-methylbenzothiazole.

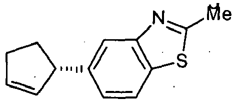

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (15 *µ*L), 2-methylbenzo[*d*]thiazol-5-yl triflate (149 mg, 0.50 mmol), *N-*ethyldiisopropylamine (170 *µ*L, 1.0 mmol) and cyclopentene (180 *µ*L, 2.0 mmol) were used. The reaction was complete in 24 h at 70°C. The product was isolated by flash chromatography (silica gel, 10:1 hexane/EtOAc) as colorless oil. Yield: 106 mg, 98%. Olefinic selectivity in the crude product: 48:1.
GC-MS (EI): Calcd for C₁₃H₁₃NS M+: 215.1, Found: 215.1.
Ee: 96%. Daicel Chiralcel IC, 95:5 hexanes/isopropanol, flow rate = 0.5 mL/min. TR = 13.0 min (minor) and 15.9 min (major).
¹H NMR (400 MHz, CDCl₃): 7.77 (d, *J* = 1.5 Hz, 1H), 7.72 (d, *J* = 8.2 Hz, 1H), 7.20 (dd, *J* = 8.2, 1.5 Hz, 1H), 5.99-5.96 (m, 1H), 5.83-5.80 (m, 1H), 4.05-4.00 (m, 1H), 2.82 (s, 3H), 28 2.57-2.40 (m, 3H), 1.81-1.72 (m, 1H).
¹³C NMR (100 MHz, CDCl₃): 167.2, 154.0, 145.1, 134.2, 133.3, 132.4, 124.6, 121.2, 120.8, 51.3, 34.1, 32.6, 20.3.

### (S)-N-Tosyl-5-(Cyclopent-2-en-1-yl)indole.

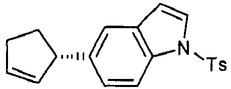

Pd(dba)₂ (5.4 mg, 0.009 mmol), (*R*)-Xyl- SDP(O) (10.8 mg, 0.011 mmol), 1,4-dioxane (0.50 mL), *n*-C₁₄H₃₀ (15 *µ*L), 1-tosyl-1*H*-indol-5-yl triflate (157 mg, 0.37 mmol), *N*-ethyldiisopropylamine (128 *µ*L, 0.75 mmol) and cyclopentene (130 *µ*L, 1.5 mmol) were used. The reaction was complete in 15 h at 70°C. The product was purified by flash chromatography (silical gel, 10:1 hexane/EtOAc) as colorless oil in 91% yield (112 mg).
Olefinic selectivity in the crude product: 18:1. GC-MS (EI): Calcd for C₂₀H₁₉NO₂S M+: 337.1, Found: 337.1.
Ee: 97%. Daicel Chiralcel AD-H, 95:5 hexanes/isopropanol, flow rate = 0.5 mL/min. TR = 23.5 min (minor) and 25.2 min (major).
¹H NMR (400 MHz, CDCl₃): 7.88 (d, *J* = 8.5 Hz, 1H), 7.76 (d, *J* = 8.4 Hz, 2H), 7.52 (d, *J* = 3.6 Hz, 1H), 7.32 (d, *J* = 1.2 Hz, 1H), 7.21 (d, *J* = 8.2 Hz, 2H), 7.14 (dd, *J* = 8.5, 1.6 Hz, 1H), 6.58 (d, *J* = 3.6 Hz, 1H), 5.95-5.92 (m, 1H), 5.77-5.75 (m, 1H), 3.97-3.92 (m, 1H), 2.53- 2.35 (m, 3H), 2.33 (s, 3H), 1.74-1.66 (m, 1H).
¹³C NMR (100 MHz, CDCl₃): 144.9, 141.9, 135.6, 134.6, 133.6, 132.1, 131.1, 130.0, 127.0, 126.6, 124.5, 119.5, 113.5, 109.1, 51.3, 34.2, 32.6, 21.7.

### (S)-3-(m-Anisyl)cyclopentene [racemate: 369650-02-2].

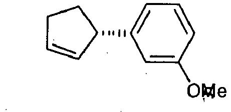

Pd(dba)₂ (1.5 mg, 0.0026 mmol), (*R*)-Xyl-SDP(O) (2.2 mg, 0.0031 mmol), 1,4-dioxane (0.50 mL), *n*-C₁₄H₃₀ (15 *µ*L), *m*-anisyl triflate (128 mg, 0.50 mmol), *N*-ethyldiisopropylamine (170 *µ*L, 1.0 mmol) and cyclopentene (180 *µ*L, 2.0 mmol) were used. The reaction was complete in 13 h at 50°C. The product was purified by flash chromatography (silica gel, 20:1 pentane/Et₂O) as pale yellow oil in 98% yield (85 mg). Olefinic selectivity in the crude product: 128:1.

Procedure for 2 mmol scale: Pd(dba)₂ (1.5 mg, 0.0026 mmol, 0.13 mol%)), (*R*)-Xyl-SDP(O) (2.3 mg, 0.0031 mol, 0.16 mol%), 1,4-dioxane (2 mL), *n*-C₁₄H₃₀ (60 *µ*L), *m*-anisyl triflate (2.0 mmol, 512 mg), *N*-ethyldiisopropylamine (680 *µ*L, 4 mmol) and cyclopentene (720 *µ*L, 8 mmol) were used. The reaction completed in 6 days at 50°C and afforded the product in 84% yield (292 mg) in 96% ee.
Ee: 96%. Daicel Chiralcel OJ-H, 99.8:0.2 hexanes/isopropanol, flow rate = 0.5 mL/min. TR = 15.7 min (major) and 17.3 min (minor).
¹H NMR (400 MHz, CDCl₃): 7.23-7.19 (m, 1H), 6.80-6.78 (m, 1H), 6.75-6.72 (m, 2H), 5.95-5.92 (m, 1H), 5.78-5.75 (m, 1H), 3.90-3.82 (m, 1H), 3.80 (s, 3H), 2.53-2.35 (m, 3H), 1.77-1.69 (m, 1H).
¹³C NMR (100 MHz, CDCl₃): 159.9, 148.4, 134.3, 132.2, 129.5, 119.8, 113.1, 111.4, 55.3, 51.5, 33.8, 32.6.

### (S)-2-(1-Naphthyl)-2,5-dihydrofuran [1072136-90-3].¹⁵

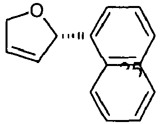

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 1-naphthyl triflate (140 mg, 0.51 mmol), *N*-ethyldiisopropylamine (170 *µ*L, 1.0 mmol) and 2,3- dihydrofuran (150*µ*L, 2.0 mmol) were used. The reaction was complete in 2.5 h at 50°C. The product was isolated by flash chromatography (10:1 to 3:1 pentane/Et₂O) as colorless oil.
Yield: 92 mg, 92%. Olefinic selectivity in the crude product: 262:1.
Ee: 99.5%. Daicel Chiralcel OD-H, 98:2 *n*-hexane/isopropanol, flow rate = 0.5 mL/min. TR = 29.4 min (minor) and 34.1 min (major).
¹H NMR (400 MHz, CDCl₃): 8.13 (d, *J* = 9.3 Hz, 1H), 7.87 (d, *J* = 7.9 Hz, 1H), 7.79 (d, *J* = 8.2 Hz, 1H), 7.56-7.44 (m, 4H), 6.58-6.55 (m, 1H), 6.16-6.13 (m, 1H), 6.10-6.07 (m, 1H), 4.97-4.85 (m, 2H).

### (S)-2-(2-Naphthyl)-2,5-dihydrofuran[131516-16-0].¹⁶

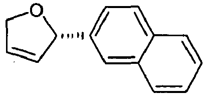

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 2-naphthyl triflate (140 mg, 0.51 mmol), *N*-ethyldiisopropylamine (170 *µ*L, 1.0 mol) and 2,3-dihydrofuran (150 *µ*L, 2.0 mmol) were used. The reaction was complete in 1 h at 50°C. The product was isolated by flash chromatography (10:1 to 3:1 pentane/Et₂O) as colorless oil.
Yield: 93 mg, 93%. Olefinic selectivity in the crude product: 81:1.
Ee: >99.5%. Daicel Chiralcel OD-H, n-hexane/isopropanol 98/2, flow rate = 0.5 mL/min. TR = 22.0 min (minor) and 24.9 min (major).
¹H NMR (400 MHz, CDCl₃): 7.84-7.80 (m, 3H), 7.76 (s, 1H), 7.49-7.43 (m, 2H), 7.41 (dd, J = 8.5, 1.6 Hz, 1H), 6.09-6.06 (m, 1H), 5.97-5.93 (m, 1H), 4.98-4.93 (m, 1H), 4.85-4.80 (m, 1H).

### (S)-2-(4-tert-Butylphenyl)-2,5-dihydrofuran.

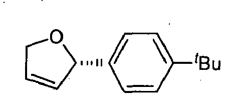

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p*-*tert*-butylphenyl triflate (142 mg, 0.50 mmol), *N*-ethyldiisopropylamine (170 *µ*L, 1.0 mmol) and 2,3-dihydrofuran (180 *µ*L, 2.0 mmol) were used. The reaction completed in 13 h at 50°C. The product was isolated by flash chromatography with pentane/Et2O (10:1 to 5:1) as colorless oil.
Yield: 90 mg, 88%. Olefinic selectivity in the crude product: 141:1.
GC-MS (EI): Calcd for C₁₄H₁₈O M+: 202.1, Found: 202.2.
Ee: 98%. Daicel Chiralcel OD-H, 98:2 *n*-hexane/isopropanol, flow rate = 0.5 mL/min. TR = 9.6 min (major) and 10.0 min (minor).
¹H NMR (400 MHz, CDCl₃): 7.39-7.36 (m, 2H), 7.25-7.23 (m, 2H), 6.05-6.03 (m, 1H), 5.91-5.88 (m, 1H), 5.79-5.76 (m, 1H), 4.89-4.83 (m, 1H), 4.78-4.73 (m, 1H), 1.31 (s, 9H).
¹³C NMR (100 MHz, CDCl₃): 151.0, 139.1, 130.1, 126.8, 126.4, 125.6, 87.8, 75.8, 34.7, 31.5.

### (S)-2-(p-Anisyl)-2,5-dihydrofuran [131516-15-9].¹⁶

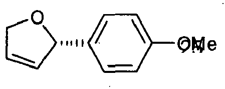

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p*-anisyl triflate(128 mg, 0.50 mmol), *N*-ethyldiisopropylamine (170 *µ*L, 1.0 mmol) and 2,3-dihydrofuran (180 *µ*L, 2.0 mmol) were used. The reaction was complete in 13 h at 50°C. The product was isolated by flash chromatography (10:1 to 5:1 pentane/Et₂O) as colorless liquid.
Yield: 80 mg, 91%. Olefinic selectivity in the crude product: 27:1.
[α]²⁰_{D} = -98° (*c* = 1.0, CHCl₃).
Ee: 99%. Daicel Chiralcel OD-H, 98:2 *n*-hexane/isopropanol, flow rate = 0.5 mL/min. TR = 18.5 min (major) and 20.8 min (minor).
¹H NMR (400 MHz, CDCl₃): 7.25-7.21 (m, 2H), 6.90-6.86 (m, 2H), 6.05-6.03 (m, 1H), 5.88-5.85 (m, 1H), 5.77-5.73 (m, 1H), 4.87-4.82 (m, 1H), 4.76-4.71 (m, 1H), 3.80 (s, 3H).

### (S)-2-[p-(Trifluoromethyl)phenyl]-2,5-dihydrofuran [331754-75-7].¹⁷

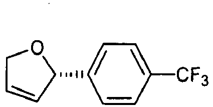

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p-*(trifluoromethyl)phenyl triflate (148 mg, 0.50 mmol), *N-*ethyldiisopropylamine (170 *µ*L, 1.0 mmol) and 2,3-dihydrofuran (150 *µ*L, 2.0 mmol) were used. The reaction was complete in 14 h at 50°C. The product was isolated by flash chromatography (10:1 to 5:1 pentane/Et₂O) as a colorless liquid.
Yield: 71 mg, 66% (77% GC yield). Olefinic selectivity in the crude product: 7:1.
Ee: 99%. Daicel Chiralcel OJ-H, 98:2 *n*-hexane/isopropanol, flow rate = 0.5 mL/min. TR = 14.0 min (major) and 15.6 min (minor).
¹H NMR (400 MHz, CDCl₃): 7.60 (d, *J* = 8.1 Hz, 2H), 7.42 (d, *J* = 8.1 Hz, 2H), 6.08-6.05 (m, 1H), 5.90-5.83 (m, 2H), 4.93-4.87 (m, 1H), 4.83-4.78 (m, 1H).

### (S)-2-(4-Chlorophenyl)-2,5-dihydrofuran [131516-11-5].¹⁶

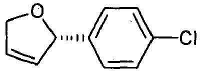

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), pchlorophenyl triflate (130 mg, 0.50 mmol), *N*-ethyldiisopropylamine (170 *µ*L, 1.0 mmol) and 2,3-dihydrofuran (180 *µ*L, 2.0 mmol) were used. The reaction was complete in 6 h at 50 °C. The product was isolated by flash chromatography (10:1 to 5:1 pentane/Et2O) as colorless oil.
Yield: 70 mg, 78%.
Olefinic selectivity in the crude product: 135:1.
Ee: 99%. Daicel Chiralcel OD-H, 98:2 *n*-hexane/isopropanol, flow rate = 0.5 mL/min. TR = 12.1 min (major) and 13.4 min (minor).
¹H NMR (400 MHz, CDCl₃): 7.33-7.30 (m, 2H), 7.25-7.22 (m, 2H), 6.06-6.03 (m, 1H), 5.87-5.84 (m, 1H), 5.78-5.74 (m, 1H), 4.89-4.83 (m, 1H), 4.79-4.74 (m, 1H).

### (S)-2-[p-(Ethoxycarbonyl)phenyl]-2,5-dihydrofuran.

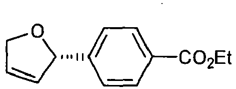

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p-*(ethoxycarbonyl)phenyl triflate (149 mg, 0.50 mmol), *N-*ethyldiisopropylamine (170 *µ*L, 1.0 mmol) and 2,3-dihydrofuran (150 *µ*L, 2.0 mmol) were used. The reaction completed in 5 h at 50°C. The product was isolated by flash chromatography (10:1 to 5:1 pentane/Et₂O) as colorless oil. Yield: 102 mg, 93%. Olefinic selectivity in the crude product: 64:1.
GC-MS (EI): Calcd for C₁₃H₁₄O₃ M+: 218.1, Found: 218.1.
Ee: 99%. Daicel Chiralcel OD-H, 98:2 *n*-hexane/isopropanol, flow rate = 0.5 mL/min. TR = 16.7 min (major) and 20.3 min (minor).
¹H NMR (400 MHz, CDCl₃): 8.04-8.01 (m, 2H), 7.39-7.36 (m, 2H), 6.06-6.03 (m, 1H), 5.90-5.87 (m, 1H), 5.86-5.82 (m, 1H), 4.93-4.87 (m, 1H), 4.83-4.80 (m, 1H), 4.37 (q, *J* = 7.1 Hz, 2H), 1.39 (t, *J* = 7.1 Hz, 3H).
¹³C NMR (100 MHz, CDCl₃): 166.6, 147.3, 130.0 (2 peaks), 129.7, 127.1, 126.2, 87.6, 76.2, 61.1, 14.5.

### (S)-2-(o-Tolyl)-2,5-dihydrofuran [1361252-01-8].¹⁸

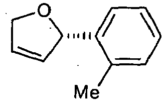

Pd(dba)₂ (7.2 mg, 0.013 mmol), (*R*)-Xyl- SDP(O) (10.8 mg, 0.015 mmol), 1,4-dioxane (0.50 mL), *n*-C₁₄H₃₀ (25 *µ*L), *o*-methylphenyl triflate (120 mg, 0.50 mmol), *N*-ethyldiisopropylamine (170 *µ*L, 1.0 mmol) and 2,3-dihydrofuran (150 *µ*L, 2.0 mmol) were used. The reaction was complete in 14 h at 50 °C. The product was isolated by flash chromatography (10:1 to 5:1 pentane/Et₂O) as colorless oil.
Yield: 67 mg, 81%. Olefinic selectivity in the crude product: 136:1
GC-MS (EI): Calcd for C₁₁H₁₂O M+: 160.1, Found: 160.1.
Ee: 99%. Daicel Chiralcel OD-H, 98:2 *n*-hexane/isopropanol, flow rate = 0.5 mL/min. TR = 11.9 min (minor) and 13.4 min (major).
¹H NMR (400 MHz, CDCl₃): 7.33-7.29 (m, 1H), 7.22-7.13 (m, 3H), 6.05-6.02 (m, 2H), 5.94-5.90 (m, 1H), 4.91-4.85 (m, 1H), 4.81-4.76 (m, 1H), 2.40 (s, 3H).
¹³C NMR (100 MHz, CDCl₃): 140.1, 135.2, 130.6, 129.2, 127.7, 126.9, 126.4, 126.3, 85.2, 75.8, 19.1.

### (S)-2-(o-Anisyl)-2,5-dihydrofuran [racemate: 479682-88-7].¹⁹

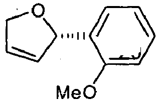

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *o*-anisyl triflate (128 mg, 0.50 mmol), *N-*ethyldiisopropylamine (170 *µ*L, 1.0 mmol) and 2,3- dihydrofuran (180 *µ*L, 2.0 mmol) were used. The reaction completed in 24 h at 50 °C.

The product was isolated by flash chromatography (10:1 to 5:1 pentane/Et₂O) as colorless oil. Yield: 78 mg, 89%.
Olefinic selectivity in the crude product: >100:1
Ee: 99%. Daicel Chiralcel OJ-H, 98:2 *n*-hexane/isopropanol, flow rate = 0.5 mL/min. TR = 16.9 min (major) and 18.2 min (minor).
¹H NMR (400 MHz, CDCl₃): 7.34 (dd, *J* = 7.5, 1.5 Hz, 1H), 7.26-7.22 (m, 1H), 6.95 (ψt, *J* = 7.4 Hz, 1H), 6.87 (d, *J* = 8.2 Hz, 1H), 6.15-6.12 (m, 1H), 6.00-5.93 (m, 2H), 4.89-4.83 (m, 1H), 4.81-4.76 (m, 1H), 3.85 (s, 3H).
¹³C NMR (100 MHz, CDCl₃): 156.3, 130.7, 129.6, 128.6, 126.6, 126.0, 120.8, 110.4, 82.6, 75.7, 55.5.

### (S)-2-(p-Benzophenonyl)-2,5-dihydrofuran [720688-72-2].²⁰

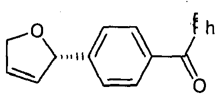

Pd(dba)₂ (7.2 mg, 0.013 mol), (*R*)-Xyl-SDP(O) (10.8 mg, 0.015 mmol), 1,4-dioxane (0.50 mL), *n*-C₁₄H₃₀ (25 *µ*L), *p*-benzophenonyl triflate (167 mg, 0.51 mmol), *N*-ethyldiisopropylamine (170 *µ*L, 1.0 mmol) and 2,3-dihydrofuran (180 *µ*L, 2.0 mmol) were used. The reaction completed in 5 h at 50 °C.

The product was isolated by flash chromatography with 10:1 to 5:1 pentane/Et2O as pale yellow liquid. Yield: 98 mg, 78%. Olefinic selectivity in the crude product: 18:1.
[α]²⁰_{D} = -91° (*c* = 1.5, CHCl₃).
Ee: 99%. Daicel Chiralcel OD-H, 99:1 *n*-hexane/isopropanol, flow rate = 0.5 mL/min. TR = 42.6 min (minor) and 46.0 min (major). (It was reported that the (-)-isomer had longer retention time than the (+)-isomer when using Chiralcel OD connected with another OD-H).
¹H NMR (400 MHz, CDCl₃): 7.81-7.78 (m, 4H), 7.61-7.56 (m, 1H), 7.50-7.41 (m, 4H), 6.09-6.06 (m, 1H), 5.93-5.86 (m, 2H), 4.95-4.89 (m, 1H), 4.85-4.79 (m, 1H).
¹³C NMR (100 MHz, CDCl₃): 196.6, 146.9, 137.8, 137.1, 132.5, 130.6, 130.2, 129.6, 128.4, 127.2, 126.2, 87.6, 76.3.

### (S)-2-(p-Formylphenyl)-2,5-dihydrofuran.

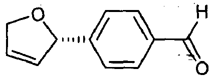

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), *p*-formylphenyl triflate (128 mg, 0.50 mmol), *N*-ethyldiisopropylamine (170 *µ*L, 1.0 mmol) and 2,3-dihydrofuran (180 *µ*L, 2.0 mmol) were used. The reaction was complete in 32 h at 50°C. The product was isolated by flash chromatography (silica gel, 10:1 to 5:1 pentane/Et₂O) as pale yellow oil.
Yield: 72 mg, 82%. Olefinic selectivity in the crude product: 73:1.
GC-MS (EI): Calcd for C₁₁H₁₀O₂ M+: 174.1, Found: 174.1.
Ee: 99%. Daicel Chiralcel OD-H, 99:1 *n*-hexane/isopropanol, flow rate = 0.5 mL/min. TR = 31.9 min (major) and 35.8 min (minor).
¹H NMR (400 MHz, CDCl₃): 10.01 (s, 1H), 7.88-7.86 (m, 2H), 7.49-7.47 (m, 2H), 6.09- 6.06 (m, 1H), 5.91-5.85 (m, 2H), 4.95-4.89 (m, 1H), 4.85-4.79 (m, 1H).
¹³C NMR (100 MHz, CDCl₃): 192.1, 149.2, 136.0, 130.2, 129.4, 127.4, 126.8, 87.5, 76.3.

### (S)-3-(1-Naphthyl)cyclohex-1-ene [303030-27-5].^{10b}

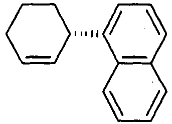

*Heck reaction using (R)-Xyl-SDP(O) as the ligand.*
Pd(dba)₂ (14.4 mg, 0.025 mmol), (*R*)-Xyl-SDP(O) (21.6 mg, 0.015 mmol), 1,4-dioxane (0.50 mL), *n*-C₁₄H₃₀ (25 *µ*L), 1-naphthyl triflate (138 mg, 0.50 mmol), and cyclohexene (203 *µ*L, 2.0 mmol) were used. Li₂CO₃ (74 mg, 1.0 mmol) was used instead of *N*-ethyldiisopropylamine. The reaction was complete in 24 h at 70 °C. The product was purified by flash chromatography with pentane as colorless oil.
Yield: 95 mg, 91%. Ee: 83%. Olefinic selectivity in the crude product: 26:1.
[α]²⁰_{D} = + 34° (*c* = 1.4, CHCl₃).

### Heck reaction using (R)-pFPh-Cy-SDP(O) as the ligand.

Pd(dba)₂ (14.4 mg, 0.025 mmol), (*R*)-*p*F-Cy-SDP(O) (19.6 mg, 0.030 mmol), *n*-C₁₄H₃₀ (25 *µ*L), 1-naphthyl triflate (138 mg, 0.50 mmol), and cyclohexene (203 *µ*L, 2.0 mmol) were used. Li2CO3 (113 mg, 1.5 mmol) was used instead of *N*-ethyldiisopropylamine. 1,2-Dimethoxybenzene (0.50 mL) was used as solvent and Li₂CO₃ (113 mg, 1.5 mmol) was used instead of *N*-ethyldiisopropylamine. The reaction was complete in 24 h at 80°C. The product was purified by flash chromatography with pentane as colorless oil.
Yield: 91 mg, 86%. Olefinic selectivity in the crude product: 9:1. Ee: 93%. Daicel Chiralcel OJ-H, 98:2 *n*-hexane/isopropanol, flow rate = 0.5 mL/min. TR = 11.5 min (major) and 13.6 min (minor).
¹H NMR (400 MHz, CDCl₃): 8.13 (d, *J* = 8.4 Hz, 1H), 7.87-7.85 (m, 1H), 7.71 (d, *J* = 7.6 Hz, 1H), 7.53-7.34 (m, 4H), 6.03-6.00 (m, 1H), 5.85-5.82 (m, 1H), 4.24-4.22 (m, 1H), 2.18- 2.14 (m, 3H), 1.76-1.68 (m, 3H).
¹³C NMR (100 MHz, CDCl₃): 142.1, 134.3, 131.6, 130.4, 129.2, 129.0, 126.8, 125.9, 125.7, 125.5, 125.3, 123.6, 37.2, 31.1, 25.5, 21.1.

### (S)-3-(1-Naphthyl)cyclohept-1-ene.

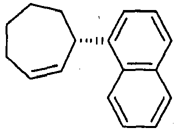

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 1-naphthyl triflate (141 mg, 0.51 mmol), *N*-ethyldiisopropylamine (170 *µ*L, 1.0 mmol) and cycloheptene (192 mg, 2.0 mmol) were used. The reaction completed in 14 h at 50 °C. The product was isolated by flash chromatography with pentane as a colorless liquid.
Yield: 112 mg, 98%. GC-MS (EI): Calcd for C₁₇H₁₈ M+: 222.1, Found: 222.1. Ee: 88%. [α]²⁰_{D} = +37° (*c* = 1.9, CHCl₃).
When the reaction was carried out at 27°C, it completed in 5 days with 91% ee.
Yield: 104 mg, 91%. Olefinic selectivity in the crude product: 14:1.
HPLC condition: Daicel Chiralcel OD-H, n-hexane/isopropanol 99.9/0.1, flow rate = 0.25 mL/min. TR = 45.1 min (minor) and 53.1 min (major).
¹H NMR (400 MHz, CDCl₃): 8.10 (d, *J* = 8.2 Hz, 1H), 7.87-7.85 (m, 1H), 7.31-7.69 (m, 1H), 7.52-7.42 (m, 4H), 5.95-5.89 (m, 1H), 5.86-5.82 (1H), 4.29 (d, *J* = 9.5 Hz, 1H), 2.40- 2.32 (m, 2H), 2.08-1.93 (m, 3H), 1.91-1.71 (m, 2H), 1.58-1.48 (m, 1H).
¹³C NMR (100 MHz, CDCl₃): 143.7, 137.6, 134.2, 131.6, 131.3, 129.0, 126.7, 125.8 (2C), 125.5, 124.2, 124.0, 42.9, 35.1, 31.0, 29.0, 27.3.

### (S)-3-(1-Naphthyl)cyclooctene.

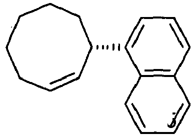

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 1-naphthyl triflate (140 mg, 0.51 mmol), *N*-ethyldiisopropylamine (170 *µ*L, 1.0 mmol) and cyclooctene (220 mg, 2.0 mmol) were used. The reaction completed in 16 h at 50°C. The product was isolated by flash chromatography with pentane as colorless oil.
Yield: 116 mg, 97%. Olefinic selectivity in the crude product: 10:1. GC-MS (EI): Calcd for C₁₈H₂₀O M+: 236.2, Found: 236.1. Ee: 88%.

The same reaction was carried out at 27°C, and it completed in 60 h with slightly higher ee (91%). Yield: 118 mg, 97%. Olefinic selectivity in the crude product: 12:1.
[α]²⁰_{D} = +197° (*c* = 1.5, CHCl₃).
HPLC condition: Daicel Chiralcel OJ-H, 99:1 *n*-hexane/isopropanol, flow rate = 0.5 mL/min.
TR = 10.2 min (minor) and 14.2 min (major).
¹H NMR (400 MHz, CDCl₃): 8.07 (d, *J* = 8.3 Hz, 1H), 7.84 (d, *J* = 8.2 Hz, 1H), 7.71 (d, *J* = 7.7 Hz, 1H), 7.51-7.42 (m, 4H), 5.76-5.70 (m, 1H), 5.60-5.55 (m, 1H), 4.53-4.46 (m, 1H), 2.66-2.57 (m, 1H), 2.32-2.24 (m, 1H), 2.01-1.91 (m, 2H), 1.88-1.73 (m, 4H), 1.68-1.59 (m, 1H), 1.54-1.43 (m, 1H).
¹³C NMR (100 MHz, CDCl₃): 142.4, 134.9, 134.1, 132.1, 128.9 (2 peaks), 126.7, 125.8 (2 peaks), 125.5, 123.8, 123.0, 37.7, 36.0, 29.9, 26.8, 26.7, 26.4.

### (S)-6-(1-Naphthyl)-3,6-dihydro-2H-pyran.

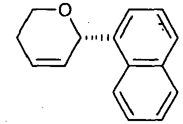

Pd(dba)₂ (14.4 mg, 0.025 mmol) and (*R*)-*p*FPh-Cy-SDP(O) (19.6 mg, 0.030 mmol), *n*-C₁₄H₃₀ (25 *µ*L), 1-naphthyl triflate (138 mg, 0.50 mmol) and 3,4-dihydro-2*H*-pyran (168 mg, 2.0 mmol) were used. 1,2-Dimethoxybenzene (0.75 mL) was used as solvent and Li₂CO₃ (74 mg, 1.0 mmol) was used instead of *N*ethyldiisopropylamine. The reaction was stirred at 70 °C for 2.5 d with 90% conversion of the triflate. The product was purified by flash chromatography (silica gel, 50:1 hexane/EtOAc) as a colorless liquid.
Yield: 82 mg, 78%. Olefinic selectivity in the crude product: 5:1.
GC-MS (EI): Calcd for C₁₅H₁₄O M+: 210.1, Found: 210.1.
Ee: 92%. Daicel Chiralcel IC, hexane/isopropanol 98/2, flow rate = 0.5 mL/min. TR = 15.6 min (major) and 17.5 min (minor).
¹H NMR (400 MHz, CDCl₃): 8.26 (d, *J* = 8.4 Hz, 1H), 7.86 (d, *J* = 7.8 Hz, 1H), 7.80 (d, *J* = 8.2 z, 1H), 7.57-7.42 (m, 4H), 6.13-6.08 (m, 1H), 6.00-5.96 (m, 1H), 5.88-5.87 (m, 1H), 4.04-3.98 (m, 1H), 3.91-3.85 (m, 1H), 2.44-2.35 (m, 1H), 2.25-2.16 (m, 1H).
¹³C NMR (100 MHz, CDCl₃): 136.5, 134.2, 131.6, 129.4, 128.8 (2 peaks), 126.3, 125.9 (2 peaks), 125.7, 125.2, 124.2, 73.3, 63.0, 25.5.

### (S)-N-Ethoxycarbonyl-2-(1-naphthyl)-2,5-dihydro-1H-pyrrole.

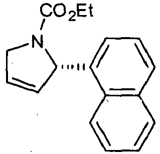

Pd(dba)₂ (7.2 mg, 0.013 mmol), (*R*)-Xyl-SDP(O) (10.8 mg, 0.015 mmol), 1,4-dioxane (0.50 mL), *n*-C₁₄H₃₀ (25 *µ*L), 1-naphthyl triflate (140 mg, 0.51 mmol), *N*-ethyldiisopropylamine (170 *µ*L, 1.0 mmol) and 2,3-dihydrofuran (180 *µ*L, 2.0 mmol) were used. The reaction was complete in 44 h at 50 °C. The product was isolated by flash chromatography (10:1 to 1:1 pentane/Et₂O) as colorless oil.
Yield: 131 mg, 96%. Olefinic selectivity in the crude product: 54:1. GC-MS (EI): Calcd for C17H17NO2 M+: 267.1, Found: 267.0.
[α]²⁰_{D} = -263° (*c* = 1.2, CHCl₃).
Ee: 99%. Daicel Chiralcel AS-H, 98:2 hexane/isopropanol, flow rate = 0.5 mL/min. TR = 24.5 min (minor) and 28.5 min (major).
¹H NMR of 2 rotamers in 4:6 ratio (400 MHz, CDCl₃): 8.13 (dd, J = 8.4, 8.0 Hz, 1H), 7.89-7.84 (m, 1H), 7.76-7.74 (m, 1H), 7.56-7.41 (m, 3H), 7.37-7.33 (m, 1H), 6.39 (br s, 0.4H), 6.33 (br s, 0.6H), 5.96-5.84 (m, 2H), 4.51-4.43 (m, 2H), 4.22-4.08 (m, 1H), 3.96-3.91 (m, 1H), 1.28 (t, *J* = 7.2 Hz, 1.2H), 0.81 (t, *J* = 7.0 Hz, 1.8H).
¹³C NMR of 2 rotamers (100 MHz, CDCl₃): 155.3, 155.0, 137.9, 137.2, 134.1, 133.9, 131.1, 130.9, 130.6, 130.5, 129.0, 127.9, 127.8, 126.1, 125.9, 125.6, 124.7, 124.6, 123.2, 123.0, 22.9, 122.8, 65.4, 64.6, 61.3, 61.2, 60.9, 54.4, 54.0, 15.0, 14.5.

### (S)-5-(1-Naphthyl)-4,5-dihydro-1,3-dioxepine.

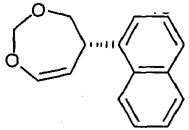

Pd(dba)₂ (7.2 mg, 0.013 mmol), (*R*)-Xyl- SDP(O) (10.8 mg, 0.015 mmol), 1,4-dioxane (0.50 mL), *n*-C₁₄H₃₀ (25 *µ*L), 1-naphthyl triflate (140 mg, 0.51 mmol), and *cis*-4,7-dihydro-1,3-dioxepine (191 *µ*L, 2.0 mmol) were used. Li₂CO₃ (74 mg, 1.0 mmol) was used instead of *N*-ethyldiisopropylamine. The reaction completed in 44 h at 50°C. The product was isolated by flash chromatography (4:1 pentane/Et₂O) as colorless oil. Yield: 112 mg, 98%. Olefinic selectivity in the crude product: 146:1.
GC-MS (EI): Calcd for C15H14O2 M+: 226.1, Found: 226.1.
[α]²⁰_{D} = +96° (*c* = 1.2, CHCl₃).
Ee: 92%. Daicel Chiralcel OJ-H, 98:2 hexane/isopropanol, flow rate = 0.5 mL/min. TR = 28.0 min (major) and 35.3 min (minor).
¹H NMR (400 MHz, CDCl₃): 8.13 (d, *J* = 8.4 Hz, 1H), 7.89-7.87 (m, 1H), 7.77 (d, *J* = 7.9 Hz, 1H), 7.55-7.42 (m, 4H), 6.56 (dd, *J* = 7.3, 2.5 Hz, 1H), 5.32 (d, *J* = 7.0 Hz, 1H), 5.07 (ddd, *J* = 7.3, 3.2, 0.6 Hz, 1H), 4.86 (d, *J* = 7.0 Hz, 1H), 4.70-4.65 (m, 1H), 4.18 (dd, *J* = 11.7, 4.6 Hz, 1H), 3.56 (dd, *J* = 11.7, 9.2 Hz, 1H).
¹³C NMR (100 MHz, CDCl₃): 146.4, 136.6, 134.2, 131.3, 129.3, 127.8, 126.4, 125.8 (2 peaks), 125.7, 123.1, 113.0, 98.4, 75.9, 44.1.

### D. Application of asymmetric Heck reaction in synthesis of drug candidates.

### (+)-(R)-Preclamol [85976-54-1].14

It was prepared from (*S*)-3-(*m*-anisyl)cyclopentene. The ee determination by HPLC was facilitated by the use of racemic material.

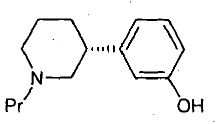

### (R)-2-(m-Anisyl)pentane-1,5-diol [racemate: 369650-35-1].¹⁴

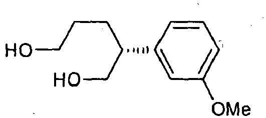

A solution of (*S*)-3-(*m*-anisyl) cyclopentene (175 mg, 1.0 mmol, 96% ee) in MeOH (30 mL) was chilled to -78 °C in a dry ice/acetone bath. Ozone was gently bubbled through the solution until the solution turned in pale blue. Excess ozone was blown off by a gentle stream of nitrogen. At -78°C, NaBH₄ (340 mg, 9 mmol) was added and the mixture was allowed to warm to RT. After stirring at RT for 1 h, the reaction was quenched by addition of saturated aqueous NH₄Cl solution (40 mL). The product was extracted with EtOAc (40 mL x 3). The combined organic portions were washed with brine, dried over Na₂SO₄ and concentrated to give the crude product. Purification by flash chromatography (silica gel, 10:1 CH₂Cl₂/MeOH) afforded the product as colorless oil in 79% yield (166 mg) in 96% ee. The material was used directly in the next step.

### Cyclization of the diol with propylamine.

Under argon, (*R*)-2-(*m*-anisyl)pentane-1,5-diol (152 mg, 0.7 mmol), dry CH₂Cl₂ (10 ml) and NEt₃ (0.40 g, 4 mmol) were charged into a 100-mL Schlenk flask. The solution was chilled to 0°C, followed by addition of MsCI (230 mg, 2.0 mmol). The resulting mixture was stirred at RT for 4 h, and it was then quenched by addition of saturated aqueous NaHCO₃ solution (20 mL). Extraction with CH₂Cl₂ (20 mL x3), drying over Na₂SO₄ and concentration on a rotavapor afforded the crude dimesylate. Dimesylate was directly mixed with *n*-PrNH₂ (0.65 g, 11 mmol) and stirred at RT for 1 d. The resulting mixture was diluted with CH₂Cl₂ (10 mL) and washed with saturated aqueous Na₂CO₃ solution. Purification by flash chromatography (silica gel, 20:1 to 10:1 CH₂Cl₂/MeOH) afforded *O*-methylpreclamol in 76% yield (130 mg) as white waxy solid in 96% ee.

### O-Demethylation.

O-Methylpreclamol (117 mg, 0.5 mmol) was mixed with 48% HBr (2 mL) under argon in a 25-mL reaction tube. The mixture was stirred at 120 °C for 2 h. The resulting mixture was cooled to RT, diluted with CH₂Cl₂ (10 mL) and quenched with saturated Na₂CO₃ (5 mL). The organic layer was separated and the aqueous layer was extracted with CH₂Cl₂ (5 mL x 6). Combined organic portions were dried over Na₂SO₄ and concentrated to give the product as colorless oil in 95% yield (105 mg) in 96% ee. The product was NMR-pure and no further purification was performed.
[α]²⁰_{D} = +23.2° (*c* = 1.0, CHCl₃). Lit. value for its HCl salt: +7.4o in MeOH. Ee: 96%. Daicel Chiralcel AD-H, 90:10:0.1 hexanes/isopropanol/NEt₃, flow rate = 0.5 mL/min. TR = 11.7 min (major) and 13.0 min (minor).
¹H NMR (400 MHz, CDCl₃): 7.18 (ψt, *J* = 7.8 Hz, 1H), 6.81-6.80 (m, 1H), 6.76-6.70 (m, 2H), 3.23 (d, *J* = 11.9 Hz, 1H), 3.07 (d, *J* = 11.2 Hz, 1H), 2.98-2.90 (m, 1H), 2.45-2.29 (m, 2H), 2.04-1.95 (m, 3H), 1.86-1.71 (m, 2H), 1.62-1.45 (m, 3H), 0.87 (t, *J* = 7.4 Hz, 3H).
¹³C NMR (100 MHz, CDCl₃): 156.9, 145.6, 130.0, 117.6, 114.8, 114.3, 61.6, 61.4, 54.2, 42.0, 30.0, 25.4, 19.4, 12.2.

### (S)-N-Boc-2-(p-fluorophenyl)pyrrolidine [CAS 174310-77-1].²²

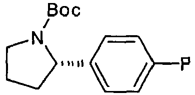

It was prepared via asymmetric Heck reaction, followed by catalytic hydrogenation. Under argon, Pd(dba)₂ (7.2 mg, 0.013 mmol) and (*R*)-Xyl-SDP(O) (10.8 mg, 0.015 mmol) were stirred in 1,4-dioxane (0.50 mL) in a 10-mL reaction tube for 30 min, followed by addition of *n-*C₁₄H₃₀ (25 *µ*L), 4- fluorophenyl triflate (122 mg, 0.50 mmol), *N*-ethyldiisopropylamine (170 *µ*L, 1.0 mmol) and *N*-Boc-2,3-dihydro-1*H*-pyrrole (169 mg, 1.0 mmol). The mixture was stirred at 50 °C in an oil bath. The reaction completed in 38 h. The reaction mixture was diluted with hexane (2 mL) and was filtered through cotton wool. The ee of this crude product was determined to be 98.6%. Daicel Chiralcel IC, 98:2 hexane/isopropanol, flow rate = 0.5 mL/min. TR = 22.2 min (minor) and 25.0 min (major).

The filtrate was used in catalytic hydrogenation directly. It was mixed with wet 5% wt/wt Pd/C (85 mg, 8 mol%) and was subjected to hydrogenation in a 125-mL Parr bomb with H₂ (80 psi) for 2 h at RT. The resulting mixture was subjected to flash chromatography (silica gel, 10:1 hexane/EtOAc). The product was isolated as pale yellow oil (96 mg, 73% yield over 2 steps) with 98% ee.
[α]²⁰_{D} = -64° (*c* = 1.5, CHCl₃).
Daicel Chiralcel IC, 90:10 hexane/isopropanol, flow rate = 0.5 mL/min. TR = 13.0 min (minor) and 15.1 min (major).
¹H NMR for 2 rotamers with 35:65 ratio (400 MHz, CDCl₃): 7.14-7.12 (m, 2H), 7.01-6.95 (m, 2H), 4.92 (br s, 0.35H), 4.74 (br s, 0.65H), 3.61 (br, 1H), 2.31 (br, 1H), 1.94-1.75 (m, 3H), 1.46 (br, 3.1H), 1.19 (br, 5.9H).
¹³C NMR of 2 rotamers with 35:65 ratio (100 MHz, CDCl₃): 161.7 (d, *J* C-F = 244.0 Hz), 154.7, 141.0, 127.1 (d, *J*C-F = 7.2 Hz), 115.0 (d, *J*C-F = 21.0 Hz), 79.5, 60.9, 60.3, 47.2, 36.2, 35.1, 28.7, 28.6, 28.3, 23.5, 23.3.
¹⁹F{¹H} NMR (376 MHz, CDCl₃): -116.9 (s, 0.65F), -117.1(s, 0.35F).

### II. EXAMPLES OF ASYMMETRIC HECK REACTIONS USING ARYL, HETEROARYL OR ALKENYL HALIDES AND OLEFINS

### A. Condition optimization of asymmetric Heck reaction of aryl halides

General procedure for condition optimization: In an argon-filled glove box, Pd(dba)₂ (1.4 mg, 0.0025 mmol) and (*R*)-Xyl-SDP(O) (2.1 mg, 0.003 mmol) were stirred in ethylene glycol (0.2 mL) for 10-20 min in a 10-mL reaction tube, followed by successive addition of *p*-tolyl bromide (17.1 mg, 0.10 mmol), *n*-dodecane (10 *µ*L), *N*-diisopropylethylamine (51 *µ*L, 0.3 mmol, 3 equiv), *p-*nitrobenzoic acid (16.7 mg, 0.1 mmol, 1 equiv) and 2,3-dihydrofuran (15 mL, 0.2 mmol, 2 equiv). The mixture was vigorously stirred in a preheated oil bath at 80°C for 12 h. The reaction mixture was cooled to room temperature, diluted by EtOH and passed through a short plug of silical gel with diethyl ether washing. The filtrate was used in GC analysis to determine the conversion of ArBr and GC yield and isomeric selectivity or s selectivity, of the desired Heck product. For the determination of enantioselectivity of main Heck product, the sample was dissolved in 1: 10 *i*PrOH/hexanes and subjected to chiral HPLC analysis (Daicel CHIRCEL IC-H; 2% *i*PrOH in hexanes; 0.5 mL/min).

**Table S2. Effect of chiral ligands**

| | | | | | |
|---|---|---|---|---|---|
| 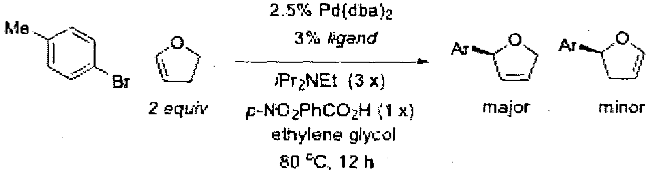 | | | | | |

| Entry | Ligand | Conv (%) | GC yield (%) | Selectivity s | ee (%) |
|---|---|---|---|---|---|
| 1 | (*R*)-Xyl-SDP(O) | 100 | 93 | 7 | 95 |
| 2 | (*R*)-Ph-SDP(O) | 80 | 67 | 5 | 92 |
| 3 | (*R*)-Xyl-Cy-SDP(O) | 83 | 40 | 1 | 45 |
| 4 | (*R*)-Ph-SDP | 80 | 40 | 1:1.3 | <5 |
| 5 | (*R*)-Xyl-SDP | 90 | 40 | 1:2 | <5 |
| 6 | (*R*)-BINAP(O) | 90 | 73 | 3 | -73 |
| 7 | (*R*)-Xyl-BINAP(O) (6 h) | 100 | 90 | 6 | -58 |
| 8 | (*R*)-BINAP | 60 | 20 | 1:3.6 | <5 |
| 9 | (*S*)-*t*Bu-PHOX | 20 | 10 | 20 | -85 |
| 10 | (*S*)-Ph-PHOX | 20 | 7 | 2 | - |

### B. Isolation of Heck products

**General procedure for asymmetric Heck reactions of 2,3-dihydrofuran:** In an argon-filled glove box, Pd(dba)₂ (7.2 mg, 0.013 mmol) and (*R*)-Xyl-SDP(O) (10.8 mg, 0.015 mmol) were stirred in degassed ethylene glycol (1.0 mL) for 10-20 min in a 10-mL reaction tube, followed by the addition of aryl bromide (0.50 mmol), *N*-diisopropylethylamine (255 *µ*L, 1.5 mmol, 3 equiv), *p*-nitrobenzoic acid (83.6 mg, 0.5 mmol, 1 equiv) and 2,3-dihydrofuran (75 *µ*L, 1.0 mmol, 2 equiv). The mixture was vigorously stirred in a preheated oil bath at 80 °C, until aryl bromide was fully consumed (monitored by GC). The reaction mixture was cooled to room temperature and subjected to flash chromatography (pentane/Et₂O) to give the purified product. The olefinic selectivity, or s selectivity, of the desired Heck isomer versus other isomers combined in the crude mixture was determined by GC. The enantioselectivity (ee) of the purified product was determined by chiral HPLC analysis using Daicel Chiralcel columns. The racemic Heck products were prepared by using racemic Xyl-SDP(O) ligand.

### (S)-2-(1-Naphthyl)-2,5-dihydrofuran [131516-16-0].

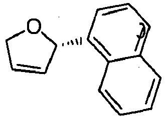

Pd(dba)₂ (7.2 mg, 0.013 mmol), (*R*)-Xyl-SDP(O) (10.8 mg, 0.015 mmol), ethylene glycol (1.0 mL), 1-bromonaphthalene (103.5 mg, 0.50 mmol), *N*-diisopropylethylamine (255 *µ*L, 1.5 mmol), *p*-nitrobenzoic acid (83.6 mg, 0.5mmol) and 2,3-dihydrofuran (75 *µ*L, 1.0 mmol) were used. The reaction completed in 10 h at 80°C. The product was isolated by flash chromatography (10:1 to 3:1 pentane/Et₂O) as colorless oil. Yield: 90 mg, 92%. Olefinic selectivity in the crude product: 12:1.
[*α*]²³_{D} = -162.3° (*c* = 0.72, CHCl₃).
GC-MS (EI): Calcd for C₁₄H₁₂O M⁺: 196.1. Found: 196.0.
Ee: 97%. Daicel Chiralcel OD-H, 98:2 *n*-hexane/isopropanol.
¹H NMR (400 MHz, CDCl₃): *δ* 8.11 (d, *J* = 8.3 Hz, 1H), 7.86-7.84 (m, 1H), 7.76 (d, *J* = 8.1 Hz, 1H), 7.53-7.42 (m, 4H), 6.56-6.53 (m, 1H), 6.13-6.10 (m, 1H), 6.06-6.03 (m, 1H), 4.95-4.83 (m, 2H).

### (S)-2-(2-Naphthyl)-2,5-dihydrofuran [131516-16-0].

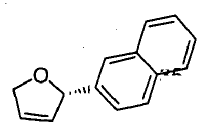

Pd(dba)₂ (7.2 mg, 0.013 mmol), (*R*)-Xyl-SDP(O) (10.8 mg, 0.015 mmol), ethylene glycol (1.0 mL), 2-bromonaphthalene (103.5 mg, 0.50 mmol), *N*-diisopropylethylamine (255 µL, 175 mmol), *p*-nitrobenzoic acid (83.6 mg, 0.5mmol) and 2,3-dihydrofuran (75 *µ*L, 1.0 mmol) were used. The reaction completed in 6 h at 80°C. The product was isolated by flash chromatography (10:1 to 3:1 pentane/Et₂O) as colorless oil. Yield: 85 mg, 86%. Olefinic selectivity in the crude product: 12:1.
[*α*]²³_{D} = -195.0° (*c* = 0.58, CHCl₃).
GC-MS (EI): Calcd for C₁₄H₁₂O M⁺: 196.1. Found: 196.0.
Ee: 96%. Daicel Chiralcel OD-H, n-hexane/isopropanol 98/2, flow rate = 0.5 mL/min.
¹H NMR (400 MHz, CDCl₃): *δ* 7.84-7.80 (m, 3H), 7.76 (s, 1H), 7.48-7.44 (m, 2H), 7.41 (d, *J* = 8.5 Hz, 1H), 6.09-6.06 (m, 1H), 5.96-5.93 (m, 2H), 4.98-4.92 (m, 1H), 4.85-4.80 (m, 1H).

### (S)-2-phenyl-2,5-dihydrofuran [131516-10-4].

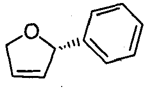

Pd(dba)₂ (7.2 mg, 0.013 mmol), (*R*)-Xyl-SDP(O) (10.8 mg, 0.015 mmol), ethylene glycol (1.0 mL), bromobenzene (78.5 mg, 0.50 mmol), *N-*diisopropylethylamine (255 µL, 1.5 mmol), p-nitrobenzoic acid (84 mg, 0.5 mmol) and 2,3-dihydrofuran (75 µL, 1.0 mmol) were used. The reaction completed in 6 h at 80 °C. The product was isolated by flash chromatography with pentane/Et₂O (10:1 to 5:1) as colorless oil. Yield: 62 mg, 84%. Olefinic selectivity in the crude product: 11:1.
[*α*]²³_{D} = -236.3° (*c* = 0.80, CHCl₃).
GC-MS (EI): Calcd for C₁₀H₁₀O M⁺: 146.1. Found: 146.0
Ee: 97%. Daicel Chiralcel OJ-H, 98:2 *n*-hexane/isopropanol, flow rate = 0.5 mL/min.
¹H NMR (400 MHz, CDCl₃): *δ* 7.36-7.25 (m, 5H), 6.05-6.02 (m, 1H), 5.90-5.87 (m, 1H), 5.80-5.77 (m, 1H), 4.90-4.84 (m, 1H), 4.79-4.74 (m, 1H).

Heck reaction of Phl: Pd(dba)₂ (1.4 mg, 0.0026 mmol), (*R*)-Xyl-SDP(O) (2.2 mg, 0.003 mmol), ethylene glycol (0.2 mL), iodobenzene (20.4 mg, 0.10 mmol), *N*-diisopropylethylamine (51 *µ*L, 0.3 mmol), *p*-nitrobenzoic acid (16.7 mg, 0.1 mmol), 10 *µ*L *n*-tetradecane and 2,3-dihydrofuran (15 *µ*L, 0.2 mmol) were used. The reaction was heated in 80 °C for 6 h. The reaction mixture was cooled to room temperature, diluted by EtOH and passed through a short plug of silical gel with diethyl ether washing. The filtrate was used in GC analysis to determine the conversion of Phl and GC yield and isomeric selectivity or s selectivity, of the desired Heck product. Conversion: 90%. GC yield: 50%. Olefinic selectivity in the crude product: 1:2. Ee of major isomer: 80%.

### (S)-2-(p-Anisyl)-2,5-dihydrofuran [131516-15-9].

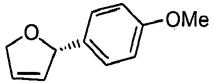

Pd(dba)₂ (7.2 mg, 0.013 mmol), (*R*)-Xyl-SDP(O) (10.8 mg, 0.015 mmol), ethylene glycol (1.0 mL), 4-bromoanisole (93.5 mg, 0.50 mmol), *N*-diisopropylethylamine (255 µL, 1.5 mmol), *p*-nitrobenzoic acid (83.6 mg, 0.5mmol) and 2,3-dihydrofuran (75 µL, 1.0 mmol) were used. The reaction completed in 3h at 80°C. The product was isolated by flash chromatography (10:1 to 5:1 pentane/Et₂O) as colorless liquid. Yield: 82 mg, 93%. Olefinic selectivity in the crude product: 14:1.
[*α*]²²_{D} = -208.9° (*c* = 0.70, CHCl₃).
GC-MS (EI): Calcd for C₁₁H₁₂O₂ M⁺: 176.1. Found: 176.0.
Ee: 98%. Daicel Chiralcel OD-H, 98:2 *n*-hexane/isopropanol, flow rate = 0.5 mL/min.
¹H NMR (400 MHz, CDCl₃): *δ* 7.24-7.20 (m, 2H), 6.89-6.86 (m, 2H), 6.04-6.02 (m, 1H), 5.87-5.84 (m, 1H), 5.76-5.72 (m, 1H), 4.87-4.81 (m, 1H), 4.75-4.70 (m, 1H), 3.79 (s, 3H).

### (S)-2-(p-Tolyl)-2,5-dihydrofuran [1041861-52-2].

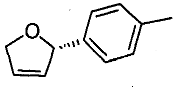

Pd(dba)₂ (7.2 mg, 0.013 mmol), (*R*)-Xyl-SDP(O) (10.8 mg, 0.015 mmol), ethylene glycol (1.0 mL), 4-bromotoluene (85.5 mg, 0.50 mmol), *N-*diisopropylethylamine (255 µL, 1.5 mmol), p-nitrobenzoic acid (83.6 mg, 0.5 mmol) and 2,3-dihydrofuran (75 µL, 1.0 mmol) were used. The reaction completed in 6 h at 80 °C. The product was isolated by flash chromatography (10:1 to 5:1 pentane/Et₂O) as a colorless liquid. Yield: 68 mg, 85%. Olefinic selectivity in the crude product: 10:1.
GC-MS (EI): Calcd for C₁₁H₁₂O M⁺: 160.1. Found: 160.1.
Ee: 96%. Daicel Chiralcel IC-H, 98:2 *n*-hexane/isopropanol, flow rate = 0.5 mL/min.

Heck reaction of ArCI: Pd(dba)₂ (14.4 mg, 0.025 mmol), (*R*)-Xyl-SDP(O) (21.6 mg, 0.030 mmol), ethylene glycol (1.0 mL), 4-chlorotoluene (63.3 mg, 0.50 mmol), *N*-diisopropylethylamine (255 *µ*L, 1.5 mmol), *p*-nitrobenzoic acid (83.6 mg, 0.5 mmol) and 2,3-dihydrofuran (150 *µ*L, 2.0 mmol) were used. The reaction completed in 55 h at 80°C. The product was isolated by flash chromatography (10:1 to 5:1 pentane/Et₂O) as a colorless liquid. Yield: 64 mg, 80%. Olefinic selectivity in the crude product: 7:1.
Ee: 95%. Daicel Chiralcel IC-H, 99:1 *n*-hexane/isopropanol, flow rate = 0.5 mL/min.
¹H NMR (400 MHz, CDCl₃): *δ* 7.20-7.14 (m, 4H), 6.03-6.00 (m, 1H), 5.88-5.85 (m, 1H), 5.77-5.75 (m, 1H), 4.88-4.82 (m, 1H), 4.77-4.72 (m, 1H).

### (S)-2-(4-Fluorophenyl)-2,5-dihydrofuran [184047-35-6].

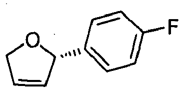

Pd(dba)₂ (7.2 mg, 0.013 mmol), (R)-Xyl-SDP(O) (10.8 mg, 0.015 mmol), ethylene glycol (3.0 mL), 1-bromo-4-fluorobenzene (87.5 mg, 0.50 mmol), *N*-diisopropylethylamine (255 µL, 1.5 mmol), p-nitrobenzoic acid (83.6 mg, 0.5 mmol) and 2,3-dihydrofuran (75 µL, 1.0 mmol) were used. The reaction completed in 19 h at 70 °C. The product was isolated by flash chromatography (10:1 to 5:1 pentane/Et₂O) as colorless oil. Yield: 68 mg, 82%.
Olefinic selectivity in the crude product: 13:1.
[*α*]²²_{D} = -187.3° (*c* = 1.00, CHCl₃).
GC-MS (EI): Calcd for C₁₀H₉FO M⁺: 164.1. Found: 164.0.
Ee: 98%. Daicel Chiralcel OJ-H, 99:1 *n*-hexane/isopropanol, flow rate = 0.5 mL/min.

Heck reaction of ArCl: Pd(dba)₂ (14.4 mg, 0.025 mmol), (*R*)-Xyl-SDP(O) (21.6 mg, 0.030 mmol), ethylene glycol (1.0 mL), 1-chloro-4-fluorobenzene (65.3 mg, 0.50 mmol), *N-*diisopropylethylamine (255 *µ*L, 1.5 mmol), *p*-nitrobenzoic acid (83.6 mg, 0.5 mmol) and 2,3-dihydrofuran (150 *µ*L, 2.0 mmol) were used. The reaction was complete in 45 h at 80°C. The product was isolated by flash chromatography (10:1 to 5:1 pentane/Et₂O) as a colorless liquid. Yield: 62 mg, 75%. Olefinic selectivity in the crude product: 7:1.
ee: 94%. Daicel Chiralcel OJ-H, 99:1 *n*-hexane/isopropanol, flow rate = 0.5 mL/min.
¹H NMR (400 MHz, CDCl₃): *δ* 7.29-7.24 (m, 2H), 7.04-6.99 (m, 2H), 6.05-6.02 (m, 1H), 5.86-5.83 (m, 1H), 5.77-5.74 (m, 1H), 4.87-4.82 (m, 1H), 4.77-4.72 (m, 1H).

### (S)-2-(o-Anisyl)-2,5-dihydrofuran [1461737-18-7].

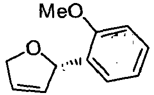

Pd(dba)₂ (7.2 mg, 0.013 mmol), (*R*)-Xyl-SDP(O) (10.8 mg, 0.015 mmol), ethylene glycol (1.0 mL), 2-bromoanisole (93.5 mg, 0.50 mmol), *N-*diisopropylethylamine (255 *µ*L, 1.5 mmol), p-nitrobenzoic acid (83.6 mg, 0.5 mmol) and 2,3-dihydrofuran (75 µL, 1.0 mmol) were used. The reaction completed in 3 h at 80 °C. The product was isolated by flash chromatography (10:1 to 5:1 pentane/Et₂O) as colorless oil. Yield: 76 mg, 86%. Olefinic selectivity in the crude product: 28:1.
[*α*]²²_{D} = -270.0° (*c* = 0.73, CHCl₃).
GC-MS (EI): Calcd for C₁₁H₁₂O₂ M⁺: 176.1. Found: 176.0.
Ee: 97%. Daicel Chiralcel OJ-H, 98:2 *n*-hexane/isopropanol, flow rate = 0.5 mL/min.
¹H NMR (400 MHz, CDCl₃): *δ* 7.34 (dd, *J* = 7.5, 0.5 Hz, 1H), 7.26-7.22 (m, 1H), 6.95 (t, *J* = 7.4 Hz, 1H), 6.87 (d, *J* = 8.2 Hz, 1H), 6.13-6.13 (m, 1H), 6.00-5.93 (m, 2H), 4.89-4.83 (m, 1H), 4.81-4.75 (m, 1H), 3.85 (s, 3H).

### (S)-2-(2-Methyl-5-benzothiazolyl)-2,5-dihydrofuran.

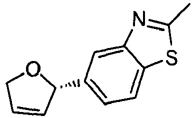

Pd(dba)₂ (7.2 mg, 0.013 mmol), (*R*)-Xyl-SDP(O) (10.8 mg, 0.015 mmol), ethylene glycol (1.0 mL), 5-bromo-2-methyl-1,3-benzothiazole (114.1 mg, 0.50 mmol), *N*-diisopropylethylamine (255 µL, 1.5 mmol), *p*-nitrobenzoic acid (83.6 mg, 0.5 mmol) and 2,3-dihydrofuran(75 µL, 1.0 mmol) were used. The reaction completed in 7 h at 80°C. The product was isolated by flash chromatography (10:1 to 1:1 pentane/Et₂O) as colorless oil. Yield: 87 mg, 80%. Olefinic selectivity in the crude product: 10:1.
GC-MS (EI): Calcd for C₁₂H₁₁NOS M⁺: 217.1. Found: 217.0.
Ee: 95%. Daicel Chiralcel AS-H, 95:5 *n*-hexane/isopropanol, flow rate = 0.5 mL/min.
¹H NMR (500 MHz, CDCl₃): *δ* 7.88 (d, *J* = 1.4 Hz, 1H), 7.78 (d, *J* = 8.3 Hz, 1H), 7.30 (dd, *J* = 8.3, 1.4 Hz, 1H), 6.07-6.05 (m, 1H), 5.93-5.91 (m, 1H), 4.93-4.89 (m, 1H), 4.82-4.78 (m, 1H), 2.82 (s, 3H).
¹³C NMR (100 MHz, CDCl₃): *δ* 167.4, 153.7, 140.5, 135.0, 129.9, 126.9, 123.3, 121.4, 120.2, 87.8, 75.9, 20.2.

### (S)-2-(3-Benzofuryl)-2,5-dihydrofuran.

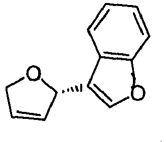

Pd(dba)₂ (7.2 mg, 0.013 mmol), (*R*)-Xyl-SDP(O) (10.8 mg, 0.015 mmol), ethylene glycol (3.0 mL), 3-bromobenzofuran (98.5 mg, 0.50 mmol), *N*-diisopropylethylamine (255 µL, 1.5 mmol), *p*-nitrobenzoic acid (167 mg, 1.0 mmol) and 2,3-dihydrofuran (75 µL, 1.0 mmol) were used. The reaction completed in 27 h at 50 °C. The product was isolated by flash chromatography (10:1 to 5:1 pentane/Et₂O) as colorless oil. Yield: 75 mg, 80%. Olefinic selectivity in the crude product: 8:1. When 1.0 equiv of *p*-nitrobenzoic acid and 1.0 mL of ethylene glycol were used, olefinic selectivity was only 7:1.
[*α*]²²_{D} = -163.2° (*c* = 0.92, CHCl₃).
GC-MS (EI): Calcd for C₁₂H₁₀O₂ M⁺: 186.1. Found: 186.0.
Ee: 97%. Daicel Chiralcel OD-H, 99:1 *n*-hexane/isopropanol, flow rate = 0.5 mL/min.
¹H NMR (400 MHz, CDCl₃): *δ* 7.58 (d, *J* = 7.5 Hz, 1H), 7.56 (s, 1H), 7.46 (d, *J*= 8.2 Hz, 1H), 7.30-7.26 (m, 1H), 7.24-7.20 (m, 1H), 6.13-6.10 (m, 1H), 6.04-6.00 (m, 1H), 6.00-5.97 (m, 1H), 4.92-4.86 (m, 1H), 4.82-4.77 (m, 1H).
¹³C NMR (100 MHz, CDCl₃): *δ* 155.9, 142.1, 128.1, 127.8, 126.2, 124.5, 122.7, 121.3, 120.3, 111.6, 79.9, 75.5.

### (S)-2-(3-Benzothienyl)-2,5-dihydrofuran.

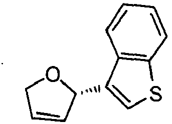

Pd(dba)₂ (7.2 mg, 0.013 mmol), (*R*)-Xyl-SDP(O) (10.8 mg, 0.015 mmol), ethylene glycol (3.0 mL), 3-bromobenzothiophene (106.5 mg, 0.50 mmol), *N-*diisopropylethylamine (255 µL, 1.5 mmol), p-nitrobenzoic acid (167 mg, 1.0 mmol, 2 equiv) and 2,3-dihydrofuran (75 µL, 1.0 mmol) were used. The reaction completed in 30 h at 50°C. The product was isolated by flash chromatography (10:1 to 5:1 pentane/Et₂O) as colorless oil. Yield: 83 mg, 82%. Olefinic selectivity in the crude product: 20:1. When 1.0 equiv of *p*-nitrobenzoic acid and 1.0 mL of ethylene glycol was used, olefinic selectivity was only 5:1.
[*α*]²²_{D} = -151.8° (*c* = 0.97, CHCl₃).
GC-MS (EI): Calcd for C₁₂H₁₀OS M⁺: 202.1. Found: 202.0.
Ee: 98%. Daicel Chiralcel OD-H, 98:2 *n*-hexane/isopropanol, flow rate = 0.5 mL/min.
¹H NMR (400 MHz, CDCl₃): *δ* 7.86-7.83 (m, 2H), 7.40-7.33 (m, 3H), 6.17-6.14 (m, 1H), 6.13-6.10 (m, 1H), 6.09-6.05 (m, 1H), 4.92-4.86 (m, 1H), 4.84-4.79 (m, 1H).
¹³C NMR (100 MHz, CDCl₃): *δ* 141.1, 137.5, 136.8, 128.3, 127.7, 124.4, 124.1, 123.3, 122.9, 122.2, 83.0, 75.5.

### (S)-2-(2-Thienyl)-2,5-dihydrofuran [1335151-41-1].

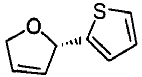

Pd(dba)₂ (7.2 mg, 0.013 mmol), (*R*)-Xyl-SDP(O) (10.8 mg, 0.015 mmol), ethylene glycol (1.0 mL), 2-bromothiophene (81.5 mg, 0.50 mmol), *N-*diisopropylethylamine (255 *µ*L, 1.5 mmol), *p*-nitrobenzoic acid (83.6 mg, 0.5 mmol) and 2,3-dihydrofuran (75 *µ*L, 1.0 mmol) were used. The reaction completed in 24 h at 50°C. The product was isolated by flash chromatography (10:1 to 5:1 pentane/Et₂O) as colorless oil.
Yield: 61 mg, 80%. Olefinic selectivity in the crude product: 9:1.
GC-MS (EI): Calcd for C₈H₈OS M⁺: 152.0. Found: 152.0.
[*α*]²²_{D} = -147.8° (*c* = 1.28, CHCl₃).
Ee: 90%. Daicel Chiralcel OD-H, 98:2 *n*-hexane/isopropanol, flow rate = 0.5 mL/min.
¹H NMR (400 MHz, CDCl₃): *δ* 7.27-7.25 (m, 1H), 7.00-7.00 (m, 1H), 6.98-6.96 (m, 1H), 6.10-6.07 (m, 1H), 6.06-6.03 (m, 1H), 5.95-5.92 (m, 1H), 4.86-4.80 (m, 1H), 4.73-4.68 (m, 1H).
¹³C NMR (100 MHz, CDCl₃): *δ* 145.9, 129.1, 127.7, 126.8, 125.5, 124.6, 82.8, 75.1.

### (S)-(E)-2-(2-Styryl)-2,5-dihydrofuran [CAS for racemate: 119946-59-7].

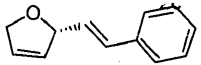

Pd(dba)₂ (7.2 mg, 0.013 mmol), (*R*)-Xyl-SDP(O) (10.8 mg, 0.015 mmol), ethylene glycol (3.0 mL), 2-bromothiophene (91.5 mg, 0.50 mmol), *N-*diisopropylethylamine (255 *µ*L, 1.5 mmol), *p*-nitrobenzoic acid (83.6 mg, 0.5 mmol) and 2,3-dihydrofuran (75 *µ*L, 1.0 mmol) were used. The reaction completed in 9 h at 40°C. The product was isolated by flash chromatography (10:1 to 5:1 pentane/Et₂O) as colorless oil. Yield: 61 mg, 70%. Olefinic selectivity in the crude product: 11:1.
GC-MS (EI): Calcd for C₁₂H₁₂O M⁺: 172.1. Found: 172.0.
[*α*]²²_{D} = -200.0° (*c* = 0.97, CHCl₃).
Ee: 88%. Daicel Chiralcel OD-H, 98:2 *n*-hexane/isopropanol, flow rate = 0.5 mL/min.
¹H NMR (400 MHz, CDCl₃): *δ* 7.40-7.35 (m, 2H), 7.32-7.28 (m, 2H), 7.26-7.21 (m, 1H), 6.60 (d, *J*= 15.8 Hz, 1H), 6.16 (dd, *J*= 15.8, 7.2 Hz, 1H), 5.99-5.96 (m, 1H), 5.83-5.80 (m, 1H), 5.41-5.37 (m, 1H), 4.80-4.75 (m, 1H), 4.71-4.66 (m, 1H).
¹³C NMR (100 MHz, CDCl₃): *δ* 136.7, 130.9, 129.3, 128.9, 128.5, 127.7, 127.1, 126.6, 86.8, 75.3.

### (S)-2-(o-Tolyl)-2,5-dihydrofuran [1361252-01-8].

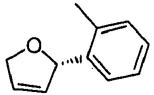

Pd(dba)₂ (14.4 mg, 0.025 mmol), (*R*)-Xyl-SDP(O) (21.6 mg, 0.030 mmol), ethyl glycol (1.0 mL), 2-chlorotoluene (63.3 mg, 0.50 mmol), *N*-diisopropylethylamine (255 *µ*L, 1.5 mmol) *p*-nitrobenzoic acid (84 mg, 0.5 mmol) and 2,3-dihydrofuran (150 *µ*L, 2.0 mmol) were used. The reaction was complete in 55 h at 80°C. The product was isolated by flash chromatography (10:1 to 5:1 pentane/Et₂O) as colorless oil. Yield: 67 mg, 81%. Olefinic selectivity in the crude product: 5:1.
GC-MS (EI): Calcd for C₁₁H₁₂O M⁺: 160.1. Found: 160.1.
[*α*]²²_{D} = -205.0° (*c* = 0.89, CHCl₃).
Ee: 97%. Daicel Chiralcel OD-H, 98:2 *n*-hexane/isopropanol, flow rate = 0.5 mL/min.
¹H NMR (500 MHz, CDCl₃): *δ* 7.31-7.29 (m, 1H), 7.21-7.13 (m, 3H), 6.04-6.02 (m, 2H), 5.93-5.90 (m, 1H), 4.89-4.85 (m, 1H), 4.80-4.76 (m, 1H), 2.39 (s, 3H).

**C. General procedure for asymmetric Heck reaction of cyclopentene:** In an argon-filled glove box, Pd(dba)₂ (14.4 mg, 0.025 mmol) and (*R*)-Xyl-SDP(O) (21.6 mg, 0.030 mmol) were stirred in degassed methanol (1.0 mL) for 10 to 20 min in a 10-mL reaction tube, followed by successive addition of aryl bromide (0.50 mmol), *N*-diisopropylethylamine (255 *µ*L, 1.5 mmol, 3 equiv), *p*-nitrobenzoic acid (83.6 mg, 0.5 mmol, 1 equiv) and cyclopentene (180 *µ*L, 2.0 mmol, 4 equiv). The mixture was vigorously stirred in a preheated oil bath at 80°C, until the aryl bromide was fully consumed (monitored by GC). The reaction mixture was cooled to room temperature and subjected to flash chromatography (pentane/Et₂O) to give the purified product. Olefinic selectivity of the product in the crude mixture was determined by GC and GCMS.

### (S)-3-(1-Naphthyl)cyclopentene [1461737-02-9].

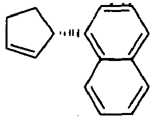

Pd(dba)₂ (14.4 mg, 0.025 mmol), (*R*)-Xyl-SDP(O) (21.6 mg, 0.030 mmol), methanol (1.0 mL), 1-naphthyl bromide (103.5 mg, 0.50 mmol), *N*-diisopropylethylamine (255 *µ*L, 1.5 mmol), *p-*nitrobenzoic acid (83.6 mg, 0.5 mmol) and cyclopentene (180 *µ*L, 2.0 mmol) were used. The reaction completed in 32 h at 80°C. The product was isolated by flash chromatography (pentane) as colorless oil. Yield: 94 mg, 95%. Olefinic selectivity in the crude product: >50:1.
[*α*]²³_{D} = +98.1° (*c* = 0.78, CHCl₃).
GC-MS (EI): Calcd for C₁₅H₁₄ M⁺: 194.1. Found: 194.1.
Ee: 98%. Daicel Chiralcel OJ-H, 98:2 hexanes/isopropanol, flow rate = 0.5 mL/min.
¹H NMR (400 MHz, CDCl₃): *δ* 8.16 (d, *J* = 8.3 Hz, 1H), 7.86-7.84 (m, 1H), 7.70 (d, *J* = 8.1 Hz, 1H), 7.53-7.45 (m, 2H), 7.41-7.38 (m, 1H), 7.31 (d, *J* = 7.1 Hz, 1H), 6.07-6.04 (m, 1H), 5.96-5.93 (m, 1H), 4.68-4.63 (m, 1H), 2.66-2.57 (m, 1H), 2.52-2.46 (m, 2H), 1.82-1.74 (m, 1H).

### (S)-3-(2-Naphthyl)cyclopentene [1461737-03-0].

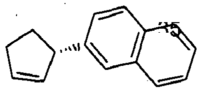

Pd(dba)₂ (14.4 mg, 0.025 mmol), (*R*)-Xyl-SDP(O) (21.6 mg, 0.030 mmol), methanol (1.0 mL), 2-naphthyl bromide (103.5 mg, 0.50 mmol), *N*-diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction completed in 12 h at 80°C. The product was isolated by flash chromatography with pentane as colorless oil. Yield: 89 mg, 91%. Olefinic selectivity in the crude product: 21:1.
[*α*]²³_{D} = -199.9° (*c* = 0.78, CHCl₃).
GC-MS (EI): Calcd for C₁₅H₁₄ M⁺: 194.1. Found: 194.1.
Ee: 97%. Daicel Chiralcel OJ-H, 98:2 hexanes/isopropanol, flow rate = 0.5 mL/min.
¹H NMR (400 MHz, CDCl₃): *δ* 7.79-7.75 (m, 3H), 7.60 (s, 1H), 7.44-7.37 (m, 2H), 7.32 (d, *J* = 8.4 Hz, 1H), 6.00-5.98 (m, 1H), 5.86-5.82 (m, 1H), 4.07-4.03 (m, 1H), 2.60-2.39 (m, 3H), 1.85-1.75 (m, 1H).

### (S)-3-Phenylcyclopentene [38941-68-3].

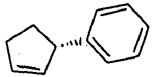

Pd(dba)₂ (14.4 mg, 0.025 mmol), (*R*)-Xyl-SDP(O) (21.6 mg, 0.030 mmol), methanol (1.0 mL), bromobenzene (78.5mg, 0.50 mmol), *N-*diisopropylethylamine (170 *µ*L, 1.5 mmol), *p*-nitrobenzoic acid (83.6 mg, 0.5 mmol) and cyclopentene (180 *µ*L, 2.0 mmol) were used. The reaction completed in 15 h at 80°C. The product was isolated by flash chromatography (silica gel, pentane) as colorless oil. Yield: 62.5 mg, 85%. Olefinic selectivity in the crude product: 43:1.
[*α*]²³_{D} = -170.9° (*c* = 0.62, CHCl₃).
GC-MS (EI): Calcd for C₁₁H₁₂ M⁺: 144.1. Found: 144.1.
Ee: 92%. Daicel Chiralcel OJ-H, 98:2 hexane/isopropanol, flow rate = 0.5 mL/min.
¹H NMR (400 MHz, CDCl₃): *δ* 7.30-7.27 (m, 2H), 7.20-7.16 (m, 3H), 5.95-5.92 (m, 1H), 5.79-5.76 (m, 1H), 3.91-3.87 (m, 1H), 2.56-2.35 (m, 3H), 1.77-1.69 (m, 1H).

Heck reaction of Phi: Pd(dba)₂ (2.9 mg, 0.005 mmol), (*R*)-Xyl-SDP(O) (4.3 mg, 0.006 mmol), MeOH (0.2 mL), iodobenzene (20.4 mg, 0.10 mmol), *N*-diisopropylethylamine (51 *µ*L, 0.3 mmol), *p*-nitrobenzoic acid (16.7 mg, 0.1 mmol), 10 *µ*L *n*-tetradecane and cyclopentene (36 *µ*L, 0.4 mmol) were used. The reaction was heated in 80°C for 16 h. The reaction mixture was cooled to room temperature, diluted by EtOH and passed through a short plug of silical gel with diethyl ether washing. The filtrate was used in GC analysis to determine the conversion of PhI and GC yield and isomeric selectivity or s selectivity, of the desired Heck product. Conversion of PhI: 100%. GC yield: 20%. Olefinic selectivity in the crude product: 50:1. Ee of major isomer: 90%.

### (S)-3-p-Tolylcyclopentene [38805-99-1].

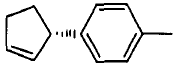

Pd(dba)₂ (14.4 mg, 0.025 mmol), (*R*)-Xyl-SDP(O) (21.6 mg, 0.030 mmol), methanol (1.0 mL), 4-bromotoluene (85.5 mg, 0.50 mmol), *N-*diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction was complete in 12 h at 80°C. The product was isolated by flash chromatography with pentane as colorless oil. Yield: 74 mg, 92%. Olefinic selectivity in the crude product: 42:1.
[*α*]²³_{D} = -174.2° (*c* = 0.90, CHCl₃).
GC-MS (EI): Calcd for C₁₂H₁₄ M⁺: 158.1. Found: 158.1.
Ee: 96%. Daicel Chiralcel OJ-H, 98:2 *n*-hexane/isopropanol, flow rate = 0.5 mL/min.
Heck reaction of ArCI: Pd(dba)₂ (14.4 mg, 0.025 mmol), (*R*)-Xyl-SDP(O) (21.6 mg, 0.030 mmol), ethylene glycol (1.0 mL), 4-chlorotoluene (63.3 mg, 0.50 mmol), *N*-diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction was complete in 120 h at 80 °C. The product was isolated by flash chromatography with pentane as colorless oil. Yield: 60 mg, 75%. Olefinic selectivity in the crude product: 38:1
Ee: 85%. Daicel Chiralcel OJ-H, 99:1 *n*-hexane/isopropanol, flow rate = 0.5 mL/min.
¹H NMR (400 MHz, CDCl₃): *δ* 7.16-7.06 (m, 4H), 5.92-5.90 (m, 1H), 5.77-5.75 (m, 1H), 3.88-3.83 (m, 1H), 2.54-2.45 (m, 1H), 2.43-2.36 (m, 2H), 2.31 (s, 3H), 1.74-1.66 (m, 1H)
¹³C NMR (100 MHz, CDCl₃): *δ* 143.5, 135.5, 134.5, 131.7, 129.1, 127.1, 50.9, 33.9, 32.5, 21.0.

### (S)-3-(p-Anisyl)cyclopentene [1462335-55-2].

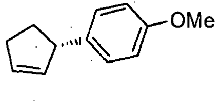

Pd(dba)₂ (14.4 mg, 0.025 mmol), (*R*)-Xyl-SDP(O) (21.6 mg, 0.030 mmol), methanol (1.0 mL), 4-bromoanisole (93.5 mg, 0.50 mmol), *N-*diisopropylethylamine (255 *µ*L, 1.5 mmol), *p*-nitrobenzoic acid (83.6 mg, 0.5 mmol) and cyclopentene (180 *µ*L, 2.0 mmol, 4 equiv) were used. The reaction completed in 9 h at 80°C. The product was isolated by flash chromatography with pentane/Et₂O (10:0 to 10:1) as a colorless liquid. Yield: 82 mg, 93%. Olefinic selectivity in the crude product: 32:1.
[*α*]²³_{D} = -177.8° (*c* = 0.94, CHCl₃).
GC-MS (EI): Calcd for C₁₂H₁₄O M⁺: 174.1. Found: 174.1.
Ee: 93%. Daicel Chiralcel OJ-H, 98:2 hexanes/isopropanol, flow rate =0.5 mL/min.
¹H NMR (400 MHz, CDCl₃): *δ* 7.12-7.09 (m, 2H), 6.85-6.81(m, 2H), 5.92-5.89 (m, 1H), 5.76-5.73 (m, 1H), 3.87-3.81 (m, 1H), 3.78 (s, 3H), 2.54-2.43 (m, 1H), 2.43-2.32 (m, 2H), 1.74-1.62 (m, 1H).

### (S)-3-(p-Fluorophenyl)cyclopentene [CAS for racemate: 78135-03-2].

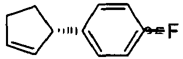

Pd(dba)₂ (14.4 mg, 0.025 mmol), (*R*)-Xyl-SDP(O) (21.6 mg, 0.030 mmol), methanol (1.0 mL), 1-bromo-4-fluorobenzene (87.5 mg, 0.50 mmol), *N-*diisopropylethylamine (255*µ*L, 1.5 mmol), *p*-nitrobenzoic acid (83.6 mg, 0.5 mmol) and cyclopentene (180 *µ*L, 2.0 mmol) were used. The reaction was complete in 26 h at 80°C. The product was isolated by flash chromatography with pentane as colorless oil.
Yield: 68 mg, 82%. Olefinic selectivity in the crude product: 28:1.
Ee: 91%, Daicel Chiralcel OJ-H, 99.8:0.2 hexanes/isopropanol, flow rate = 0.7 mL/min.
¹H NMR (400 MHz, CDCl₃): *δ* 7.16-7.11 (m, 2H), 6.99-6.93 (m, 2H), 6.95-6.92 (m, 1H), 5.76-5.73 (m, 1H), 3.90-3.85 (m, 1H), 2.55-2.45 (m, 1H), 2.44-2.34 (m, 2H), 1.71-1.63 (m, 1H).

### (S)-3-(o-Tolyl)cyclopentene [1461737-06-3].

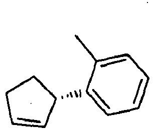

Pd(dba)₂ (14.4 mg, 0.025 mmol), (*R*)-Xyl-SDP(O) (21.6 mg, 0.030 mmol), methanol (1.0 mL), 2-bromotoluene (85.5 mg, 0.50 mmol), *N-*diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction completed in 30 h at 80°C. The product was isolated by flash chromatography with pentane as colorless oil. Yield: 75 mg, 93%. Olefinic selectivity in the crude product: 50:1.
[*α*]²³_{D} = -72.0° (*c* = 1.01, CHCl₃).
GC-MS (EI): Calcd for C₁₂H₁₄ M⁺: 158.1. Found: 158.1.
Ee: 92%. Chiral GC using BPH (isothermal at 90 °C; then 15 deg/min).

Heck reaction of ArCl: Pd(dba)₂ (14.4 mg, 0.025 mmol), (*R*)-Xyl-SDP(O) (21.6 mg, 0.030 mmol), ethylene glycol (1.0 mL), 2-chlorotoluene (63.3 mg, 0.50 mmol), *N*-diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction completed in 120 h at 80°C. The product was isolated by flash chromatography with pentane as colorless oil.
Yield: 60 mg, 74%. Olefinic selectivity in the crude product: 50:1.
Ee: 82%. Chiral GC using BPH (isothermal at 90 °C; then 15 deg/min).
¹H NMR (400 MHz, CDCl₃): *δ* 7.15-7.07 (m, 4H), 5.98-5.96 (m, 1H), 5.79-5.77 (m, 1H), 4.12-4.07 (m, 1H), 2.51-2.38 (m, 3H), 2.37 (s, 3H), 1.65-1.57 (m, 1H).

### (S)-3-(o-Anisyl)cyclopentene [1461737-07-4].

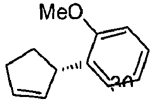

Pd(dba)₂ (14.4 mg, 0.025 mmol), (*R*)-Xyl-SDP(O) (21.6 mg, 0.030 mmol), methanol (1.0 mL), 2-bromoanisole (93.5 mg, 0.50 mmol), *N-*diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction completed in 7 h at 80°C. The product was isolated by flash chromatography with pentane/Et₂O (10:1) as colorless oil. Yield: 75 mg, 85%. Olefinic selectivity in the crude product: 39:1.
[*α*]²³_{D} = -68.5° (*c* = 1.00, CHCl₃).
GC-MS (EI): Calcd for C₁₂H₁₄O M⁺: 174.1. Found: 174.1.
ee: 96%. Chiral GC using BPH (isothermal at 90°C for 100 min; then 15 deg/min).
¹H NMR (400 MHz, CDCl₃): *δ* 7.19-7.15 (m, 1H), 7.13-7.10 (m, 1H), 6.89 (t, *J* = 7.4 Hz, 1H), 6.86 (d, *J* =8.2 Hz, 1H), 5.94-5.92 (m, 1H), 5.77-5.76 (m, 1H), 4.27-4.27 (m, 1H), 3.83 (s, 3H), 2.48-2.33 (m, 3H), 1.67-1.57 (m, 1H).

### (S)-3-(3-Benzothienyl)cyclopentene.

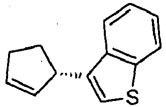

Pd(dba)₂ (14.4 mg, 0.025 mmol), (*R*)-Xyl-SDP(O) (21.6 mg, 0.030 mmol), methanol (3.0 mL), 3-bromobenzothiophene (106.5 mg, 0.50 mmol), *N-*diisopropylethylamine (255 *µ*L, 1.5 mmol), *p*-nitrobenzoic acid (167.1 mg, 1.0 mmol) and cyclopentene (180 *µ*L, 2.0 mmol) were used. The reaction completed in 27 h at 80°C. The product was isolated by flash chromatography with pentane as colorless oil. Yield: 87 mg, 86%. Olefinic selectivity in the crude product: 50:1. When 1.0 equiv of p-nitrobenzoic acid and 1.0 mL of MeOH were used, only 84% ee was obtained.
GCMS (EI): Calcd for C₁₃H₁₂S M⁺: 200.1. Found: 200.0.
Ee: 96%, Daicel Chiralcel OJ-H, 90:10 hexanes/isopropanol, flow rate = 1.0 mL/min.
¹H NMR (400 MHz, CDCl₃): *δ* 7.85-7.80 (m, 2H), 7.38-7.30 (m, 2H), 7.03 (s, 1H), 6.01-5.98 (m, 1H), 5.94-5.91 (m, 1H), 4.27-4.22 (m, 1H), 2.54-2.41 (m, 3H), 1.91-1.80 (m, 1H).
¹³C NMR (CDCl₃, 100 MHz): *δ* 141.0, 140.7, 138.7, 132.7, 132.4, 124.2, 123.8, 122.9, 122.1, 120.2, 44.8, 32.3, 31.3.

### (S)-3-(2-Methyl-5-benzothiazolyl)cyclopentene [1461737-12-1].

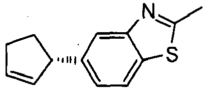

Pd(dba)₂ (14.4 mg, 0.025 mmol), (*R*)-Xyl-SDP(O) (21.6 mg, 0.030 mmol), methanol (3.0 mL), 5-Bromo-2-methyl-1,3-benzothiazole (114.1 mg, 0.50 mmol), *N*-diisopropylethylamine (255 *µ*L, 1.5 mmol), *p-*nitrobenzoic acid (83.6 mg, 0.5 mmol) and cyclopentene (180 *µ*L, 2.0 mmol) were used. The reaction was complete in 40 h at 80°C. The product was isolated by flash chromatography (silica gel, 10:1 hexane/diethyl ether) as colorless oil.
Yield: 87 mg, 80%. Olefinic selectivity in the crude product: 50:1. When 1.0 equiv of *p-*nitrobenzoic acid and 1.0 mL of MeOH were used, only 50% ee was obtained.
GCMS (EI): Calcd for C₁₃H₁₃NS M⁺: 215.1. Found: 215.0.
[*α*]²³_{D} = -151.5° (*c* = 0.82, CHCl₃).
Ee: 88%. Daicel Chiralcel IC, 95:5 hexanes/isopropanol, flow rate = 0.5 mL/min.
¹H NMR (400 MHz, CDCl₃): *δ* 7.77 (d, *J* = 1.6 Hz, 1H), 7.71 (d, *J* = 8.2 Hz, 1H), 7.20 (dd, *J* = 8.2, 1.6 Hz, 1H), 5.99-5.96 (m, 1H), 5.83-5.80 (m, 1H), 4.05-4.00 (m, 1H), 2.81 (s, 3H), 2.59-2.37 (m, 3H), 1.81-1.72 (m, 1H).

### (S)-3-(4-Chrom-3-enyl)cyclopentene

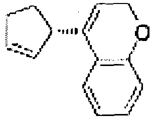

Pd(dba)₂ (28.8 mg, 0.050 mmol), (*R*)-Xyl-SDP(O) (43.2 mg, 0.060 mmol), methanol (1.0 mL), 4-bromo-3-chromene²³ (105.5 mg, 0.50 mmol), *N-*diisopropylethylamine (255 *µ*L, 1.5 mmol), *p*-nitrobenzoic acid (83.6 mg, 0.5 mmol) and cyclopentene (180 *µ*L, 2.0 mmol) were used. The reaction stopped in 26 h at 80 °C. The product was isolated by flash chromatography (pentane) as colorless oil. Yield: 73 mg, 75%. Olefinic selectivity in the crude product: >50:1.
GCMS (EI): Calcd for C₁₄H₁₄O M⁺: 198.1. Found: 198.1.
ee: 90%. Chiral GC using BPH (isothermal at 150°C for 90 min; then 10 deg/min).
¹H NMR (400 MHz, CDCl₃): *δ* 7.26 (dd, *J* = 7.7, 1.6 Hz, 1H), 7,12 (ψtd, *J* = 7.9, 1.6 Hz, 1H), 6.91 (ψtd, *J* = 7.5, 1.2 Hz, 1H), 6.82 (dd, *J* = 8.0, 1.2 Hz, 1H), 5.97-5.94 (m, 1H), 5.76-5.71 (m, 1H), 5.51 (td, *J* = 3.7, 0.9 H, 1H), 4.76-4.73 (m, 2H), 3.81-3.79 (m, 1H), 2.40-2.28 (m, 3H), 1.72-1.66 (m, 1H).
¹³C NMR (100 MHz, CDCl₃): 154.6, 137.7, 132.8, 132.0, 128.7, 123.7, 123.6, 121.1, 116.0, 115.9, 65.6, 456, 32.0, 30.6.

### (S)-3-(m-Anisyl)cyclopentene [1461737-14-3].

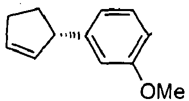

Pd(dba)₂ (28.8 mg, 0.050 mmol), (*R*)-Xyl-SDP(O) (43.2 mg, 0.060 mmol), methanol (2.0 mL), 3-bromoanisole (187 mg, 1.0 mmol), *N-*diisopropylethylamine (510 *µ*L, 3.0 mmol), *p*-nitrobenzoic acid (167.1 mg, 1.0 mmol) and cyclopentene (360 *µ*L, 4.0 mmol, 4 equiv) were used. The reaction completed in 12 h at 80°C. The product was isolated by flash chromatography with pentane/Et₂O (10:1) as colorless oil. Yield: 163 mg, 92%. Olefinic selectivity in the crude product: >50:1.
GCMS (EI): Calcd for C₁₂H₁₄O M⁺: 174.1. Found: 174.1.
Ee: 87%. Daicel Chiralcel OJ-H, 99.8:0.2 hexanes/isopropanol, flow rate = 0.5 mL/min.
¹H NMR (500 MHz, CDCl₃): *δ* 7.22-7.19 (m, 1H), 6.80-6.78 (m, 1H), 6.74-6.72 (m, 2H), 5.95-5.92 (m, 1H), 5.78-5.76 (m, 1H), 3.89-3.84 (m, 1H), 3.79 (s, 3H), 2.54-2.44(m, 1H), 2.43-2.35 (m, 2H), 1.76-1.68 (m, 1H).

### D. General procedure for asymmetric Heck reaction of N-Boc-2,3-dihydro-1H-pyrrole:

In an argon-filled glove box, Pd(dba)₂ (14.4 mg, 0.025 mmol) and (*R*)-Xyl-SDP(O) (21.6 mg, 0.030 mmol) were stirred in degassed ethylene glycol (1.0 mL) for 10-20 min in a 10-mL reaction tube, followed by successive addition of aryl bromide (0.50 mmol), *N-*diisopropylethylamine (255 *µ*L, 1.5 mmol, 3 equiv), *p*-nitrobenzoic acid (83.6 mg, 0.5 mmol, 1 equiv) and *N*-Boc-2,3-dihydro-1*H*-pyrrole (169 mg, 1.0 mmol). The mixture was vigorously stirred in a preheated oil bath at 70°C, until the aryl bromide was fully consumed (monitored by GC). The reaction mixture was cooled to room temperature and subjected to flash chromatography (pentane/Et₂O) to give the purified product. The olefinic selectivity of the product in the crude mixture was determined by GC and GCMS.

### (S)-N-Boc-2-phenyl-2,5-dihydropyrrole [316813-68-0].

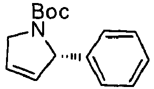

Pd(dba)₂ (14.4 mg, 0.025 mmol), (*R*)-Xyl-SDP(O) (21.6 mg, 0.030 mmol), ethylene glycol (1.0 mL), bromobenzene (78.5 mg, 0.50 mmol), *N-*diisopropylethylamine (255 *µ*L, 1.5 mmol), p-nitrobenzoic acid (83.6 mg, 0.5 mmol) and N-Boc-2,3-dihydro-1H-pyrrole (169 mg, 1.0 mmol) were used. The reaction completed in 36 h at 70°C. The product was isolated by flash chromatography (silica gel, pentane/Et₂O) as pale yellow solid.
Yield: 103 mg, 84%. Olefinic selectivity in the crude product: 9:1.
GCMS (EI): Calcd for C₁₅H₁₉NO₂ M⁺: 245.1. Found: 245.1.
[*α*]²³_{D} = -141.2° (c = 0.85, CHCl₃).
Ee: 91%. Daicel Chiralcel IC-H, 95:5 *n*-hexane/isopropanol, flow rate = 0.5 mL/min.
¹H NMR of two rotamers in 2.3:1 ratio (400 MHz, CDCl₃): *δ* 7.32-7.18 (m, 5H), 5.91-5.84 (m, 1H), 5.77-5.71 (m, 1H), 5.53-5.52 (br s, 0.3H), 5.37 (br s, 0.7H), 4.35-4.27 (m, 2H), 1.43 (br s, 2.6H), 1.21 (br s, 6.4H).

Heck reaction of Phl: Pd(dba)₂ (2.9 mg, 0.005 mmol), (*R*)-Xyl-SDP(O) (4.3 mg, 0.006 mmol), ethylene glycol (0.2 mL), iodobenzene (20.4 mg, 0.10 mmol), *N*-diisopropylethylamine (51 *µ*L, 0.3 mmol), *p*-nitrobenzoic acid (16.7 mg, 0.1 mmol), 10 *µ*L *n*-tetradecane and *N*-Boc-2,3-dihydro-1*H*-pyrrole (34 mg, 0.2 mmol) were used. The reaction was heated in 70 °C for 40 h. The reaction mixture was cooled to room temperature, diluted by EtOH and passed through a short plug of silical gel with diethyl ether washing. The filtrate was used in GC analysis to determine the conversion of Phl and GC yield and isomeric selectivity or s selectivity of the desired Heck product. Conversion of Phl: 100%. GC yield: 67%. Olefinic selectivity in the crude product: 21:1. Ee of major isomer: 66%.

### (S)-N-Boc-2-(p-tolyl)-2,5-dihydropyrrole.

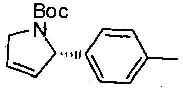

Pd(dba)₂ (14.4 mg, 0.025 mmol), (*R*)-Xyl-SDP(O) (21.6 mg, 0.030 mmol), ethylene glycol (1.0 mL), 4-bromotoluene (85.5 mg, 0.50 mmol), N-diisopropylethylamine (255 *µ*L, 1.5 mmol), *p*-nitrobenzoic acid (83.6 mg, 0.5 mmol) and *N*-Boc-2,3-dihydro-1*H*-pyrrole (169 mg, 1.0 mmol) were used. The reaction completed in 16 h at 70°C. The product was isolated by flash chromatography (silica gel, pentane/Et₂O) as colorless oil. Yield: 119 mg, 92%. Olefinic selectivity in the crude product: 32:1.
GCMS (EI): Calcd for C₁₆H₂₁NO₂ M⁺: 259.2. Found: 259.1.
[*α*]²³_{D} = -224.9° (*c* = 1.00, CHCl₃).
Ee: 94%. Daicel Chiralcel OJ-H, 98:2 *n*-hexane/isopropanol, flow rate = 0.5 mL/min.

Heck reaction of ArCl: Pd(dba)₂ (14.4 mg, 0.025 mmol), (*R*)-Xyl-SDP(O) (21.6 mg, 0.030 mmol), ethylene glycol (1.0 mL), 4-chlorotoluene (63.0 mg, 0.50 mmol), *N*-diisopropylethylamine (255 *µ*L, 1.5 mmol), *p*-nitrobenzoic acid (83.6 mg, 0.5 mmol) and *N*-Boc-2,3-dihydro-1*H*-pyrrole (169 mg, 1.0 mmol) were used. The reaction completed in 60 h at 80°C. The product was isolated by flash chromatography (silica gel, pentane/Et₂O) as colorless oil.
Yield: 122 mg, 94%. Olefinic selectivity in the crude product: 48:1.
Ee: 94%. Daicel Chiralcel OJ-H, 99:1 *n*-hexane/isopropanol, flow rate = 0.5 mL/min.
¹H NMR of two rotamers in 2.2:1 ratio (400 MHz, CDCl₃): *δ* 7.16-7.06 (m, 4H), 5.88-5.81 (m, 1H), 5.74-5.68 (m, 1H), 5.49 (br s, 0.31H), 5.35-5.34 (br s, 0.68H), 4.33-4.25 (m, 2H), 2.32 (br s, 2.1H), 2.31 (br s, 0.9H), 1.43 (br s, 2.9H), 1.23 (br s, 6.2H).
¹³C NMR of two rotamers in 2.2:1 ratio (100 MHz, CDCl₃): *δ* 154.2, 153.9, 139.4, 138.8, 136.8, 131.4, 131.3, 129.2, 128.8, 126.6, 126.5, 124.4, 79.5, 79.4, 67.8, 67.6, 54.0, 53.6, 28.5, 28.2, 21.1.

### (S)-N-Boc-2-(p-anisyl)-2,5-dihydropyrrole.

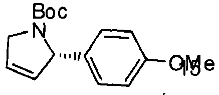

Pd(dba)₂ (14.4 mg, 0.025 mmol), (*R*)-Xyl-SDP(O) (21.6 mg, 0.030 mmol), ethylene glycol (1.0 mL), 4-bromoanisole (93.5mg, 0.50 mmol), *N*-diisopropylethylamine (255 *µ*L, 1.5 mmol), *p*-nitrobenzoic acid (83.6 mg, 0.5 mmol) and *N*-Boc-2,3-dihydro-1*H*-pyrrole (169 mg, 1.0 mmol) were used. The reaction completed in 16 h at 70°C. The product was isolated by flash chromatography (silica gel, pentane/Et₂O) as colorless oil.
Yield: 113 mg, 82%. Olefinic selectivity in the crude product: 11:1.
GCMS (EI): Calcd for C₁₆H₂₁NO₃ M⁺: 275.2. Found: 275.1.
[*α*]^{*2*3}_{D} = -203.5.0° (*c* = 1.56, CHCl₃).
Ee: 97%. Daicel Chiralcel OJ-H, 98:2 *n*-hexane/isopropanol, flow rate = 0.5 mL/min.
¹H NMR of two rotamers in 2:1 ratio (400 MHz, CDCl₃): *δ* 7.20-7.10 (m, 2H), 6.85-6.82 (m, 2H), 5.89-5.82 (m, 1H), 5.74-5.68 (m, 1H), 5.48 (br s, 0.32H), 5.34-5.33 (br s, 0.68H), 4.32-4.24 (m, 2H), 3.79 (br s, 2H), 3.77 (br s, 1H), 1.43 (br s, 2.8H), 1.24 (br s, 6.2H).
¹³C NMR of two rotamers in 2:1 ratio (100 MHz, CDCl₃): *δ* 158.8, 154.2, 153.9, 134.6, 133.9, 131.3, 128.0, 127.9, 124.5, 124.4, 113.8, 113.5, 79.5, 79.4, 67.5, 67 2, 55.2, 53.9, 53.5, 28.5, 28.2.

### (S)-N-Boc-2-(o-anisyl)-2,5-dihydropyrrole.

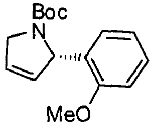

Pd(dba)₂ (14.4 mg, 0.025 mmol), (*R*)-Xyl-SDP(O) (21.6 mg, 0.030 mmol), ethylene glycol (1.0 mL), 2-bromoanisole (93.5 mg, 0.50 mmol), *N-*diisopropylethylamine (255 *µ*L, 1.5 mmol), *p*-nitrobenzoic acid (83.6 mg, 0.5 mmol) and *N*-Boc-2,3-dihydro-1*H*-pyrrole (169 mg, 1.0 mmol) were used. The reaction completed in 16 h at 70 °C. The product was isolated by flash chromatography (silica gel, pentane/Et₂O) as colorless oil.
Yield: 124 mg, 90%. Olefinic selectivity in the crude product: 45:1.
GCMS (EI): Calcd for C₁₆H₂₁NO₃ M⁺: 275.2. Found: 275.1.
Ee: 96%. Daicel Chiralcel OJ-H, 98:2 *n*-hexane/isopropanol, flow rate = 0.5 mL/min.
¹H NMR of two rotamers in 2.3:1 ratio (500 MHz, CDCl₃): *δ* 7.21-7.16 (m, 1H), 7.09-7.08 (m, 1H), 6.90 (ψt, *J* = 7.5 Hz, 1H), 6.84 (d, *J* = 8.2 Hz, 1H), 5.91-5.79 (m, 2H), 5.77-5.70 (m, 1H), 4.34-4.25 (m, 2H), 3.83 (s, 3H), 1.47 (br s, 2.8H), 1.19 (brs, 6.4H).
¹³C NMR of two rotamers in 2.3:1 ratio (100 MHz, CDCl₃): *δ* 156.2, 154.2, 153.9, 130.0 130.8, 130.6, 130.0, 127.9, 126.7, 125.5, 124.0, 123.6, 120.8, 120.6, 110.6, 110.1, 79.4, 79.1, 62.9, 62.3, 55.4, 55.3, 54.0, 53.6, 28.5, 28.1.

### (S)-N-Boc-N'-methyl-2-(2-pyrrolyl)-2,5-dihydropyrrole.

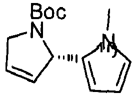

Pd(dba)₂ (14.4 mg, 0.025 mmol), (*R*)-Xyl-SDP(O) (21.6 mg, 0.03 mmol), ethylene glycol (1.0 mL), *N*-methyl-2-bromopyrrole²⁴ (93.5 mg, 0.50 mmol), N-diisopropylethylamine (255 *µ*L, 1.5 mmol), *p*-nitrobenzoic acid (83.6 mg, 0.5 mmol) and *N*-Boc-2,3-dihydro-1*H*-pyrrole (169 mg, 1.0 mmol) were used. The reaction completed in 12 h at 50°C. The product was isolated by flash chromatography (silica gel, pentane/Et₂O) as colorless oil.
Yield: 114 mg, 92%. Olefinic selectivity in the crude product: >50:1.
GCMS (EI): Calcd for C₁₄H₂₀N₂O₂ M⁺: 248.2. Found: 248.0.
[*α*]²³D = -194.7° (*c* = 0.75, CHCl₃).
Ee: 96%. Daicel Chiralcel OJ-H, 95:5 n-hexane/isopropanol, flow rate = 0.5 mL/min.
¹H NMR of two rotamers in 1.6:1 ratio (400 MHz, CDCl₃): *δ* 6.51-6.48 (m, 1H), 6.03-5.97 (m, 1H), 5.86 (m, 1H), 5.74-5.69 (m, 1H), 5.58-5.53 (m, 1H), 4.28-4.13 (m, 2H), 3.72(br s, 1.3H), 3.53 (br s, 1.9H), 1.43 (br s, 3.8H), 1.27 (br s, 6.1H).
¹³C NMR of two rotamers in 1.6:1 ratio (125 MHz, CDCl₃): *δ* 154.3, 154.1, 131.8, 131.6, 129.6, 124.9, 124.6, 122.6, 122.4, 108.1, 107.4, 106.7, 106.6, 79.7, 61.1, 59.7, 53.2, 34.1, 33.8, 28.9, 28.5, 28.2, 25.8, 25.4.

### (S)-N-Boc-2-(p-fluorophenyl)-2,5-dihydropyrrole.

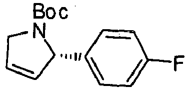

Pd(dba)₂ (28.8 mg, 0.05 mmol), (*R*)-Xyl-SDP(O) (43.2 mg, 0.06 mmol), ethylene glycol (1.0 mL), 1-bromo-4-fluorobenzene (87.5 mg, 0.50 mmol), *N*-diisopropylethylamine (255 *µ*L, 1.5 mmol), *p*-nitrobenzoic acid (83.6 mg, 0.5 mmol) and *N*-Boc-2,3-dihydro-1*H*-pyrrole (169 mg, 1.0 mmol) were used. The reaction completed in 22 h at 70 °C. The product was isolated by flash chromatography (silica gel, pentane/Et₂O) as colorless oil. Yield: 92 mg, 70%. Olefinic selectivity in the crude product: 8:1.
GCMS (EI): Calcd for C₁₅H₁₈FNO₂ M⁺: 263.1. Found: 263.1.
Ee: 93%. Daicel Chiralcel OJ-H, 98:2 *n*-hexane/isopropanol, flow rate = 0.5 mL/min.
¹H NMR of two rotamers in 2.1:1 ratio (400 MHz, CDCl₃): *δ* 7.27-7.15 (m, 2H), 7.01-6.96 (m, 2H), 5.92-5.85 (m, 1H), 5.74-5.68 (m 1H), 5.51-5.50 (m, 0.33H), 5.37-5.36 (m, 0.67H), 4.33-4.22 (m, 2H), 1.43 (br s, 2.9H), 1.27 (br s, 6.1H).
¹³C NMR of two rotamers in 2.1:1 ratio (125 MHz, CDCl₃): *δ* 163.2, 160.8, 138.3, 137.5, 131.0, 128.4, 128.3, 124.9, 115.3, 115.1, 115.1, 114.9, 79.7, 67.4, 67.1, 54.0, 53.6, 28.5, 28.2. No *J*_{C-F} coupling was assigned.
¹⁹F NMR of two rotamers in 2.1:1 ratio (376 MHz, CDCl₃): *δ* -115.6, -115.8.

### (S)-N-Boc-2-(p-tolyl)-2,5-dihydropyrrole.

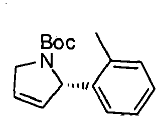

Pd(dba)₂ (14.4 mg, 0.025 mmol), (R)-Xyl-SDP(O) (21.6 mg, 0.030 mmol), ethylene glycol (1.0 mL), 2-chlorotoluene (63.0 mg, 0.50 mmol), *N-*diisopropylethylamine (255 *µ*L, 1.5 mmol), p-nitrobenzoic acid (83.6 mg, 0.5 mmol) and *N*-Boc-2,3-dihydro-1*H*-pyrrole (169 mg, 1.0 mmol) were used. The reaction completed in 42 h at 80 °C. The product was isolated by flash chromatography (silica gel, pentane/Et₂O) as colorless oil.
Yield: 121 mg, 93%. Olefinic selectivity in the crude product: 48:1.
Ee: 90%. Daicel Chiralcel OJ-H, 99:1 *n*-hexane/isopropanol, flow rate = 0.5 mL/min.
¹H NMR of two rotamers in 2.8:1 ratio (500 MHz, CDCl₃): *δ* 7.16-7.08 (m, 4H), 5.86-5.65 (m, 3H), 4.42-4.28 (m, 2H), 2.43 (br s, 0.8H), 2.37 (br s, 2.2H), 1.45 (br s, 2.4H), 1.16 (br s, 6.8H).
¹³C NMR of two rotamers in 2.8:1 ratio (125 MHz, CDCl₃): *δ* 154.0, 140.9, 139.9, 134.5, 134.3, 130.5, 130.4, 130.4, 130.0, 126.9, 126.8, 126.4, 126.3, 126.1, 125.4, 124.4, 124.3, 79.4, 65.0, 64.9, 54.2, 53.8, 28.5, 28.1, 19.1.

**(*S*)-3-(o-Anisyl)-1-cycloheptene.** Pd(dba)₂ (14.4 mg, 0.025 mmol), (*R*)-Xyl-SDP(O) (21.6 mg, 0.030 mmol), methanol (1.0 mL), 2-bromoanisole (93.5 mg, 0.50 mmol), *N-*diisopropylethylamine (255 *µ*L, 1.5 mmol), *p*-nitrobenzoic acid (83.6 mg, 0.5 mmol) and cycloheptene (233 *µ*L, 2.0 mmol) were used. The reaction completed in 18 h at 80°C. The product was isolated by flash chromatography with pentane as colorless oil.
Yield: 91 mg, 90%. Olefinic selectivity in the crude product: > 50:1.
GCMS (EI): Calcd for C₁₄H₁₈O M⁺: 202.1. Found: 202.0.
Ee: 88%. Daicel Chiralcel OD-H, 100% n-hexane flow rate = 0.5 mL/min.
¹H NMR (400 MHz, CDCl₃): *δ* 7.23-7.21 (m, 1H), 7.19-7.14 (m, 1H), 6.91 (ψt, *J* = 7.4 Hz, 1H), 6.84 (d, *J* = 8.1 Hz, 1H), 5.87-5.80 (m, 1H), 5.72-5.69 (m, 1H), 3.97 (br s, 1H), 3.81 (s, 3H), 2.25-2.22 (m, 2H), 1.93-1.89 (m, 1H), 1.80-1.62 (m, 4H), 1.51-1.42 (m, 1H).
¹³C NMR (100 MHz, CDCl₃): *δ* 156.4, 137.2, 136.0, 131.2, 128.1, 126.8, 120.6, 110.6, 55.5, 39.7, 34.8, 30.3, 28.8, 27.3.

### (S)-3-(o-Anisyl)-1-cyclooctene.

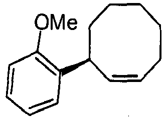

Pd(dba)₂ (14.4 mg, 0.025 mmol), (*R*)-Xyl-SDP(O) (21.6 mg, 0.030 mmol), methanol (1.0 mL), 2-bromoanisole(93.5 mg, 0.50 mmol), *N-*diisopropylethylamine (255 *µ*L, 1.5 mmol), *p*-nitrobenzoic acid (83.6 mg, 0.5 mmol) and cyclooctene (260 *µ*L, 2.0 mmol) were used. The reaction completed in 18 h at 80 °C. The product was isolated by flash chromatography with pentane as colorless oil.
Yield: 92 mg, 85%. Olefinic selectivity in the crude product: 11:1.
GCMS (EI): Calcd for C₁₅H₂₀O M⁺: 216.2. Found: 216.0.
Ee: 84%. Daicel Chiralcel OJ-H, 99.5:0.5 n-hexane/isopropanol, flow rate = 0.5 mL/min.
¹H NMR (400 MHz, CDCl₃): *δ* 7.28 (dd, *J* = 7.5, 1.5 Hz, 1H), 7.18-7.14 (m, 1H), 6.93 (dt, *J* = 7.4, 1.6 Hz, 1H), 6.85 (d, *J* = 8.2 Hz, 1H), 5.71-5.59 (m, 2H), 4.24-4.18 (m, 1H), 3.81 (s, 3H), 2.50-2.41 (m, 1H), 2.15-2.08 (m, 1H), 1.79-1.69 (m, 4H), 1.67-1.60 (m, 2H), 1.58-1.50 (m, 1H), 1.44-1.35 (m, 1H).
¹³C NMR (100 MHz, CDCl₃): *δ* 156.9, 134.9, 133.8, 128.9, 127.5, 126.7, 120.7, 110.8, 55.6, 36.6, 35.2, 29.8, 26.7, 26.4, 26.3.

### E. Stoichiometric olefin insertion

Synthesis of [(*R*)-Xyl-SDP(O)](*p*-F-Ph)(Br)Pd(II). In an argon-filled glove box, Pd(dba)₂ (127 mg, 0.22 mmol) and (*R*)-Xyl-SDP(O) (158 mg, 0.22 mmol) were stirred in degassed toluene (4 mL) in a 10-mL vial at RT for 6 h, followed by the addition of *p*-fluorophenyl bromide (154 mg, 0.88 mmol, 4 equiv). The vial mixture was stirred rigorously at RT for 3 days. ³¹P NMR spectroscopy of the crude mixture showed >90% purity of the expected complex. The resulting mixture was passed through Celite to remove some Pd black. The filtrate was concentrated to dryness under vacuum and the residue was dissolved in a small amount trace amount of CH₂Cl₂. The solvent was covered by hexane and cooled to -30°C. The precipitate was filtrated and washed with hexane. A large amount of yellow solid precipitated from the filtrate on standing. The solid was filtrated and recrystallized from MeOH to give the pure complex as yellow powder (75 mg, 34%). ESI-MS: calcd for [M-Br]⁺ C₅₅H₅₄FOP₂Pd: 917.27. Found: 917.56.
¹H NMR (500 MHz, CDCl₃): *δ* 7.68-7.52 (m, 4H), 7.41-7.34 (m, 3H), 7.10-7.03 (m, 3H), 6.98 (t, *J* = 7.5 Hz) (m, 5H), 6.91 (s, 1H), 6.78 (br s, 3H), 6.42-6.25 (m, 3H), 6.51-6.47 (m 2H), 5.85 (d, *J* = 10.9 Hz, 2H), 3.01-2.94 (m, 1H), 2.74 (m, 1H), 2.63-2.56 (m, 1H), 2.33-2.32 (m, 12H), 2.17 (s, 6H), 2.13-2.03 (m, 4H), 1.95 (s, 6H), 1.60-1.54 (m, 1H).
³¹P{¹H} NMR (202MHz, CDCl₃): *δ* 39.3 (s), 20.9 (s).
¹⁹F{¹H} NMR (376 MHz, CH₂Cl₂): *δ* -125.5 (s).

### Stoichiometric insertion of cyclopentene into [(R)-Xyl-SDP(O)](p-F-phenyl)(Br)Pd.

Typical procedure: In an argon-filled glove box, arylpalladium bromide complex (20 mg, 0.020 mmol) was dissolved in degassed MeOH (0.50 mL) in a 4-mL vial. GC standard triglyme (5 *µ*L), *i*Pr₂NEt (21 *µ*L, 0.12 mmol, 6 equiv), *p*-nitrobenzoic acid (6.7 mg, 0.04 mmol, 2 equiv) and cyclopentene (7 mg, 0.10 mmol, 5 equiv) were added. The mixture was stirred at 50°C with aliquots taken at intervals for GC analysis. GC yield of the Heck product was determined to be 53% (5 h) and no more Heck product was formed afterwards. The olefinic selectivity of the Heck product in the crude mixture was determined to be 28:1 by GC. The ee was determined to be 94% (chiral HPLC using Daicel Chiralcel OJ-H and 0.2% IPA in hexanes). The olefin insertion was very slow at room temperature. If *p*-nitrobenzoic acid additive was omitted, the stoichiometric reaction (50°C, 5 h) gave 40% yield, 13:1 olefinic selectivity and 67 % ee. When the stoichiometric reaction was conducted in dioxane, no Heck product was detected after 5 h at 50°C.

### References:

1. Grushin, V. V. Organometallics 2001, 20, 3950.
2. Morris, D. J.; Docherty, G.; Woodward, G.; Wills, M. Tetrahedron Lett. 2007, 48, 949.
3. Faller, J. W.; Grimmond, B. J.; D'Alliessi, D. G. J. Am. Chem. Soc. 2001, 123, 2525.
4. Xie, J.-H.; Wang, L.-X.; Fu, Y.; Zhu, S.-F.; Fan, B.-M.; Duan, H.-F.; Zhou, Q.-L. J. Am. Chem. Soc. 2003, 125, 4404.
5. Fukuda, Y.; Kondo, K.; Aoyama, T. Chem. Pharm. Bull. 2007, 55, 955.
6. Klemm, L. H.; Ho, B. T.; Lind, C. D.; MacGowan, B. I.; Mak, E. Y. K. J. Org. Chem. 1959, 24, 949.
7. Leermann, T.; Leroux, F. e. e. R.; Colobert, F. Org. Lett. 2011, 13, 4479.
8. Busacca, C. A.; Lorenz, J. C.; Grinberg, N.; Haddad, N.; Hrapchak, M.; Latli, B.; Lee, H.; Sabila, P.; Saha, A.; Sarvestani, M.; Shen, S.; Varsolona, R.; Wei, X.; Senanayake, C. H. Org. Lett. 2005, 7, 4277.
9. Han, X.; Zhang, Y.; Wu, J. J. Am. Chem. Soc. 2010, 132, 4104.
10. a) Hopps, H. B. Diss. Abstr. 1962, 23. b) Chung, K.-G.; Miyake, Y.; Uemura, S. J. Chem. Soc., Perkin Trans. 1 2000, 2725. c) de la Rosa, J. C.; Diaz, N.; Carretero, J. C. Tetrahedron Lett. 2000, 41, 4107.
11. Larock, R. C.; Baker, B. E. Tetrahedron Lett. 1988, 29, 905.
12. Kim, C. J.; Brown, H. C. J. Am. Chem. Soc. 1972, 94, 5051.
13. Kikukawa, K.; Nagira, K.; Wada, F.; Matsuda, T. Tetrahedron 1981, 37, 31.
14. Büchner, I. K.; Metz, P. Tetrahedron Lett. 2001, 42, 5381.
15. Kaukoranta, P.; Källström, K.; Andersson, P. G. Adv. Synth. Catal. 2007, 349, 2595.
16. Ozawa, F.; Kubo, A.; Hayashi, T. J. Am. Chem. Soc. 1991, 113, 1417.
17. Zhang, X. U.S. Patent 6521769, 2003.
18. Wang, Y.; Zheng, K.; Hong, R. J. Am. Chem. Soc. 2012, 134, 4096.
19. Duffy, M. G.; Grayson, D. H. J. Chem. Soc., Perkin Trans. 1 2002, 1555.
20. Dai, W.-M.; Yeung, K. K. Y.; Wang, Y. Tetrahedron 2004, 60, 4425.
21. Arnold, W.; Daly, J. J.; Imhof, R.; Kyburz, E. Tetrahedron Lett. 1983, 24, 343.
22. Wu, S.; Lee, S.; Beak, P. J. Am. Chem. Soc. 1996, 118, 715.
23. Spaggiari, A.; Vaccari, D.; Davoli, P.; Torre, G.; Prati, F. J. Org. Chem. 2007, 72, 2216.
24. Dvornikova, E.; KamieŇska-Trela, K. Synlett 2002, 1152.
25. XIE, J.-H. et al. Advanced Synthesis & Catalysis (2004) Vol.346 No.6 pages 625 to 632.

## Claims

1. Catalyst complex comprising Pd and at least one ligand of Formula (I) 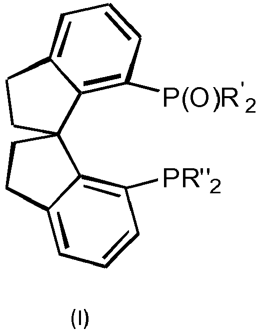 wherein,
R' and R" are independently selected from the group consisting of substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

2. Catalyst complex according to claim 1, wherein
(a) the ligand of Formula (I) is a ligand of Formula (II) or Formula (III) 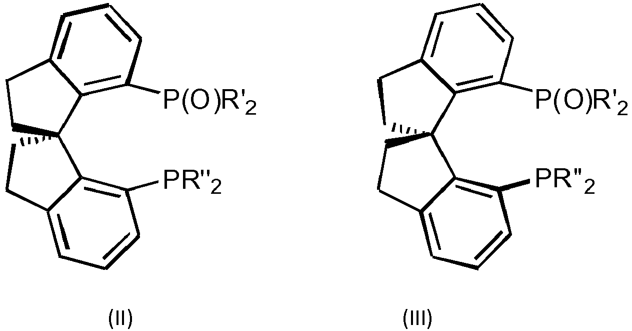 wherein,
(i) R' and R" are independently selected from the group consisting of substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms; or
(ii) R' and R" are independently selected from the group consisting of cyclohexanyl (Cy), phenyl (Ph), 1,3-dimethylbenzene (*meta*-Xylyl), and *para*-FC₆H₄; or
(b) the ligand is independently selected from the group consisting of 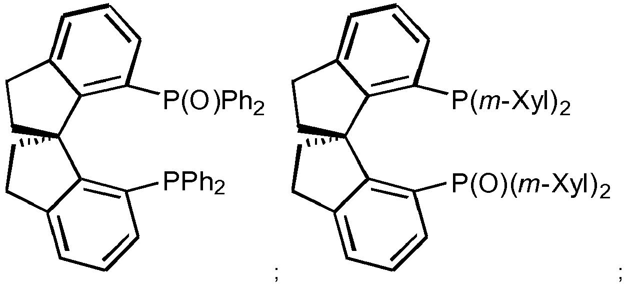 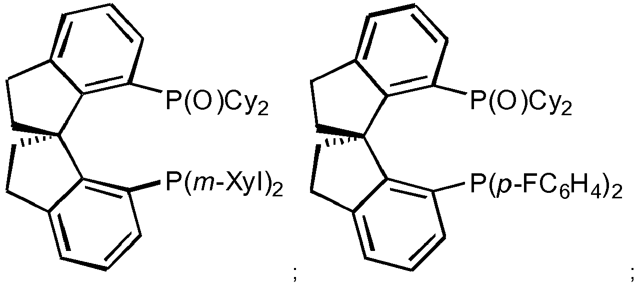 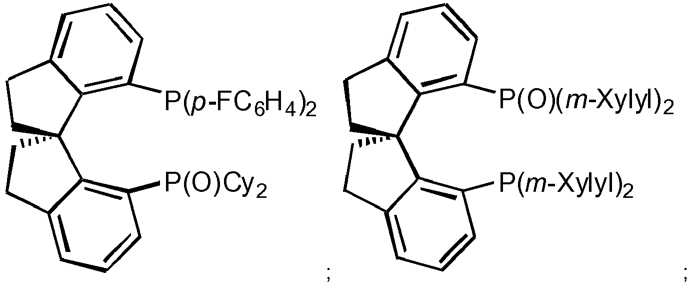 and mixtures thereof.

3. Catalyst complex according to any of claims 1 to 2, wherein
(a) the Pd is a Pd(0) and/or a Pd(+II) specie; or
(b) the Pd is a Pd(0) specie; or
(c) the Pd(0) specie is generated *in situ;* or
(d) the Pd is generated from a Pd precursor selected from the group consisting of Pd(dba)₂, Pd₂(dba)₃, Pd(PPh₃)₄, Pd(OAc)₂, PdCI₂, PdBr₂, Pdl₂, PdCl₂(PPh₃)₂, Pd(OAc)₂, Pd(P*tert-*butyl₃)₂, optionally the Pd precursor is selected from the group consisting of Pd(OAc)₂, Pd(dba)₂, or Pd₂(dba)₃.

4. Method for forming a covalent carbon-carbon single bond in the Pd-catalyzed Heck carbon-carbon coupling reaction, the method comprising:
(i) providing the catalyst complex according to any of claims 1 to 3; and
(ii) reacting at least one electrophilic compound of the Formula (IV) with at least one olefin of the Formula (V)
R¹-LG¹ (IV) 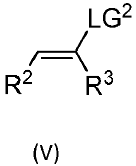
wherein
R¹ is any organic compound;
R² and R³ combine to form together with the carbon atoms to which they are attached a substituted or unsubstituted 5- to 40-membered cycloalkenyl or heterocycloalkenyl; and
LG¹ and LG² are leaving groups; and
in the presence of the catalyst complex under conditions suitable for forming the covalent carbon-carbon single bond.

5. Method according to claim 4, wherein
(a) the organic moiety is independently selected from the group consisting of linear or branched, substituted or unsubstituted C₁-Cₓ alkyl; linear or branched, substituted or unsubstituted alkenyl with 2 to x carbon atoms; linear or branched, substituted or unsubstituted alkynyl with 2 to x carbon atoms; linear or branched, substituted or unsubstituted alkoxy with 1 to x carbon atoms; substituted or unsubstituted cycloalkyl with 3 to x carbon atoms; substituted or unsubstituted cycloalkenyl with 3 to x carbon atoms; substituted or unsubstituted aryl with 6 to x carbon atoms; and substituted or unsubstituted heteroaryl with 3 to x carbon atoms; with x being any integer of 2 or more, preferably up to 50, more preferably up to 30; and/or
(b) (i) R¹ is selected from the group consisting of linear or branched, substituted or unsubstituted alkenyl with 2 to x carbon atoms; substituted or unsubstituted cycloalkenyl with 3 to x carbon atoms; substituted or unsubstituted aryl with 6 to x carbon atoms; and substituted or unsubstituted heteroaryl with 3 to x carbon atoms; with x being any integer of 2 or more, preferably up to 50, more preferably up to 30; or
(b) (ii) R¹ is selected from the group consisting of substituted or unsubstituted, linear or branched alkenyl with 3 to 15 carbon atoms; substituted or unsubstituted benzene; and substituted or unsubstituted naphthalene; and/or
(c) (i) LG¹ is selected from the group consisting of halogen, -OSO₂C₄F₉, -OSO₂CF₃, -OSO₂F, -OTs, and -OMs; or
(c) (ii) LG¹ is selected from the group consisting of Cl, Br, I, and -OSO₂CF₃; or
(c) (iii) LG¹ is -OSO₂CF₃ or Br.

6. Method according to claim 4 or 5, wherein the at least one electrophilic compound of Formula (IV) is selected from the group consisting 1-bromonaphthalene, 2-bromonaphthalene, bromobenzene, 4-bromoanisole, 4-bromotoluene, 1-bromo-4-fluorobenzene, 2-bromoanisole, *N*-methyl-2-bromopyrrole, 3-bromoindole, 5-bromo-2-methyl-1,3-benzothiazole, 3-bromobenzofuran, 3-bromobenzothiophene, 2-bromothiophene, 2-bromothiophene, 4-bromo-3-chromene, 1-bromostyrene and (*E*)-2-bromostyrene, 1-bromocyclohexene, 1-bromocyclopentene, bromoethene, (*E*)-1-bromopropene, 2-bromopropene, iodobenzene, 1-iodonaphthalene, 2-iodonaphthalene, 4-iodoanisole, 4-iodotoluene, 4-chlorotoluene, 2-chlorotoluene, 1-chloronaphthalene, 2-chloronaphthalene, chlorobenzene, 4-chloroanisole, 2-chloroanisole, 3-chloroindole, *N*-methyl-2-chloropyrrole, 5-chloro-1,3-benzothiazole, 3-chlorobenzofuran, 3-chlorobenzothiophene, 2-chlorothiophene, 1-naphthyl triflate, 2-naphthyl triflate, phenyl triflate, *para-tert*-butylphenyl triflate, *para*-(trifluoromethyl)phenyl triflate, *para-*chlorophenyl triflate, and *para*-fluorophenyl triflate, *para*-formylphenyl triflate, *para-*(ethoxycarbonyl)phenyl triflate, *para*-anisyl triflate, *para*-*tert*-butytphenyl triflate, *ortho-*methylphenyl triflate, *para*-chlorophenyl triflate, *para*-benzophenonyl triflate, *ortho*-anisyl triflate, *meta*-anisyl triflate, 1-tosyl-1*H*-indol-5-yl triflate, 2-methylbenzo[*d*]thiazol-5-yl triflate, 2-thienyl and 3-thienyl trilfates and their benzo-derivatives, 2-furanyl and 3-furanyl triflates and their benzo-derivatives, *N*-Boc-2-pyrrolidinyl and *N*-Boc-3-pyrrolidinyl trilfates, cyclohexenyl triflate, 2-methylcyclohexenyl triflate, 1-styryl and (*E*)-2-styryl trilfates.

7. Method according to any one of claims 4 to 6, wherein
(a) R² and R³ combine to form together with the carbon atoms to which they are attached a substituted or unsubstituted 5- to 20-membered cycloalkenyl or heterocycloalkenyl; or
(b) R² and R³ combine to form together with the carbon atoms to which they are attached a substituted or unsubstituted 5- or 6-membered cycloalkenyl or heterocycloalkenyl.

8. Method according to any one of claims 4 to 7, wherein
(a) LG² is hydrogen; and/or
(b) the at least one olefin of Formula (V) is selected from the group consisting cyclopentene; cyclohexene; cyloheptene; cyclooctene; 2,3-dihydrofuran; 2,5-dihydrofuran; 2,3-dihydropyran; 3,4-dihydro-2*H*-pyran; 1,3-dioxep-5-ene and its 2-substituted derivatives; *cis*-4,7-dihydro-1,3-dioxepine and its 2-substituted derivatives; *N*-acyl-, *N*-formyl and *N*-alkoxycarbonyl-2,3-dihydro-1*H*-pyrroles.

9. Method according to any of claims 4 to 8, wherein
the method further comprises the step of providing a base,
optionally the base is selected from the group consisting of inorganic carbonate; inorganic phosphate; inorganic acetate; nitrogen containing organic compound; or a mixture thereof and diisopropylethyl amine; dicyclohexylmethyl amine; *N*-methyl-2,2,6,6,tetramethyl piperidine; 2,2,6,6,tetramethyl piperidine; pyridine; 2,6-dimethyl pyridine; 2,6-di-*tert*-butyl pyridine; DABCO (1,4-Diazabicyclo[2.2.2]octane); Li₂CO₃; Na₂CO₃; K₂CO₃; K₃PO₄; LiOAc; NaOAc; and KOAc;
optionally the nitrogen containing organic compound is selected from the group consisting of trialkyl amine; dialkyl amine; *N,N*-dialkylpyridine; *N,N*-dilalkylaniline; or a mixture thereof;
optionally the trialkyl amine is of the formula NR^{a}₃, wherein each R^{a} is independently selected from group consisting of ethyl; *n*-propyl; and *n*-butyl;
optionally the dialkyl amine is of the formula HNR^{b}₂, wherein each R^{b} is independently selected from group consisting of ethyl; *n*-propyl; and *n*-butyl.

10. Method according to any of claims 4 to 9, wherein
the method is carried out in a solvent,
optionally the solvent is selected from the group consisting of an organic solvent; water; and a mixture thereof,
optionally the organic solvent is selected from the group consisting of aromatic solvents;
chlorinated solvents; ester solvents; amide solvents; urea solvents; and mixture thereof; and diethyl ether; THF (tetrahydrofuran); 1,4-dioxane; tetrahydropyran; *tert*-butyl methyl ether; cyclopentyl methyl ether; di-*iso*-propyl ether; 1,2-dimethoxyethane; diglyme; triglyme; benzene; *ortho*-xylene, *meta*-xylene, *para*-xylene, and mixtures of xylenes; toluene; mesitylene; anisole; 1,2-dimethoxybenzene; α,α,α-triftuoromethytbenzene; fluorobenzene; chlorobenzene; DCM (dichloromethane); 1,2-dichloroethane; 1,1-dichloroethane; chloroform; acetone; ethyl acetate; propyl acetates, acetonitrile; Dimethyl sulfoxide (DMSO); Dimethylacetamide (DMA); *N*-Methyl-2-pyrrolidone (NMP), 1,3-Dimethyl-3,4,5,6-tetrahydro-2(1H)-pyrimidinone (DMPU), and a mixture thereof and dioxane.

11. Method according to any one of claims 4 to 10, wherein
(a) the solvent is a mixture of an organic solvent and water in a ratio of 1 : 1 to 100 : 1; or
(b) the solvent is a mixture of an organic solvent and water in a ratio of 5 : 1 to 50 : 1; or
(c) the solvent is a mixture of an organic solvent and water in a ratio of 10 : 1 to 30 : 1; or
(d) the solvent is a mixture of an organic solvent and water in a ratio of 15 : 1 to 25 : 1; or
(e) the solvent is a mixture of an organic solvent and water in a ratio of 19 : 1.

12. Method according to any one of claims 4 to 11, wherein
(a) the reaction temperature of step (ii) is from 0°C to 120°C; or
(b) the reaction temperature of step (ii) is from 15°C to 90°C; or
(c) the reaction temperature of step (ii) is from 30°C to 80°C; or
(d) the reaction temperature of step (ii) is from 50°C to 70°C; or
(e) the reaction time of step (ii) is from 0.1 hour to 144 hours; or
(f) the reaction time of step (ii) is from 0.1 hour to 72 hours; or
(g) the reaction time of step (ii) is from 0.1 hour to 48 hours; or
(h) the reaction time of step (ii) is from 0.1 hour to 24 hours; or
(i) the reaction time of step (ii) is from 0.25 hour to 18 hours; or
(j) the reaction time of step (ii) is from 0.5 hour to 12 hours; or
(k) the reaction time of step (ii) is from 0.5 hour to 6 hours; or
(l) the reaction time of step (ii) is from 1 hour to 4 hours.

13. Use of a catalyst complex in the Pd-catalyzed Heck coupling reaction for forming a covalent carbon-carbon single bond, the catalyst complex comprising Pd and at least one ligand of Formula (I) 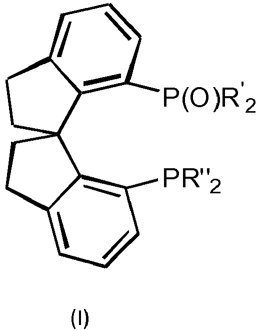 wherein,
R' and R" are independently from each other substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

14. Use according to claim 13, wherein
(a) the ligand of Formula (I) is a ligand of Formula (II) or Formula (III) 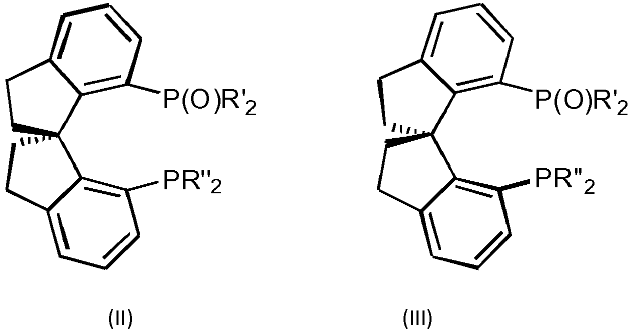 wherein,
R' and R" are independently from each other substituted or unsubstituted, linear or branched alkyl with 1 to 20 carbon atoms; substituted or unsubstituted, linear or branched alkenyl with 2 to 20 carbon atoms; substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms; or
(b) the ligand is selected from the group consisting of 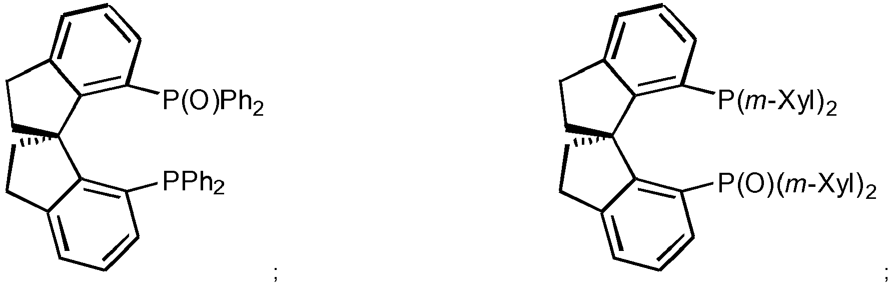 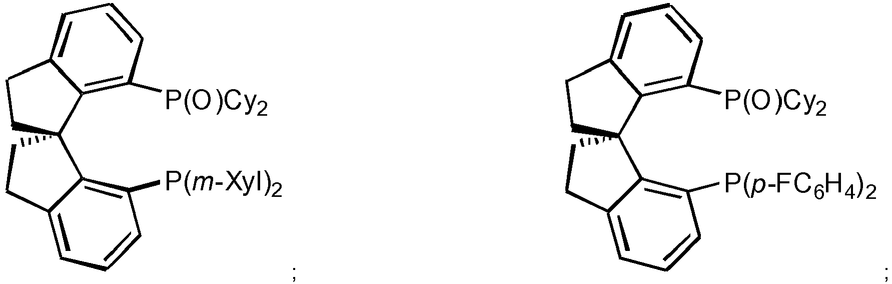 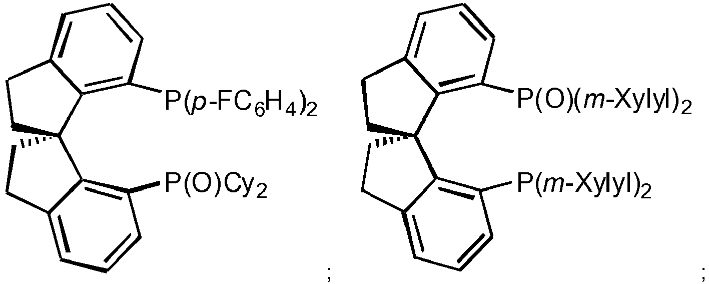 and a mixture thereof.

15. Use according to claim 13 or 14, wherein
(a) the Pd is a Pd(0); and/or a Pd(+II) species; or
(b) the Pd is a Pd(0) specie.

## Patentansprüche

1. Katalysatorkomplex umfassend Pd und mindestens einen Liganden der Formel (I) 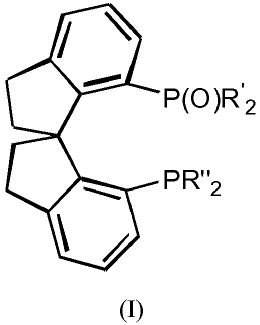 wobei R' und R" unabhängig ausgewählt sind aus der Gruppe bestehend aus substituiertem oder unsubstituiertem, linearem oder verzweigtem Alkyl mit 1 bis 20 Kohlenstoffatomen; substituiertem oder unsubstituiertem, linearen oder verzweigtem Alkenyl mit 2 bis 20 Kohlenstoffatomen; substituiertem oder unsubstituiertem Cycloalkyl mit 5 bis 20 Kohlenstoffatomen; substituiertem oder unsubstituiertem Cycloalkenyl mit 5 bis 20 Kohlenstoffatomen; substituiertem oder unsubstituiertem Aryl mit 5 bis 14 Kohlenstoffatomen; und substituiertem oder unsubstituiertem Heteroaryl mit 5 bis 14 Kohlenstoffatomen.

2. Katalysatorkomplex gemäß Anspruch 1, wobei
(a) der Ligand der Formel (I) ein Ligand der Formel (II) oder der Formel (III) ist 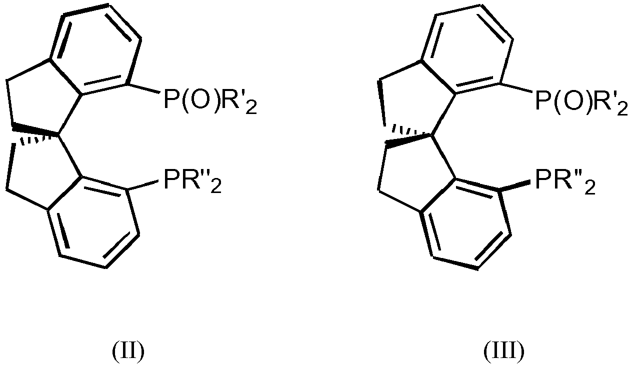 wobei
(i) R' und R" unabhängig ausgewählt sind aus der Gruppe bestehend aus substituiertem oder unsubstituiertem, linearem oder verzweigtem Alkyl mit 1 bis 20 Kohlenstoffatomen; substituiertem oder unsubstituiertem, linearem oder verzweigtem Alkenyl mit 2 bis 20 Kohlenstoffatomen; substituiertem oder unsubstituiertem Cycloalkyl mit 5 bis 20 Kohlenstoffatomen; substituiertem oder unsubstituiertem Cycloalkenyl mit 5 bis 20 Kohlenstoffatomen; substituiertem oder unsubstituiertem Aryl mit 5 bis 14 Kohlenstoffatomen; und substituiertem oder unsubstituiertem Heteroaryl mit 5 bis 14 Kohlenstoffatomen; oder
(ii) R' und R" unabhängig ausgewählt sind aus der Gruppe bestehend aus Cyclohexanyl (Cy), Phenyl (Ph), 1,3-Dimethylbenzol (*meta*-Xylyl) und *para-*FC₆H₄; oder
(b) der Ligand unabhängig ausgewählt ist aus der Gruppe bestehend aus 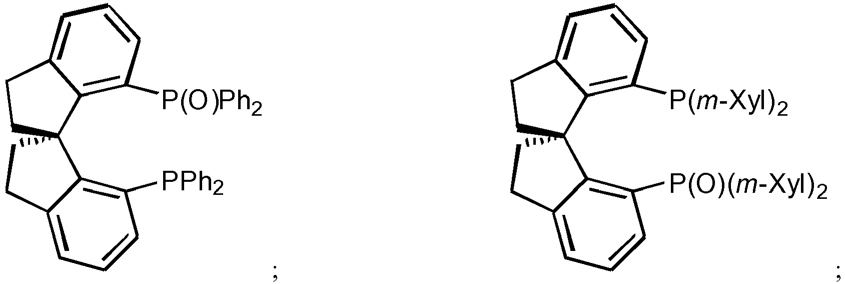 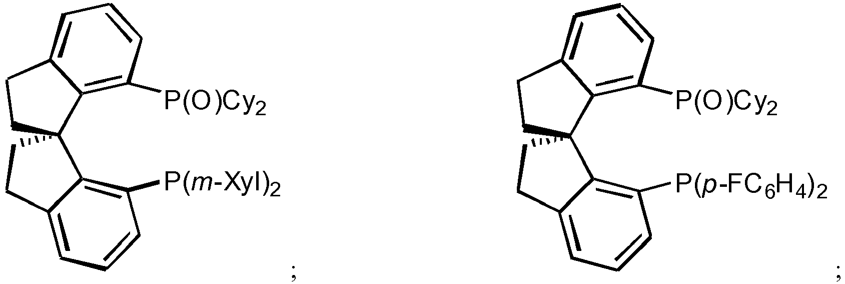 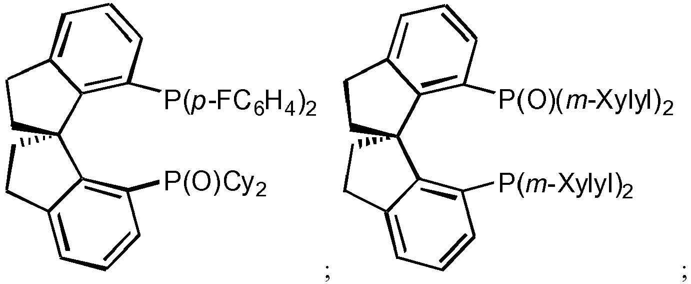 und Mischungen davon.

3. Kalysatorkomplex gemäß einem der Ansprüche 1 bis 2, wobei
(a) das Pd eine (Pd(0)- und/oder eine Pd(+II)-Spezies ist; oder
(b) das Pd eine Pd(0)-Spezies ist; oder
(c) die Pd(0)-Spezies *in situ* erzeugt wird; oder
(d) das Pd aus einem Pd-Prekursor ausgewählt aus der Gruppe bestehend aus Pd(dba)₂, Pd₂(dba)₃, Pd(PPh₃)₄, Pd(OAc)₂, PdCl₂, PdBr₂, PdI₂, PdCl₂(PPh₃)₂, Pd(OAc)₂, Pd(P*tert*-butyl₃)₂ erzeugt wird, wahlweise ist der Pd-Prekursor ausgewählt aus der Gruppe bestehend aus Pd(OAc)₂, Pd(dba)₂, or Pd₂(dba)₃.

4. Verfahren zur Herstellung einer kovalenten Kohlenstoff-Kohlenstoff-Einfachbindung in der Pd-katalysierten Heck-Kohlenstoff-Kohlenstoff-Kupplungsreaktion, wobei das Verfahren umfasst:
(i) Bereitstellen des Katalysatorkomplexes gemäß einem der Ansprüche 1 bis 3; und
(ii) Reagierenlassen mindestens einer elektrophilen Verbindung der Formel (IV) mit mindestens einem Olefin der Formel (V)
R¹-LG¹ (IV) 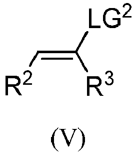
wobei
R¹ eine beliebige organische Verbindung ist;
R² und R³ sich verbinden, um zusammen mit den Kohlenstoffatomen, an die sie gebunden sind, ein substituiertes oder unsubstituiertes 5- bis 40-gliedriges Cycloalkenyl oder Heterocycloalkenyl zu bilden; und LG¹ und LG² Abgangsgruppen sind; und
in der Anwesenheit des Katalysatorkomplexes unter für die Bildung der kovalenten Kohlenstoff-Kohlenstoff-Einfachbindung geeigneten Bedingungen.

5. Verfahren gemäß Anspruch 4, wobei
(a) Die organische funktionelle Gruppe unabhängig ausgewählt ist aus der Gruppe bestehend aus linearem oder verzweigtem, substituiertem oder unsubstituiertem C₁-Cₓ-Alkyl; linearem oder verzweigtem, substituiertem oder unsubstituiertem Alkenyl mit 2 bis x Kohlenstoffatomen, linearem oder verzweigtem, substituiertem oder unsubstituiertem Alkinyl mit 2 bis x Kohlenstoffatomen; linearem oder verzweigtem, substituiertem oder unsubstituiertem Alkoxy mit 1 bis x Kohlenstoffatomen; substituiertem oder unsubstituiertem Cycloalkyl mit 3 bis x Kohlenstoffatomen; substituiertem oder unsubstituiertem Cycloalkenyl mit 3 bis x Kohlenstoffatomen; substituiertem oder unsubstituiertem Aryl mit 6 bis x Kohlenstoffatomen; und substituiertem oder unsubstituiertem Heteroaryl mit 3 bis x Kohlenstoffatomen; wobei x eine beliebige ganze Zahl von 2 oder mehr, bevorzugt bis zu 50, noch bevorzugter bis zu 30 ist; und/oder
(b) (i) R¹ ausgewählt ist aus der Gruppe bestehend aus linearem oder verzweigtem, substituiertem oder unsubstituiertem Alkenyl mit 2 bis x Kohlenstoffatomen; substituiertem oder unsubstituiertem Cycloalkenyl mit 3 bis x Kohlenstoffatomen; substituiertem oder unsubstituiertem Aryl mit 6 bis x Kohlenstoffatomen; und substituiertem oder unsubstituiertem Heteroaryl mit 3 bis x Kohlenstoffatomen; wobei x eine beliebige ganze Zahl von 2 oder mehr, bevorzugt bis zu 50, noch bevorzugter bis zu 30 ist; oder
(b) (ii) R¹ ausgewählt ist aus der Gruppe bestehend aus substituiertem oder unsubstituiertem, linearem oder verzweigtem Alkenyl mit 3 bis 15 Kohlenstoffatomen; substituiertem oder unsubstituiertem Benzol; und substituiertem oder unsubstituiertem Naphtalin; und/oder
(c) (i) LG¹ ausgewählt ist aus der Gruppe bestehend aus Halogen, -OSO₂C₄F₉, - OSO₂CF₃, -OSO₂F, -OTs, und -OMs; oder
(c) (ii) LG¹ ausgewählt ist aus der Gruppe bestehend aus Cl, Br, I, and -OSO₂CF₃; oder
(c) (iii) LG¹ -OSO₂CF₃ oder Br ist.

6. Verfahren gemäß Anspruch 4 oder 5, wobei die mindestens eine elektrophile Verbindung der Formel (IV) ausgewählt ist aus der Gruppe bestehend aus 1-Bromnaphthalin, 2-Bromnaphthalin, Brombenzol, 4-Bromanisol, 4-Bromtoluol, 1-Brom-4-fluorbenzol, 2-Bromanisol, *N*-Methyl-2-brompyrrol, 3-Bromindol, 5-Brom-2-methyl-1,3-benzothiazol, 3-Brombenzofuran, 3-Brombenzothiophen, 2-Bromthiophen, 2-Bromthiophen, 4-Brom-3-chromen, 1-Bromstyrol und (*E*)-2-Bromstyrol, 1-Bromcyclohexen, 1-Bromcyclopenten, Bromethen, (*E*)-1-Brompropen, 2-Brompropen, Iodbenzol, 1-Iodnaphthalin, 2-Iodnaphthalin, 4-Iodanisol, 4-Iodtoluol, 4-Chlortoluol, 2-Chlortoluol, 1-Chlornaphthalin, 2-Chlornaphthalin, Chlorbenzol, 4-Chloranisol, 2-Chloranisol, 3-Chlorindol, *N-*Methyl-2-chlorpyrrol, 5-Chlor-1,3-benzothiazol, 3-Chlorbenzofuran, 3-Chlorbenzothiophen, 2-Chlorthiophen, 1-Naphthyltriflat, 2- Naphthyltriflat, Phenyltriflat, *para-tert*-Butylphenyltriflat, *para-* (Trifluormethyl)phenyltriflat, *para-*Chlorophenyltriflat und *para*-Fluorphenyltriflat, *para*-Formylphenyltriflat, *para-*(Ethoxycarbonyl)phenyltriflat, *para*-Anisyltriflat, *para-tert*-Butylphenyltriflat, *ortho-*Methylphenyltriflat, *para*-Chlorphenyltriflat, *para*-Benzphenonyltriflat, *ortho-*Anisyltriflat, *meta*-Anisyltriflat, 1-Tosyl-1*H*-indol- 5-yltriflat, 2-Methylbenzo[*d*]thiazol-5-yltriflat, 2-Thienyl- und 3-Thienyltrilfaten und ihren Benzo-Derivativen, 2-Furanyl- und 3-Furanyltriflaten und ihren Benzo- Derivativen, *N*-Boc-2-Pyrrolidinyl- und *N*-Boc-3-Pyrrolidinyltrilfaten, Cyclohexenyltriflat, 2-Methylcyclohexenyltriflat, 1-Styryl- und (*E*)-2- Styryltrilfaten.

7. Verfahren gemäß einem der Ansprüche 4 bis 6, wobei
(a) R² und R³ sich verbinden, um zusammen mit den Kohlenstoffatomen, an die sie gebunden sind, ein substituiertes oder unsubstituiertes 5- bis 20-gliedriges Cycloalkenyl oder Heterocycloalkenyl zu bilden; oder
(b) R² und R³ sich verbinden, um zusammen mit den Kohlenstoffatomen, an die sie gebunden sind, ein substituiertes oder unsubstituiertes 5- oder 6-gliedriges Cycloalkenyl oder Heterocycloalkenyl zu bilden.

8. Verfahren gemäß einem der Ansprüche 4 bis 7, wobei
(a) LG² Wasserstoff ist; und/oder
(b) das mindestens eine Olefin der Formel (V) ausgewählt ist aus der Gruppe bestehend aus Cyclopenten; Cyclohexen; Cylohepten; Cycloocten; 2,3-Dihydrofuran; 2,5-Dihydrofuran; 2,3-Dihydropyran; 3,4-Dihydro-2*H*-pyran; 1,3-Dioxep-5-en und seinen 2-substituierten Derivativen; *cis*-4,7-Dihydro-1,3-dioxepin und seinen 2-substituierten Derivativen; *N*-Acyl-, *N*-Formyl und *N-*Alkoxycarbonyl-2,3-dihydro-1*H*-pyrrolen.

9. Verfahren gemäß einem der Ansprüche 4 bis 8, wobei
das Verfahren des Weiteren den Schritt des Bereitstellens einer Base umfasst,
wobei, wahlweise, die Base ausgewählt ist aus der Gruppe bestehend aus anorganischem Carbonat; anorganischem Phosphat; anorganischem Acetat; Stickstoff-haltiger organischer Verbindung; oder einer Mischung davon und Diisopropylethylamin; Dicyclohexylmethylamin; *N*-Methyl-2,2,6,6,tetramethylpiperidin; 2,2,6,6,Tetramethylpiperidin; Pyridin; 2,6-Dimethylpyridin; 2,6-Di-*tert*-butyl pyridin; DABCO (1,4-Diazabicyclo[2.2.2]octan); Li₂CO₃; Na₂CO₃; K₂CO₃; K₃PO₄; LiOAc; NaOAc; und KOAc;
wobei, wahlweise, die Stickstoff-haltige organische Verbindung ausgewählt ist aus der Gruppe bestehend aus Trialkylamin; Dialkylamin; *N,N*-Dialkylpyridin; *N,N-*Dilalkylanilin; oder einer Mischung davon;
wobei, wahlweise, das Trialkylamin die Formel NR^{a}₃ hat, wobei jedes R^{a} unabhängig ausgewählt ist aus der Gruppe bestehend aus Ethyl; *n*-Propyl; und *n-*Butyl;
wobei, wahlweise, das Dialkylamin die Formel HNR^{b}₂ hat, wobei jedes R^{b} unabhängig ausgewählt ist aus der Gruppe bestehend aus Ethyl; *n*-Propyl; und *n-*Butyl.

10. Verfahren gemäß einem der Ansprüche 4 bis 9, wobei
das Verfahren in einem Lösungsmittel durchgeführt wird,
wobei, wahlweise, das Lösungsmittel ausgewählt ist aus der Gruppe bestehend aus organischem Lösungsmittel; Wasser; und einer Mischung davon,
wobei, wahlweise, das organische Lösungsmittel ausgewählt ist aus der Gruppe bestehend aus aromatischen Lösungsmitteln; chlorierten Lösungsmitteln; Ester-Lösungsmitteln; Amid-Lösungsmitteln; Urea-Lösungsmitteln; und einer Mischung davon; und Diethylether; THF (Tetrahydrofuran); 1,4-Dioxan; Tetrahydropyran; *tert*-Butylmethylether, Cyclopentylmethylether; Di-*iso*-propylether; 1,2-Dimethoxyethan; Diglyme; Triglyme; Benzol; *ortho*-Xylol, *meta*-Xylol, *para*-Xylol und Mischungen von Xylolen; Toluol; Mesitylen; Anisol; 1,2-Dimethoxybenzol;
α,α,α-Trifluormethylbenzol; Fluorbenzol; Chlorbenzol; DCM (Dichlormethan); 1,2-Dichlorethan; 1,1-Dichlorethan; Chloroform; Aceton; Ethylacetat; Propylacetaten, Acetonitril; Dimethylsulfoxid (DMSO); Dimethylacetamid (DMA); *N*-Methyl-2-pyrrolidon (NMP), 1,3-Dimethyl-3,4,5,6-tetrahydro-2(1H)-pyrimidinon (DMPU) und einer Mischung davon und Dioxan.

11. Verfahren gemäß einem der Ansprüche 4 bis 10, wobei
(a) das Lösungsmittel eine Mischung aus einem organischen Lösungsmittel und Wasser in einem Verhältnis von 1 : 1 bis 100 : 1 ist: oder
(b) das Lösungsmittel eine Mischung aus einem organischen Lösungsmittel und Wasser in einem Verhältnis von 5 : 1 bis 50 : 1 ist: oder
(c) das Lösungsmittel eine Mischung aus einem organischen Lösungsmittel und Wasser in einem Verhältnis von 10 : 1 bis 30 : 1 ist: oder
(d) das Lösungsmittel eine Mischung aus einem organischen Lösungsmittel und Wasser in einem Verhältnis von 15 : 1 bis 25 : 1 ist: oder
(e) das Lösungsmittel eine Mischung aus einem organischen Lösungsmittel und Wasser in einem Verhältnis von 19 : 1 ist.

12. Verfahren gemäß einem der Ansprüche 4 bis 11, wobei
(a) die Reaktionstemperatur von Schritt (ii) 0 °C bis 120 °C beträgt; oder
(b) die Reaktionstemperatur von Schritt (ii) 15 °C bis 90 °C beträgt; oder
(c) die Reaktionstemperatur von Schritt (ii) 30 °C bis 80 °C beträgt; oder
(d) die Reaktionstemperatur von Schritt (ii) 50 °C bis 70 °C beträgt; oder
(e) die Reaktionszeit von Schritt (ii) 0,1 bis 144 Stunden beträgt; oder
(f) die Reaktionszeit von Schritt (ii) 0,1 bis 72 Stunden beträgt; oder
(g) die Reaktionszeit von Schritt (ii) 0,1 bis 48 Stunden beträgt; oder
(h) die Reaktionszeit von Schritt (ii) 0,1 bis 24 Stunden beträgt; oder
(i) die Reaktionszeit von Schritt (ii) 0,25 bis 18 Stunden beträgt; oder
(j) die Reaktionszeit von Schritt (ii) 0,5 bis 12 Stunden beträgt; oder
(k) die Reaktionszeit von Schritt (ii) 0,5 bis 6 Stunden beträgt; oder
(l) die Reaktionszeit von Schritt (ii) 1 bis 4 Stunden beträgt.

13. Verwendung eines Katalysatorkomplexes in der Pd-katalysierten Heck-Kupplungsreaktion zur Bildung einer kovalenten Kohlenstoff-Kohlenstoff-Einfachbindung, wobei der Katalysatorkomplex Pd und mindestens einen Liganden der Formel (I) umfasst 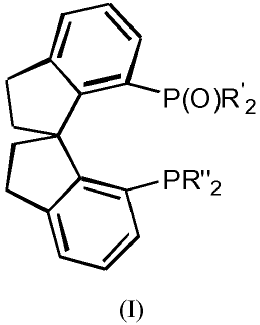 wobei
R' und R" unabhängig voneinander substituiertes oder unsubstituiertes, lineares oder verzweigtes Alkyl mit 1 bis 20 Kohlenstoffatomen; substituiertes oder unsubstituiertes, lineares oder verzweigtes Alkenyl mit 2 bis 20 Kohlenstoffatomen; substituiertes oder unsubstituiertes Cycloalkyl mit 5 bis 20 Kohlenstoffatomen; substituiertes oder unsubstituiertes Cycloalkenyl mit 5 bis 20 Kohlenstoffatomen; substituiertes oder unsubstituiertes Aryl mit 5 bis 14 Kohlenstoffatomen; und substituiertes oder unsubstituiertes Heteroaryl mit 5 bis 14 Kohlenstoffatomen sind.

14. Verwendung gemäß Anspruch 13, wobei
(a) der Ligand der Formel (I) ein Ligand der Formel (II) oder der Formel (III) ist 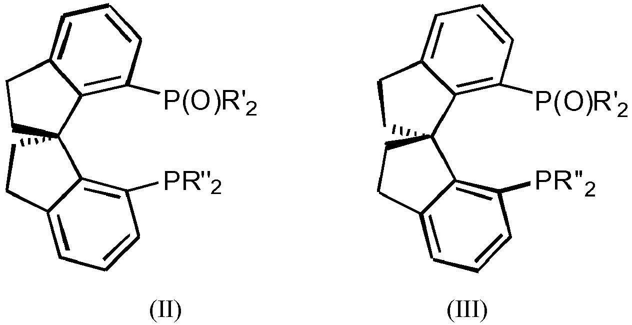 wobei R' und R" unabhängig voneinander substituiertes oder unsubstituiertes, lineares oder verzweigtes Alkyl mit 1 bis 20 Kohlenstoffatomen; substituiertes oder unsubstituiertes, lineares oder verzweigtes Alkenyl mit 2 bis 20 Kohlenstoffatomen; substituiertes oder unsubstituiertes Cycloalkyl mit 5 bis 20 Kohlenstoffatomen; substituiertes oder unsubstituiertes Cycloalkenyl mit 5 bis 20 Kohlenstoffatomen; substituiertes oder unsubstituiertes Aryl mit 5 bis 14 Kohlenstoffatomen; und substituiertes oder unsubstituiertes Heteroaryl mit 5 bis 14 Kohlenstoffatomen sind; oder
(b) der Ligand ausgewählt ist aus der Gruppe bestehend aus 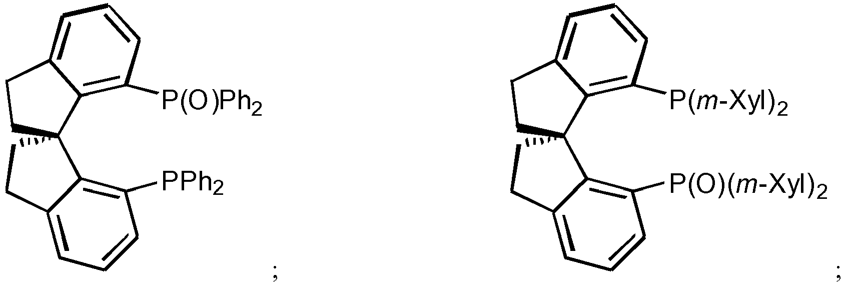 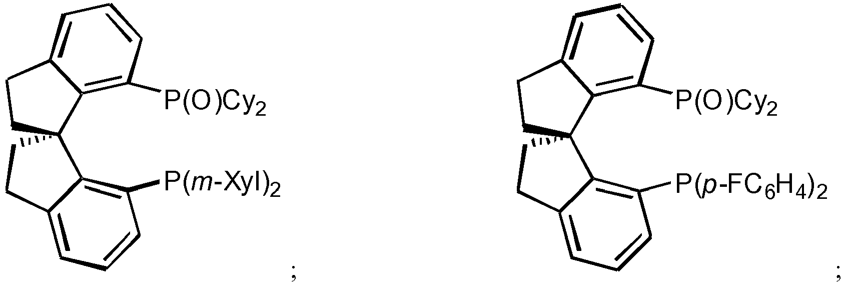 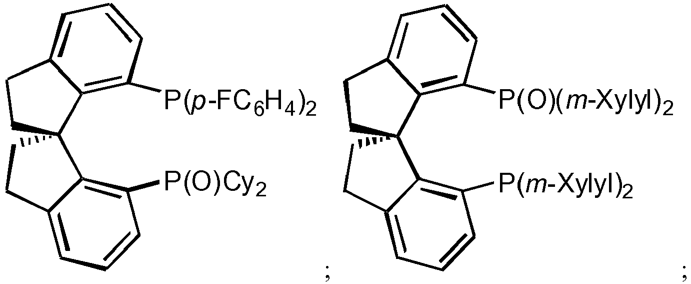 und einer Mischung davon.

15. Verwendung gemäß Anspruch 13 oder 14, wobei
(a) das Pd eine eine (Pd(0)- und/oder eine Pd(+II)-Spezies ist; oder
(b) das Pd eine Pd(0)-Spezies ist.

## Revendications

1. Complexe catalytique comprenant du Pd et au moins un ligand de formule (I) 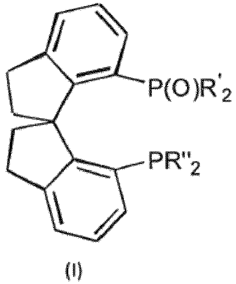 dans laquelle
R' et R" sont indépendamment choisis dans le groupe constitué par un alkyle linéaire ou ramifié, substitué ou non substitué ayant de 1 à 20 atomes de carbone ; un alcényle linéaire ou ramifié, substitué ou non substitué ayant de 2 à 20 atomes de carbone ; un cycloalkyle substitué ou non substitué ayant de 5 à 20 atomes de carbone ; un cycloalcényle substitué ou non substitué ayant de 5 à 20 atomes de carbone ; un aryle substitué ou non substitué ayant de 5 à 14 atomes de carbone ; et un hétéroaryle substitué ou non substitué ayant de 5 à 14 atomes de carbone.

2. Complexe catalytique selon la revendication 1, dans lequel
(a) le ligand de formule (I) est un ligand de formule (II) ou de formule (III) 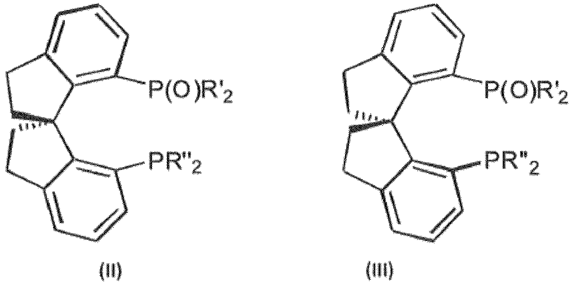 dans lesquelles
(i) R' et R" sont indépendamment choisis dans le groupe constitué par un alkyle linéaire ou ramifié, substitué ou non substitué ayant de 1 à 20 atomes de carbone ; un alcényle linéaire ou ramifié, substitué ou non substitué ayant de 2 à 20 atomes de carbone ; un cycloalkyle substitué ou non substitué ayant de 5 à 20 atomes de carbone ; un cycloalcényle substitué ou non substitué ayant de 5 à 20 atomes de carbone ; un aryle substitué ou non substitué ayant de 5 à 14 atomes de carbone ; et un hétéroaryle substitué ou non substitué ayant de 5 à 14 atomes de carbone ; ou
(ii) R' et R" sont indépendamment choisis dans le groupe constitué par un cyclohexanyle (Cy), un phényle (Ph), un 1,3-diméthylbenzène (*méta*)-xylyle) et un *para*-FC₆H₄; ou
(b) le ligand est indépendamment choisi dans le groupe constitué par 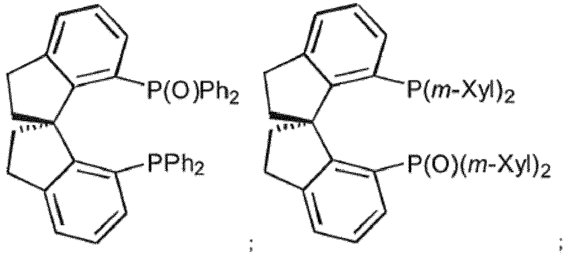 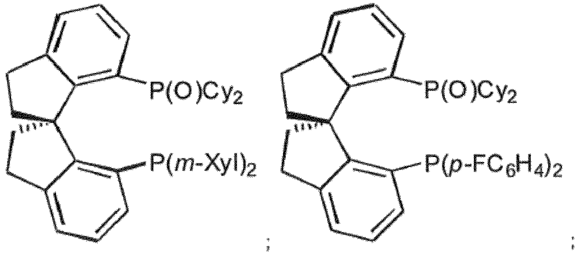 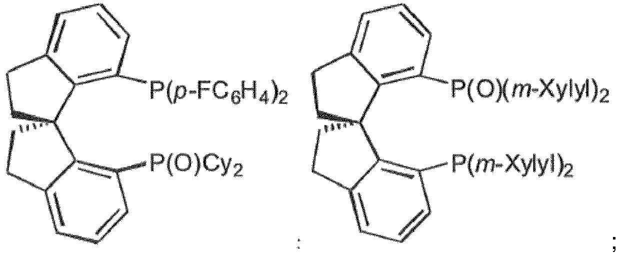 et des mélanges de ceux-ci.

3. Complexe catalytique selon l'une quelconque des revendications 1 à 2, dans lequel
(a) le Pd est une espèce Pd(0) et/ou une espèce Pd(+II) ; ou
(b) le Pd est une espèce Pd(0) ; ou
(c) l'espèce Pd(0) est générée *in situ* ; ou
(d) le Pd est généré à partir d'un précurseur de Pd choisi dans le groupe constitué par Pd(dba)₂, Pd₂(dba)₃, Pd(PPh₃)₄, Pd(OAc)₂, PdCl₂, PdBr₂, PdI₂, PdCl₂(PPh₃)₂, Pd(OAc)₂, Pd(P*tert*-butyle₃)₂, facultativement le précurseur de Pd est choisi dans le groupe constitué par Pd(OAc)₂, Pd(dba)₂ ou Pd₂(dba)₃.

4. Procédé de formation d'une liaison simple carbone-carbone covalente dans la réaction de couplage carbone-carbone de Heck catalysée par du Pd, le procédé comprenant :
(i) l'utilisation du complexe catalytique selon l'une quelconque des revendications 1 à 3 ; et
(ii) la mise en réaction d'au moins un composé électrophile de formule (IV) avec au moins une oléfine de formule (V)
R¹-LG¹ (IV) 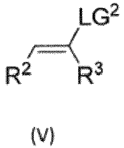
dans lesquelles
R¹ est un quelconque composé organique ;
R² et R³ sont combinés pour former ensemble avec les atomes de carbones auxquels ils sont attachés un cycloalcényle ou un hétérocycloalcényle de 5 à 40 chaînons substitué ou non substitué ; et
LG¹ et LG² sont des groupements partants ; et
en présence du complexe catalytique dans des conditions appropriées pour former la liaison simple carbone-carbone covalente.

5. Procédé selon la revendication 4, dans lequel
(a) le fragment organique est indépendamment choisi dans le groupe constitué par un alkyle en C₁ à Cₓ substitué ou non substitué, linéaire ou ramifié ; un alcényle substitué ou non substitué, linéaire ou ramifié ayant de 2 à x atomes de carbone ; un alcynyle substitué ou non substitué, linéaire ou ramifié ayant de 2 à x atomes de carbone ; un alcoxy substitué ou non substitué, linéaire ou ramifié ayant de 1 à x atomes de carbone ; un cycloalkyle substitué ou non substitué ayant de 3 à x atomes de carbone ; un cycloalcényle substitué ou non substitué ayant de 3 à x atomes de carbone ; un aryle substitué ou non substitué ayant de 6 à x atomes de carbone ; et un hétéroaryle substitué ou non substitué ayant de 3 à x atomes de carbone ; x étant un quelconque nombre entier de 2 ou plus, de préférence allant jusqu'à 50, plus préférablement allant jusqu'à 30 ; et/ou
(b)(i) R¹ est choisi dans le groupe constitué par un alcényle substitué ou non substitué, linéaire ou ramifié ayant de 2 à x atomes de carbone ; un cycloalcényle substitué ou non substitué ayant de 3 à x atomes de carbone ; un aryle substitué ou non substitué ayant de 6 à x atomes de carbone ; et un hétéroaryle substitué ou non substitué ayant de 3 à x atomes de carbone ; x étant un quelconque nombre entier de 2 ou plus, de préférence allant jusqu'à 50, plus préférablement allant jusqu'à 30 ; ou
(b)(ii) R¹ est choisi dans le groupe constitué par un alcényle linéaire ou ramifié, substitué ou non substitué ayant de 3 à 15 atomes de carbone ; un benzène substitué ou non substitué ; et un naphtalène substitué ou non substitué ; et/ou
(c)(i) LG¹ est choisi dans le groupe constitué par un halogène, - OSO₂C₄F₉, -OSO₂CF₃, -OSO₂F, -OTs et -OMs ; ou
(c)(ii) LG¹ est choisi dans le groupe constitué par Cl, Br, I et -OSO₂CF₃; ou
(c)(iii) LG¹ est -OSO₂CF₃ ou Br.

6. Procédé selon la revendication 4 ou 5, dans lequel l'au moins un composé électrophile de formule (IV) est choisi dans le groupe constitué par le 1-bromonaphtalène, le 2-bromonaphtalène, le bromobenzène, le 4-bromoanisole, le 4-bromotoluène, le 1-bromo-4-fluorobenzène, le 2-bromoanisole, le *N*-méthyl-2-bromopyrrole, le 3-bromoindole, le 5-bromo-2-méthyl-1,3-benzothiazole, le 3-bromobenzofurane, le 3-bromobenzothiophène, le 2-bromothiophène, le 2-bromothiophène, le 4-bromo-3-chromène, le 1-bromostyrène et le (*E*)-2-bromostyrène, le 1-bromocyclohexène, le 1-bromocyclopentène, le bromoéthène, le (*E*)-1-bromopropène, le 2-bromopropène, l'iodobenzène, le 1-iodonaphtalène, le 2-iodonaphtalène, le 4-iodoanisole, le 4-iodotoluène, le 4-chlorotoluène, le 2-chlorotoluène, le 1-chloronaphtalène, le 2-chloronaphtalène, le chlorobenzène, le 4-chloroanisole, le 2-chloroanisole, le 3-chloroindole, le *N-*méthyl-2-chloropyrrole, le 5-chloro-1,3-benzothiazole, le 3-chlorobenzofurane, le 3-chlorobenzothiophène, le 2-chlorothiophène, le triflate de 1-naphtyle, le triflate de 2-naphtyle, le triflate de phényle, le triflate de *para-tert*-butylphényle, le triflate de *para*-(trifluorométhyl)phényle, le triflate de para-chlorophényle et le triflate de *para*-fluorophényle, le triflate de para-formylphényle, le triflate de para-(éthoxycarbonyl)phényle, le triflate de *para*-anisyle, le triflate de *para-tert*-butylphényle, le triflate d'*ortho-*méthylphényle, le triflate de *para*-chlorophényle, le triflate de *para-*benzophénonyle, le triflate d'*ortho*-anisyle, le triflate de *méta*-anisyle, le triflate de 1-tosyl-1*H*-indol-5-yle, le triflate de 2-méthylbenzo[d]thiazol-5-yle, les triflates de 2-thiényle et 3-thiényle et leurs dérivés benzoïques, les triflates de 2-furanyle et 3-furanyle et leurs dérivés benzoïques, les triflates de *N*-Boc-2-pyrrolidinyle et *N*-Boc-3-pyrrolidinyle, le triflate de cyclohexényle, le triflate de 2-méthylcyclohexényle, les triflates de 1-styryle et (*E*)-2-styryle.

7. Procédé selon l'une quelconque des revendications 4 à 6, dans lequel
(a) R² et R³ sont combinés pour former ensemble avec les atomes de carbone auxquels ils sont attachés un cycloalcényle ou un hétérocycloalcényle de 5 à 20 chaînons substitué ou non substitué ; ou
(b) R² et R³ sont combinés pour former ensemble avec les atomes de carbone auxquels ils sont attachés un cycloalcényle ou un hétérocycloalcényle de 5 ou 6 chaînons substitué ou non substitué.

8. Procédé selon l'une quelconque des revendications 4 à 7, dans lequel
(a) LG² est un hydrogène ; et/ou
(b) l'au moins une oléfine de formule (V) est choisie dans le groupe constitué par le cyclopentène ; le cyclohexène ; le cycloheptène ; le cyclooctène ; le 2,3-dihydrofurane ; le 2,5-dihydrofurane ; le 2,3-dihydropyrane ; le 3,4-dihydro-2*H*-pyrane ; le 1,3-dioxép-5-ène et ses dérivés substitués en position 2 ; la *cis*-4,7-dihydro-1,3-dioxépine et ses dérivés substitués en position 2 ; les *N*-acyl-, *N*-formyl- et *N-*alcoxycarbonyl-2,3-dihydro-1*H*-pyrroles.

9. Procédé selon l'une quelconque des revendications 4 à 8, dans lequel
le procédé comprend en outre l'étape d'utilisation d'une base, facultativement la base est choisie dans le groupe constitué par un carbonate inorganique ; un phosphate inorganique ; un acétate inorganique ; un composé organique contenant de l'azote ; ou un mélange de ceux-ci et la diisopropyléthylamine ; la dicyclohexyl-méthylamine ; la *N*-méthyl-2,2,6,6-tétraméthyl-pipéridine ; la 2,2,6,6-tétraméthylpipéridine ; la pyridine ; la 2,6-diméthylpyridine ; la 2,6-di-tert-butylpyridine ; le DABCO (1,4-diazabicyclo[2.2.2]octane) ; Li₂CO₃ ; Na₂CO₃ ; K₂CO₃ ; K₃PO₄ ; LiOAc ; NaOAc ; et KOAc ;
facultativement le composé organique contenant de l'azote est choisi dans le groupe constitué par une trialkylamine ; un dialkyamine ; une *N,N-*dialkylpyridine ; une *N,N*-dialkylaniline ; ou un mélange de celles-ci ; facultativement la trialkylamine est de formule NR^{a}₃, dans laquelle chaque R^{a} est indépendamment choisi dans le groupe constitué par un éthyle ; un n-propyle ; et un n-butyle ;
facultativement la dialkylamine est de formule HNR^{b}₂, dans laquelle chaque R^{b} est indépendamment choisi dans le groupe constitué par un éthyle ; un n-propyle ; et un n-butyle.

10. Procédé selon l'une quelconque des revendications 4 à 9, dans lequel
le procédé est réalisé dans un solvant,
facultativement le solvant est choisi dans le groupe constitué par un solvant organique ; de l'eau ; et un mélange de ceux-ci,
facultativement le solvant organique est choisi dans le groupe constitué par les solvants aromatiques ; les solvants chlorés ; les solvants de type ester ; les solvants de type amide ; les solvants de type urée ; et des mélanges de ceux-ci ; et l'éther diéthylique ; le THF (tétrahydrofurane) ; le 1,4-dioxane ; le tétrahydropyrane ; l'éther *tert*-butylméthylique ; l'éther cyclopentylméthylique ; l'éther di-*iso*-propylique ; le 1,2-diméthoxyéthane ; le diglyme ; le triglyme ; le benzène ; l'*ortho*-xylène, le *méta*-xylène, le *para-*xylène et des mélanges de xylènes ; le toluène ; le mésitylène ; l'anisole ; le 1,2-diméthoxybenzène ; le α,α,α-trifiuorométhyibenzène ; le fluorobenzène ; le chlorobenzène ; le DCM (dichlorométhane) ; le 1,2-dichloroéthane ; le 1,1-dichloroéthane ; le chloroforme ; l'acétone ; l'acétate d'éthyle ; les acétates de propyle ; l'acétonitrile ; le diméthylsulfoxyde (DMSO) ; le diméthylacétamide (DMA) ; la *N*-méthyl-2-pyrrolidone (NMP), la 1,3-diméthyl-3,4,5,6-tétrahydro-2(1H)-pyrimidinone (DMPU) et un mélange de ceux-ci et le dioxane.

11. Procédé selon l'une quelconque des revendications 4 à 10, dans lequel
(a) le solvant est un mélange d'un solvant organique et d'eau selon un rapport de 1 : 1 à 100 :1 ; ou
(b) le solvant est un mélange d'un solvant organique et d'eau selon un rapport de 5 : 1 à 50 :1 ; ou
(c) le solvant est un mélange d'un solvant organique et d'eau selon un rapport de 10 : 1 à 30 :1 ; ou
(d) le solvant est un mélange d'un solvant organique et d'eau selon un rapport de 15 : 1 à 25 :1 ; ou
(e) le solvant est un mélange d'un solvant organique et d'eau selon un rapport de 19 : 1.

12. Procédé selon l'une quelconque des revendications 4 à 11, dans lequel
(a) la température de réaction de l'étape (ii) est de 0 °C à 120 °C ; ou
(b) la température de réaction de l'étape (ii) est de 15 °C à 90 °C ; ou
(c) la température de réaction de l'étape (ii) est de 30 °C à 80 °C ; ou
(d) la température de réaction de l'étape (ii) est de 50 °C à 70 °C ; ou
(e) le temps de réaction de l'étape (ii) est de 0,1 heure à 144 heures ; ou
(f) le temps de réaction de l'étape (ii) est de 0,1 heure à 72 heures ; ou
(g) le temps de réaction de l'étape (ii) est de 0,1 heure à 48 heures ; ou
(h) le temps de réaction de l'étape (ii) est de 0,1 heure à 24 heures ; ou
(i) le temps de réaction de l'étape (ii) est de 0,25 heure à 18 heures ; ou
(j) le temps de réaction de l'étape (ii) est de 0,5 heure à 12 heures ; ou
(k) le temps de réaction de l'étape (ii) est de 0,5 heure à 6 heures ; ou
(l) le temps de réaction de l'étape (ii) est de 1 heure à 4 heures.

13. Utilisation d'un complexe catalytique dans la réaction de couplage de Heck catalysée par du Pd pour former une liaison simple carbone-carbone covalente, le complexe catalytique comprenant du Pd et au moins un ligand de formule (I) 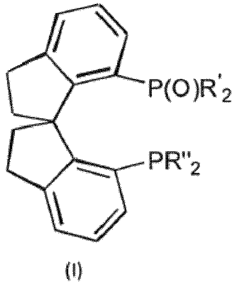 dans laquelle
R' et R" sont indépendamment l'un de l'autre un alkyle linéaire ou ramifié, substitué ou non substitué ayant de 1 à 20 atomes de carbone ; un alcényle linéaire ou ramifié, substitué ou non substitué ayant de 2 à 20 atomes de carbone ; un cycloalkyle substitué ou non substitué ayant de 5 à 20 atomes de carbone ; un cycloalcényle substitué ou non substitué ayant de 5 à 20 atomes de carbone ; un aryle substitué ou non substitué ayant de 5 à 14 atomes de carbone ; et un hétéroaryle substitué ou non substitué ayant de 5 à 14 atomes de carbone.

14. Utilisation selon la revendication 13, dans laquelle
(a) le ligand de formule (I) est un ligand de formule (II) ou de formule (III) 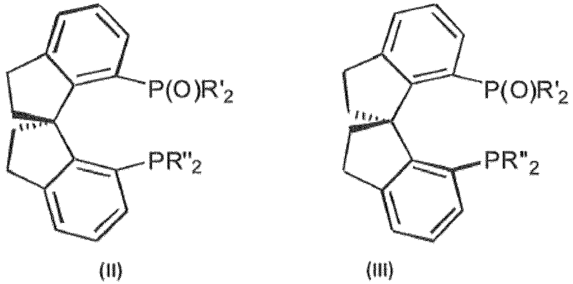 dans lesquelles
(i) R' et R" sont indépendamment l'un de l'autre un alkyle linéaire ou ramifié, substitué ou non substitué ayant de 1 à 20 atomes de carbone ; un alcényle linéaire ou ramifié, substitué ou non substitué ayant de 2 à 20 atomes de carbone ; un cycloalkyle substitué ou non substitué ayant de 5 à 20 atomes de carbone ; un cycloalcényle substitué ou non substitué ayant de 5 à 20 atomes de carbone ; un aryle substitué ou non substitué ayant de 5 à 14 atomes de carbone ; et un hétéroaryle substitué ou non substitué ayant de 5 à 14 atomes de carbone ; ou
(b) le ligand est indépendamment choisi dans le groupe constitué par 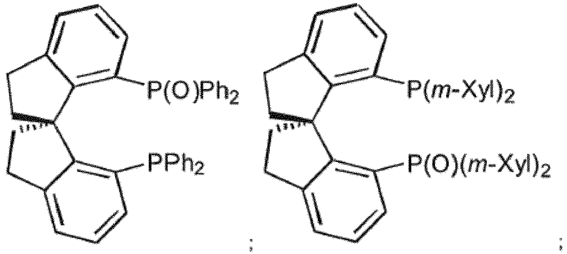 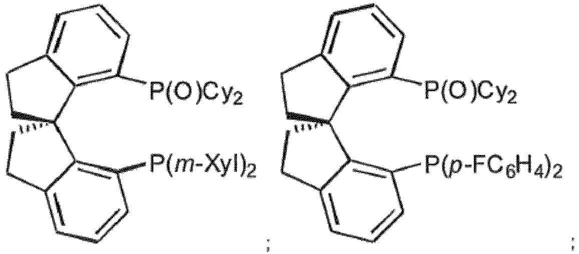 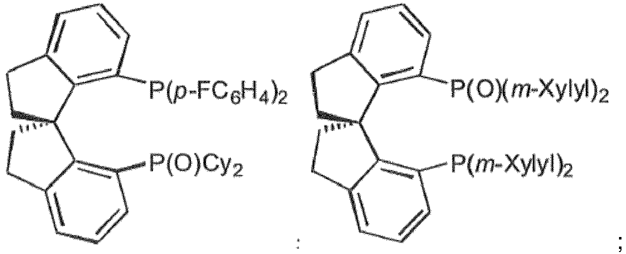 et un mélange de ceux-ci.

15. Utilisation selon la revendication 13 ou 14, dans laquelle
(a) le Pd est une espèce Pd(0) ; et/ou une espèce Pd(+II) ; ou
(b) le Pd est une espèce Pd(0).
